(19) **Europäisches Patentamt / European Patent Office / Office européen des brevets**

(11) **EP 2 977 052 B1**

(12) **EUROPEAN PATENT SPECIFICATION**

(45) Date of publication and mention of the grant of the patent:
**11.12.2019 Bulletin 2019/50**

(51) Int Cl.:
*A61K 31/716* (2006.01)     *A61P 1/00* (2006.01)
*A61P 1/16* (2006.01)     *A61P 3/00* (2006.01)

(21) Application number: **14757504.7**

(22) Date of filing: **27.02.2014**

(86) International application number:
**PCT/JP2014/054819**

(87) International publication number:
**WO 2014/133060 (04.09.2014 Gazette 2014/36)**

(54) **AGENT FOR LIFESTYLE-RELATED DISEASE AND ORAL COMPOSITION COMPRISING SAME**

WIRKSTOFF FÜR ERKRANKUNGEN IM ZUSAMMENHANG MIT DEM LEBENSSTIL UND ORALE ZUSAMMENSETZUNG DAMIT

AGENT DESTINÉ À UNE MALADIE LIÉE AU STYLE DE VIE ET COMPOSITION ORALE LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2013   JP 2013041270**
          **14.05.2013   JP 2013102193**
          **08.10.2013   JP 2013211477**
          **15.01.2014   JP 2014005489**

(43) Date of publication of application:
**27.01.2016 Bulletin 2016/04**

(73) Proprietor: **Hayashibara Co., Ltd.**
**Okayama-shi, Okayama 702-8006 (JP)**

(72) Inventors:
• **TANIGUCHI, Yoshifumi**
  **Okayama-shi**
  **Okayama 702-8006 (JP)**
• **INOUE, Shinichiro**
  **Okayama-shi**
  **Okayama 702-8006 (JP)**

• **WATANABE, Hikaru**
  **Okayama-shi**
  **Okayama 702-8006 (JP)**

(74) Representative: **Daniels, Jeffrey Nicholas**
**Page White & Farrer**
**Bedford House**
**John Street**
**London WC1N 2BF (GB)**

(56) References cited:
**WO-A1-2008/136331     JP-A- H03 285 653**
**JP-A- H09 224 608     JP-A- 2010 095 701**
**JP-A- 2010 100 583     JP-A- 2010 241 699**

• **OCON ET AL: "Active hexose correlated compound and bifidobacterium longum BB536 exert symbiotic effect in experimentlal colitis", EUR. J. NUTR, vol. 52, 1 September 2012 (2012-09-01), - 1 September 2012 (2012-09-01), pages 457-466, XP002760222,**

**Description**

**TECHNICAL FIELD**

The present invention relates to an agent for use in treating or preventing lifestyle-related diseases, more particularly, to an agent for use in treating or preventing lifestle-related diseases, being fatty liver disease and ulcerative colitis, which can be taken daily, easily, safely, and continuously. The invention further relates to the non-therapeutic use of an agent to maintain or increase a sense of fullness.

**BACKGROUND ART**

Rapid aging of the population is progressing in the developed world as a result of rise in life expectancy accompanied by improved living standards and medical technology. Since both ageing and changing in living standards have been markedly increasing the incidence of various diseases called lifestyle-related diseases, researches on preventing or improving such diseases are being underway. There have been recommended to restrict alcohol intake, control meals, and do aerobics such as walking and jogging to prevent or improve lifestyle-related diseases; however, it would be difficult to practice them daily for an effectively long period of time under appropriate instructions. Under these circumstances, there have been progressing studies on effectively preventing or treating lifestyle-related diseases with the aid of easily and continuously ingestible edible materials, food products containing the same, and pharmaceuticals (see, for example, Non-Patent Literatures 1 to 4). Among conventionally proposed edible materials, when used as foods, etc., some might possibly have a defect of causing the reduction of flavors such as taste, odor/fragrance, and texture; some others might possibly have a defect of being effective only when ingested in a relatively large amount; or some others might possibly have a defect of inducing diarrhea depending on their body conditions or constitutions. To meet a current variety of dietary habits, desired are edible materials that effectively prevent or improve lifestyle-related diseases and that can be ingested easily and continuously without causing any reduction of the flavor and taste of food products even when incorporated therein.

Among lifestyle-related diseases, "*fatty liver*", which is associated with a daily excessive intake of alcoholic beverages and an increased intake of high fat diets due to Westernized diets, is a particularly serious social problem. Fatty liver is a condition that neutral fat is abnormally accumulated in liver cells (liver) and is defined as a condition, where the content of neutral fat becomes to be at least 5% by mass though normal internal liver part normally has a neutral fat content of 2 to 4% by mass. In addition to the above neutral fat content, fatty liver is diagnosed based on the values of glutamate oxaloacetate transaminase (GOT) and glutamate pyruvate transaminase (GPT), as well as triglyceride (TG), cholinesterase, albumin, total cholesterol, low-density lipoprotein, etc. Patients suffering from fatty liver have been recently increasing and it is said that about 40% of males and about 15% of females have such disease.

Fatty liver is categorized into alcoholic fatty liver and non-alcoholic fatty liver; among which the former, induced by an excessive intake of alcohol, may successively progress to alcoholic hepatitis, hepatic fibrosis, and hepatic cirrhosis. A series of these liver diseases are called alcoholic hepatitis, and once these disorders develop to hepatic fibrosis or hepatic cirrhosis, recovery of liver functions becomes to be extremely difficult. On the other hand, there exists high-fat-diet-fatty-liver, induced by a high fat diet intake, as non-alcoholic fatty liver and the symptom is recognized to be induced in such a manner that, when humans constantly ingest high fat diets daily or for a relatively long period of time, fats derived from high fat diets remain in the bodies without consumption and convert into neutral fat, followed by accumulation thereof. Both alcoholic fatty liver and high-fat-diet-fatty-liver are representatives of lifestyle related diseases that would develop metabolic syndrome. Accordingly, it goes without saying that lifestyle should be improved so as not to become fatty liver, and if fatty liver can be prevented or improved by daily ingestible foods or medicaments, the significance thereof is huge.

Detailed mechanism of the onset of alcoholic fatty liver and high-fat-diet-fatty-liver is unknown; however, the following are proposed: Such symptoms are induced by (1) increase of inflow of fatty acids into the body by a diet intake or (2) reduction or the like of $\beta$- and $\omega$-oxidations of fatty acids in the liver, wherein the above item (1) can be induced by an excessive intake of high fat diets and the item (2) can be caused by daily alcohol intake.

Insulin resistance improvers, eicosapentaenoic acid (EPA), betaine, choline, etc., are known as agents for preventing or improving fatty liver (see, for example, Patent-Literature 1). As far as the present inventors know, there has not yet been provided any agent for improving lifestyle-related diseases, which can be ingested by humans daily, easily, safely, and continuously; and which effectively prevents or improves fatty liver induced by lifestyle-related diseases.

Prior Art Literatures

Patent Literature

[Patent Literature 1] Japanese Patent Kokai No. 300828/92

Non-Patent Literatures

[Non-Patent Literature 1] ″Food Processing and Ingredients″, Vol. 48, No. 1, pp. 15 to 17, 2013, UBM Media Co., Ltd., Tokyo, Japan
[Non-Patent Literature 2] ″Shokumotsu-Seni, Kiso-to-Rinsho (Dietary Fiber - Foundation and Clinics), pp. 191 to 203, 1997, Asakura Publishing Co., Ltd., Tokyo, Japan
[Non-Patent Literature 3] ″Food science″, pp. 83 to 87, August, 2003, Published by Kabushiki-Gaisha Shokuhin-to-Kagaku-Sha
[Non-Patent Literature 4] ″Nihon-Chori-Kagakukai-Shi″, Vol. 42, No. 6, pp. 386 to 393, 2009, Published by The Japan Society of Cookery Science

**Summary**

Object of the Invention

The present invention has objects to provide an agent for use in treating or preventing lifestyle-related diseases, which can be taken by humans daily, easily, safely, and continuously, and is capable of effectively preventing or improving lifestyle-related diseases, particularly, alcoholic or high-fat-diet-fatty liver.

Means to Attain the Object

The present applicant has disclosed a novel branched $\alpha$-glucan in International Patent Publication No. WO2008/136331, and also disclosed that the branched $\alpha$-glucan has non-carcinogenicity, indigestibility, adequate viscosity, and improved actions of inhibiting the increment of blood sugar- and serum insulin-levels in living bodies . Also, the present applicant has disclosed that the above branched $\alpha$-glucan has an action of improving lipid metabolism in Japanese Patent Kokai No. 2010-100583. During further research on the physiological and pharmacological actions of the above branched $\alpha$-glucan, the present inventors found that the branched $\alpha$-glucan is a material with a relatively low glycemic index (GI) value and is capable of producing hydrogen gas by intestinal bacterial fermentation when the $\alpha$-glucan is ingested by humans and then a part of the water soluble dietary fiber (hereinafter abbreviated as "WSDF", unless specified otherwise) contained in the resulting ingested $\alpha$-glucan is transported to the large intestine. The present inventors also found that, after digestion of the above branched $\alpha$-glucan, humans enjoy improved bowel movements and satisfactory regulatory functions of the intestines (action of increasing the occupancy of intestinal bifid bacteria). Further, the present inventors found that, when taken by humans, the branched $\alpha$-glucan brings a sense of fullness and satisfaction and sustains the effect. As known well, GI value represents a relative value for an increasing ratio of blood-sugar level determined when a subject ingests a food material as compared to the case when the subject ingests glucose; and as disclosed, for example, in Non-Patent Literatures 3 and 4, our risk of lifestyle-related diseases is reduced when ingesting foods with a relatively low GI value as compared to the case of ingesting foods with a relatively high GI value. As disclosed in Non-Patent Literature 1, it is known that hydrogen gas, generated in the intestines by the action of intestinal bacteria, has an effect of quenching active oxygen to prevent or improve lifestyle-related diseases recognized to be induced by oxygen stress. Therefore, the above branched $\alpha$-glucan, which has a relatively low GI value and induces the formation of hydrogen gas by intestinal bacteria, is beneficial for effectively preventing or improving lifestyle-related diseases. Since the branched $\alpha$-glucan exerts improved bowel movements and satisfactory regulatory functions of the intestines when ingested, it is beneficial for effectively preventing or improving diseases such as lifestyle-related diseases inducible by constipation. When ingested, the branched $\alpha$-glucan prevents overeating and effectively prevents and improves lifestyle-related diseases because it brings a relatively high sense of fullness and satisfaction and still sustains the effect. In addition, the branched $\alpha$-glucan exerts an effect of inhibiting the development of ulcerative colitis when ingested; wherein the pathogenic mechanism of ulcerative colitis has not yet been revealed; however, causatives thereof are deemed to be changes in intestinal environment. As described above, the branched $\alpha$-glucan exerts an action of improving intestinal environment when ingested, meaning that it has also an inhibitory effect on the progress of ulcerative colitis. Based on these findings, the present inventors further continued studying and confirmed that the branched $\alpha$-

glucan has an action of effectively preventing or improving lifestyle-related diseases, particularly, fatty liver caused by the intake of high fat diets or alcoholic beverages, and thus they accomplished the present invention.

The present invention solves the above objects by providing an agent for use in treating or preventing lifestyle-related diseases selected from the group consisting of fatty liver and ulcerative colitis, which agent comprises a branched α-glucan having the following characteristics (A) to (D):

(A) being constructed by glucose molecules;
(B) having a branched structure with a glucose polymerization degree of one or higher, which is bound via a linkage except for α-1,4 linkage to a glucose residue at the non-reducing end of a linear glucan with a glucose polymerization degree of 3 or higher which has a structure of binding glucose *via* α-1,4 linkage;
(C) having a glucose polymerization degree of 6 to 430 and having a value of weight-average molecular weight (Mw) divided with number average molecular weight (Mn), Mw/Mn, being not more than 20; and
(D) having a content of water-soluble dietary fiber, obtained by applying a high performance liquid chromatography method (Enzyme-HPLC method), of 20% by mass or higher. In another aspect, the present invention provides a non-therapeutic use of an agent to maintain or increase a sense of fullness, according to claim 7.

The branched α-glucan, having the above characteristic features (A) to (D), has a relatively low GI value and induces the generation of hydrogen gas by intestinal bacteria, as well as exerting an improved effect on bowel movements and regulating the function of the intestines . Further, the branched α-glucan imparts a satisfactorily high sense of fullness and satisfaction and sustains the effect. Thus, the branched α-glucan has an action of significantly preventing or improving lifestyle-related diseases. The wording "an agent for lifestyle-related diseases" as referred to herein means an agent usable for preventing, improving, or treating lifestyle-related diseases.

The branched α-glucans, having the above characteristic features (A) to (D), are more preferably those which give the following characteristics upon methylation analysis:

(1) Ratio of 2,3,6-trimethyl-1,4,5-triacetyl-glucitol to 2,3,4-trimethyl-1,5,6-triacetyl-glucitol, is in the range of 1:0.6 to 1:4;
(2) Total content of 2,3,6-trimethyl-1,4,5-triacetyl-glucitol and 2,3,4-trimethyl-1,5,6-triacetyl-glucitol acetates is 60% or higher in the partially methylated glucitol acetates;
(3) Content of 2,4,6-trimethyl-1,3,5-triacetyl-glucitol is 0.5% or higher but not higher than 10% in the partially methylated glucitol acetates; and
(4) Content of 2,4-dimethyl-1,3,5,6-tetraacetyl-glucitol is 0.5% or higher in the partially methylated glucitol acetates.

The branched α-glucans, having the above characteristic features (1) to (4) upon methylation analysis, are quite useful as effective ingredients for agents for lifestyle-related diseases because they have lower GI values and generate hydrogen gas from intestinal bacteria when ingested to exert an action of significantly preventing or improving lifestyle-related diseases, as well as having an action of effectively preventing or improving fatty liver inducible by the intake of high fat diets or alcoholic beverages .

The agent for use in treating or preventing lifestyle-related diseases of the present invention can be used, for example, in combination with one or more ingredients selected from polyphenols, sweeteners, and high intensity sweeteners into oral compositions containing the combined mixtures. The wording "oral compositions" as referred to herein means any compositions which can be orally ingestible by humans or animals other than humans. Examples of the above polyphenols usable in combination with the agent for lifestyle-related diseases include glycosyl-rutins, glycosyl-hesperidins, glycosyl- naringins, and of which can be used alone or in two or more combinations. Preferred examples of the above sweeteners usable in combination with the agent for lifestyle-related diseases include sugar, maltose, glucose, isomerized sugar, trehalose, honey, maple sugar, sorbitol, and maltitol, one or more of which can be used alone or in combination. Examples of the above high intensity sweeteners usable in combination with the agent for lifestyle-related diseases are, for example, NEOTAME, ASPARTAME, stevia extract, enzyme treated stevia, sucralose, ACESULFAME, saccharin, thaumatin, and glycyrrhizin, one or more of which can be used alone or in combination.

The agent for use in treating or preventing lifestyle-related diseases and the oral composition containing the same according to the present invention are preferably ingested before, during, or after the intake of high fat diets, more preferably, either the agent or the oral composition should be incorporated into high fat diets and provided as high fat diets containing either of them. Since, when high fat diets contain the agent or the oral composition for use according to the present invention, users can ingest the agent or the oral composition simultaneously with high fat diets without any special consciousness, followed by obtaining an advantageous benefit of ingesting the agent or the oral composition effortlessly and continuously depending on the intake amount of high fat diets to be ingested.

Needless to say that the agent for use in treating or preventing lifestyle-related diseases, the oral composition containing the same, and the high fat diets containing any of them are preferably for use in preventing or treating fatty liver particularly among lifestyle-related diseases. When ingested, the above-mentioned branched α-glucan, contained as an effective ingredient in the agent for lifestyle-related diseases, exerts an action of inducing the generation of intestinal hydrogen gas from intestinal bacteria, the agent for lifestyle-related diseases, the oral composition containing the same, and the high fat diets containing any of them can be used as an agent for accelerating the generation of intestinal hydrogen gas . Since the above branched α-glucan, contained as an effective ingredient in the agent for lifestyle-related diseases, improves constipation and regulates the functions of the intestines (or an action of increasing the predominant ratio of intestinal bifid bacteria), the agent for lifestyle-related diseases, the oral composition containing the same, and the high fat diets containing any of them can be also used as a constipation ameliorant or an antiflatulent. When digested, the above branched α-glucan, contained as an effective ingredient in the agent for lifestyle-related diseases, brings a satisfactorily high sense of fullness and satisfaction and sustains the effect, and therefore the agent for lifestyle-related diseases, the oral composition containing the same, and the high fat diets containing any of them can be also used as a sustainer or improver for sense of fullness, or agent for lasting sense of fullness. Further, the above branched α-glucan, contained as an effective ingredient in the agent for lifestyle-related diseases, promotes the secretion of active glucagon like peptide-1 (GLP-1) when digested, the agent for lifestyle-related diseases, the oral composition containing the same, and the high fat diets containing any of them can be used as an agent for promoting the secretion of GLP-1. The above branched α-glucan, contained as an effective ingredient in the agent for lifestyle-related diseases, has an effect of inhibiting the development of ulcerative colitis when digested, the agent for lifestyle-related diseases, the oral composition containing the same, and the high fat diets containing any of them can be also used as a prophylactic or therapeutic agent for ulcerative colitis.

Effect of the Invention

The aforesaid branched α-glucan is an edible material with a relatively low glycemic index value (GI value) and, when digested, at least part of which reaches the large intestine, induces the generation of hydrogen gas through the action of fermentation by intestinal bacteria, improves constipation, and regulates the functions of the intestines. The above branched α-glucan prevents overeating because it brings a sense of fullness and satisfaction and sustains the effect when ingested. Thus, according to the agent for lifestyle-related diseases or the oral composition containing the same, lifestyle-related diseases can be effectively prevented or improved as an advantageous benefit. Further, according to the agent for lifestyle-related diseases or the oral composition containing the same, among lifestyle-related diseases, particularly alcoholic- and high fat dietic-fatty-liver can be effectively prevented or improved as an advantageous benefit. In addition, according to the agent for improving lifestyle-related diseases or the oral composition containing the same, there can be obtained an advantageous benefit that the agent or the oral composition can be ingested in an amount depending on the amount of high fat diets to be ingested while users ingest the high fat diets without any care for such combinational intake. The above branched α-glucan as the effective ingredient of the agent for lifestyle-related diseases can be enzymatically prepared from starches, partial hydrolyzates thereof, or amyloses as materials, and it is an edible material safely usable without fear of causing side effects even if it is allowed to ingest humans and animals other than humans for a relatively long period of time; the branched α-glucan can be provided stably at a lower cost and ingested by humans or animals other than humans daily, safely, easily, and continuously. The branched α-glucan facilitates continuous ingestion of oral compositions and high fat diets such as food products by humans because it improves the flavor, taste, and texture of the oral compositions and the high fat diets when incorporated thereunto.

**BRIEF DESCRIPTION OF DRAWINGS**

FIG. 1 shows a chromatogram of a branched α-glucan, obtained by the method of Production Example 1, performed on gel filtration high performance liquid chromatography (HPLC).
FIG. 2 shows a figure of halftone image for a photomicrography of a rat liver of Test group 1, after completion of feeding test, stained with hematoxylin and eosin stain (H-E stain).
FIG. 3 shows a figure of halftone image for a photomicrography of a rat liver of Test group 2, after completion of feeding test, stained with hematoxylin and eosin stain (H-E stain).
FIG. 4 shows a figure of halftone image for a photomicrography of a rat liver of Test group 3, after completion of feeding test, stained with hematoxylin and eosin stain (H-E stain).
FIG. 5 shows a figure of halftone image for a photomicrography of a rat liver of Test group 4, after completion of feeding test, stained with hematoxylin and eosin stain (H-E stain).
FIG. 6 is a graph which shows a time-dependent change of breath hydrogen-gas exhaust in a human (the horizontal axis is an elapsed time after Branched α-Glucan 1 intake and the longitudinal axis is a breath hydrogen gas yield (ppm)).

FIG. 7 is a figure which shows a questionnaire on "sense of fullness" and "satisfaction level" used in Experiments 6-1-1 and 6-2-2 (VAS: Visual Analogue Scale).

FIG. 8 is a figure which shows a time-dependent change of scores of "sense of fullness" from a subject in Experiment 6-1-1.

FIG. 9 is a figure which shows a time-dependent change of scores of "satisfaction level" from a subject in Experiment 6-1-1.

FIG. 10 is a figure which shows a time-dependent change of scores of "sense of fullness" from a subject in Experiment 6-1-2.

FIG. 11 is a figure which shows a time-dependent change of scores of "satisfaction level" from a subject in Experiment 6-1-2.

FIG. 12 is a graph which shows the production of active GLP-1 in a rat, wherein the horizontal axis "0" means before intake; "Control group", a group administered with maltodextrin; and "Test group", a group administered with a branched $\alpha$-glucan according to the present invention; and the longitudinal axis means the production of active GLP-1 (pg/ml).

## DETAILED DESCRIPTION

The branched $\alpha$-glucan used as an effective ingredient of the agent for use in treating or preventing lifestyle-related diseases in the present invention is the one disclosed in International Patent Publication No. WO2008/136331 by the same applicant as the present invention, and it has the following characteristic features (A) to (D) :

(A) being constructed by glucose molecules;
(B) having a branched structure with a glucose polymerization degree of one or higher, which is bound via a linkage except for $\alpha$-1,4 linkage to a glucose residue at the non-reducing end of a linear glucan with a glucose polymerization degree of 3 or higher which has a structure of binding glucose via $\alpha$-1,4 linkage;
(C) having a glucose polymerization degree of 6 to 430 and having a value of weight-average molecular weight (Mw) divided with number average molecular weight (Mn), Mw/Mn, being not more than 20; and
(D) having a content of water-soluble dietary fiber, obtained by applying a high performance liquid chromatography method (Enzyme-HPLC method), of 20% by mass or higher.

The branched $\alpha$-glucan used as an effective ingredient of the agent for use in treating or preventing lifestyle-related diseases of the present invention (called "the branched $\alpha$-glucan", hereinafter) is a glucan constructed by glucose as a sole constituent saccharide (Characteristic feature (A)), which has a branched structure with a glucose polymerization degree of one or higher which binds to a glucose residue at the non-reducing end, positioning at one end of a linear glucan with a glucose polymerization degree of 3 or higher that are linked together via the $\alpha$-1,4 linkage, via a linkage other than the $\alpha$-1,4 linkage (Characteristic feature (B)) . The term "a glucose residue at the non-reducing end" means a glucose residue positioning at the non-reducing end among glucan chains linked together via the $\alpha$-1,4 linkage; and the term "a linkage except for $\alpha$-1,4 linkage" means in literally a linkage other than the $\alpha$-1,4 linkage such as $\alpha$-1,2, $\alpha$-1,3, $\alpha$-1,6, $\alpha$-1,4,6 and $\alpha$-1,3,6.

The branched $\alpha$-glucan has a glucose polymerization degree of 6 to 430 and a value [(a weight-average molecular weight (Mw)) / (a number average molecular weight (Mn)] of 20 or lower, when calculated by the equation (Characteristic feature (C)), wherein the term "glucose polymerization degree", which can be determined, for example, by usual manner using gel-filtration chromatography (HPLC) or the like, means several numbers of glucose residues that constitute the glucan. The braced $\alpha$-glucan usually has a glucose polymerization degree of 6 to 430, preferably, 10 to 100, and more preferably, 25 to 75. The branched $\alpha$-glucan has a value [(a weight-average molecular weight (Mw)) / (a number average molecular weight (Mn)] of 20 or lower, when calculated by the equation, preferably, 10 or lower, and more preferably, 1 to 3.

Further, the branched $\alpha$-glucan has as a characteristic feature (D) a WSDF content of 20% by mass or higher, when determined on high-performance liquid chromatography (enzyme-HPLC method). Usually, the branched $\alpha$-glucan should preferably be those which have WSDF content of 60% by mass or higher, and more preferably, 75 to 85% by mass or higher.

The method "high-performance liquid chromatography (Enzyme-HPLC method)" for quantifying the water-soluble dietary fibers in the branched $\alpha$-glucan (called simply "Enzyme-HPLC method", hereinafter) means the one disclosed in the section "Dietary Fibers" at page eight in the section "Analytical Method, etc., for Nutritional Components (Appendix 1-3 of Nutrition Labeling Standard) in Nutrition Labeling Standard (Notification No. 146 of Ministry of Health, Labour and Welfare, May, 1996)". The outline of the above high-performance liquid chromatography method (called "Enzyme -HPLC method", hereinafter) is as follows: A sample is hydrolyzed by a series of enzyme-treatments using a thermostable $\alpha$-amylase, protease, and amyloglucosidase (or glucoamylase) . Then, proteins, organic acids, and inorganic salts are removed from the resulting enzyme-treated mixture using ion-exchange resins to make into a sample solution for high-performance liquid chromatography (HPLC). Successively, the sample solution is subjected to gel-filtration HPLC for measuring peak areas of undigested glucan and glucose in the HPLC chromatogram. Then, the WSDF content of the sample is calculated based on the peak areas and the amount of glucose in the sample solution, separately determined by conventional glucose oxidase-peroxidase method. Throughout the specification, the term "WSDF content" means a water-soluble dietary fiber content determined on the above Enzyme-HPLC method.

The branched $\alpha$-glucan is the one characterized by the above characteristic features (A) to (D) and on the other hand it is characterized by the following features (1) to (4) in terms of its binding fashion of glucoses as the constituents thereof:

(1) Ratio of 2,3,6-trimethyl-1,4,5-triacetyl-glucitol to 2,3,4-trimethyl-1,5,6-triacetyl-glucitol is in the range of 1:0.6 to 1:4;
(2) Total content of 2,3,6-trimethyl-1,4,5-triacetyl-glucitol and 2,3,4-trimethyl-1,5,6-triacetyl-glucitol acetates is 60% or higher in the partially methylated glucitol acetates;
(3) Content of 2,4,6-trimethyl-1,3,5-triacetyl-glucitol is 0.5% or higher but not higher than 10% in the partially methylated glucitol acetates; and
(4) Content of 2,4-dimethyl-1,3,5,6-tetraacetyl-glucitol is 0.5% or higher in the partially methylated glucitol acetates.

As is well known, the term "methylation analysis" as referred to herein means a generally widely used method for determining the linkages of monosaccharides that constitute poly- or oligo-saccharides. When methylation analysis is applied to the analysis of binding fashions of glucose residues in glucan, all the free hydroxyl groups of the glucose residues are first methylated and then the resulting completely methylated glucan is hydrolyzed. The resulting methylated glucoses are reduced to eliminate the anomers to obtain methylated glucitols, in which the free hydroxyl groups are acetylated to obtain partially methylated glucitol acetates, wherein the acetylated positions in "partially methylated glucitol acetates" and the expression of "glucitol acetate" are omitted and may be abbreviated as "partially methylated products". Upon gas chromatographic analysis of the resulting partially methylated products, all the partially methylated products derived from glucose residues each having a different binding fashion in glucan can be expressed with a percentage (%) of a peak area covering the peak areas of all the partially methylated products in gas chromatography. Based on the peak areas (%), there can be determined the abundance ratios of glucose residues with different binding fashions in the glucan, i.e., the abundance ratios of each glucosidic linkage.

The "ratio" for each partially methylated product means the ratio of a peak area in gas chromatogram on the methylation analysis, and "%" of each partially methylated product means "percentage of area" in gas chromatogram on the methylation analysis.

In the above (1), the definition of "2,3,6-trimethyl-1,4,5-triacetyl-glucitol" (abbreviated as "2,3,6-trimethylated product", hereinafter) corresponds to the glucose residues linked together via the $\alpha$-1,4 linkage in the branched $\alpha$-glucan, and "2,3,4-trimethyl-1,5,6-triacetyl-glucitol" (abbreviated as "2,3,4-trimethylated product", hereinafter,) corresponds to the glucose residues linked together via the 1,6 linkage. The term "a ratio of 2,3,6-trimethylated product to 2,3,4-trimethylated product is in the range of 1:0.6 to 1:4" as defined by the above (1) means that the branched $\alpha$-glucan has a ratio of the glucose residues linked together via the $\alpha$-1,6 linkage to the glucose residues linked together via the $\alpha$-1,4 linkage is in the range of "0.6 to 4". The branched $\alpha$-glucan usually has a ratio of 2,3,6-trimethylated product to 2,3,4-trimethylated product being in the range of 1:0.6 to 1:4, preferably, 1:2 to 1:4, and more preferably, 1:2.3 to 1:3.3.

In the above (2), the definition of "Total content of 2,3,6-trimethylated product and 2,3,4-trimethylated product is 60% or higher in the partially methylated products", means that, in the branched $\alpha$-glucan, the total content of the glucose residues linked together via the $\alpha$-1,4 linkage and the glucose residues linked together via the $\alpha$-1,6 linkage is 60% or higher in all the glucose residues that constitute the glucan. The branched $\alpha$-glucan usually contains 2,3,6-trimethylated product and 2,3,4-trimethylated product in a total amount of 60% or higher in the partially methylated products (or the sum of the glucose residues linked together via the $\alpha$-1,4 linkage and the glucose residues linked together via the $\alpha$-1,6 linkage is 60% or higher), preferably, 65 to 75%, and more preferably, 65 to 72%.

Similarly, in the above (3), the definition of "2,4,6-trimethyl-1,3,5-triacetyl-glucitol" (abbreviated as "2,4,6-trimethylated product", hereinafter) corresponds to the glucose residues linked together via the $\alpha$-1,3 linkage; and the definition of "Content of 2,4,6-dimethyl product is 0.5% or higher but not higher than 10%" means the branched $\alpha$-glucan usually contains the glucose residues linked together via the $\alpha$-1,3 linkage in an amount of 0.5% or higher but not higher than 10% of the total glucose residues that constitute the glucan. Preferred examples of the branched $\alpha$-glucan include those which contain 2,4,6-trimethyl product in an amount of 0.5% or higher but not higher than 10%, and more preferably, at least one percent but less than three percent of the partially methylated products.

Further, in the above (4), the definition of "2,4-dimethyl-1,3,5,6-tetraacetyl-glucitol" (abbreviated as "2,4-dimethylated product", hereinafter) corresponds to the glucose residues linked together via the $\alpha$-1,3 linkage and the $\alpha$-1,6 linkage (or the glucose residues linked together via the $\alpha$-1,3,6 linkages), and the definition of "Content of 2,4-dimethyl product is 0.5% or higher in the partially methylated products" in the above (4) means that the glucose residues linked together via the α-1,3,6 linkages are present in the branched α-glucan in an amount of 0.5% or higher of the total glucose residues that constitute the glucan. The branched α-glucan usually contains 2,4-dimethyl product in an amount of 0.5% or higher in the partially methylated products, preferably, four to nine percent, and more preferably, five to nine percent.

When subj ected to the action of isomaltodextranase (EC 3.2.1.94) having characteristic features of hydrolyzing any of α-1,2, α-1,3, α-1,4, and α-1,6 linkages adjacent to the reducing end of the isomaltose structure in glucans, the branched α-glucan forms isomaltose usually in a yield of at least 20% but not more than 50% by mass to the enzyme hydrolyzates, on a dry solid basis.

As long as the branched α-glucan has the above-identified characteristic features (A) to (D) or the characteristic features (1) to (4) upon methylation analysis, any one of which can be used in the present invention independently of its production methods; however, it is preferably produced, for example, by enzymatic reactions . In the case of producing the branched α-glucan by enzymatic reactions, it can be prepared with enzymes having an α-glucosyl-transferring action (transglycosylation) . Throughout the specification, an enzyme having an α-glucosyl-transferring action is called "a-glucosyltransferase", unless specified otherwise.

Examples of the α-glucosyltransferase used in producing the branched α-glucan include, for example, those disclosed in International Patent Publication No. WO2008/136331 applied for by the same applicant as the present invention. The aforesaid α-glucosyltransferase acts on α-glucans having a maltose and/or glucose polymerization degree of three or higher to produce the branched α-glucan by catalyzing α-glucosyl transfer without substantially hydrolyzing the α-glucans. The above α-glucosyltransferase is characteristic in that it has a relatively weak hydrolytic activity, a relatively high transferring activity independently of concentrations of substrate solutions ranging from relatively low concentrations to high concentrations, and an ability of forming not only α-1,3 linkage but α-1,3,6 linkage. Examples of enzymes with an α-glucosyl-transferring action include α-glucosidase (EC 3.2.1.20), etc. The person skilled in the art appropriately determines the additional amount, reaction time, and reaction condition of enzymes used in order to control the WSDF content, molecular distribution, and ratio of linkages other than α-1,4 linkage in the branched α-glucan.

The α-glucosyltransferase used in producing the branched α-glucan should not be restricted to the source and any commercialized ones and those isolated from microorganisms can be used, wherein such microorganisms can be recombinant microorganisms capable of producing α-glucosyltransferase. Preferable examples of the source include *Bacillus circulans* PP710 (FERM BP-10771) and *Arthrobacter globiformis* PP349 (FERM BP-10770), both of which have been deposited on February 1, 2007, in International Patent Organism, National Institute of Advanced Industrial Science and Technology, AIST Tsukuba Central 6, 1-1, Higashi 1-Chome Tsukuba-shi, Ibaraki-ken Japan, and accepted under the accession numbers of FERM BP-10771 and FERM BP-10770.

The enzymatic reaction mechanisms of α-glucosyltransferases, derived from the above *Bacillus circulans* PP710 (FERM BP-10771) and *Arthrobacter globiformis* PP349 (FERM BP-10770), are specifically disclosed in International Patent Publication No. WO2008/136331, and the α-glucosyltransferases are explained as follows:

(1) Such enzymes act on maltose and/or α-1,4 glucans having a glucose polymerization degree of three or higher as a substrate and forms α-1,4 glucans, in which a glucose residue is bound *via* α-linkage to the hydroxyl group at the C-4 or C-6 position of the non-reducing end glucose residue (an a-glucan whose glucose polymerization degree is increased by one), and an α-1,4 glucan whose glucose polymerization degree is decreased by one, by mainly transferring the non-reducing end glucose residue to the non-reducing end glucose residue of the other α-1,4 glucan by α-1,4 or α-1,6 transglucosylation;

(2) The enzymes further act on an α-1,4 glucan whose glucose polymerization degree is decreased by one, formed in the above step (1); and transfer a glucose residue to the C-4 or C-6 hydroxyl group of the non-reducing end glucose residue of the α-glucan whose glucose polymerization degree is increased by one, similarly as in the above step (1), by intermolecular α-1,4 or α-1,6 transglucosylation to elongate the glucose chain;

(3) By repeating the enzymatic reactions in the above steps (1) and (2), the above enzymes form glucans having both α-1,4 and α-1,6 linkages from maltose and/or α-glucans having a glucose polymerization degree of three or higher;

(4) Although low in incidence, the above enzymes form glucans having further α-1,3, α-1,4,6, and α-1,3,6 linkages by catalyzing α-1,3 transglucosylation and α-1,4 or α-1,3 transglucosylation to a glucose residue bound via α-1,6 linkage existing in the glucans;

(5) As a result of repeating the enzymatic reactions in the above steps (1) to (4), the branched a-glucan, in which glucoses are mainly bound via α-1,4 and α-1,6 linkages and which has α-1,3, α-1,4,6, and α-1,3,6 linkages in a low incidence, is formed by any of the above enzymes.

The branched α-glucan formed by the enzymatic reactions using enzymes such as the above-mentioned α-glucosyltransferases is usually in the form of a mixture of branched α-glucans having a various branching structures and polymerization degrees. In practicing the present invention, the branched α-glucan in such a mixture form can be used intact or used after being fractionated into fractions with a relatively uniform glucose polymerization degree, depending on its molecular weight. Accordingly, the branched α-glucan naturally includes fractions thereof fractionated with the index of molecular weight or the glucose polymerization degree of the branched α-glucan within the range of glucose polymerization degrees of 6 to 430.

The branched α-glucan formed by the above production method using the above α-glucosyltransferase possibly contains a water soluble polysaccharide having a high molecular weight ranging from 20,000,000 to 30,000,000 usually in an amount of about 0.01 to 2% by mass against the branched α-glucan. As long as the branched α-glucan has the above characteristic features (A) to (D) and the aforesaid characteristic features (1) to (4) on methylation analysis, the branched α-glucan exerts the desired effect as an effective ingredient for the agent for lifestyle-related diseases even though it contains the above-mentioned water-soluble polysaccharides. Thus, the above water-soluble polysaccharides can be fractionated or removed from the branched α-glucan using appropriate means, or such means can be even omitted when the branched α-glucan is incorporated into the agent for use in treating or preventing lifestyle-related diseases of the present invention.

The branched α-glucan can be reduced in conventional manner for use as a reduced product. The term "reduced product" means the branched α-glucan, wherein the aldehyde group of the glucose positioning at the reducing end of the saccharide is reduced into hydroxyl group. Such reduced product can be prepared according to conventional manner, for example, in such a manner of providing an aqueous solution containing a mixture of the branched α-glucans at a concentration of 30 to 60% by mass, placing the aqueous solution in an autoclave, adding Raney nickel as a catalyst in an amount of 8 to 15% by mass to the solution, heating the resulting solution to a temperature of 90 to 140°C while stirring, increasing the hydrogen pressure to 20 to 150 kg/cm$^2$ to terminate the hydrogenation of the α-glucans in the solution, removing the remaining Raney nickel from the solution, subjecting the resulting solution to purification steps such as decoloration with an activated charcoal and desalting with an ion-exchange resin, and concentrating the purified solution. Any reduced product can be prepared under a relatively low pressure in such a manner of, for example, providing a mixture of the branched α-glucans into an aqueous solution with a concentration of 30 to 50% by mass, placing the aqueous solution in an autoclave, adding Raney nickel as a catalyst in an amount of 30 to 50% by mass to the solution, heating the resulting solution to a temperature of 100 to 130°C while stirring, increasing the hydrogen pressure to 9.0 to 9.8 kg/cm$^2$ to terminate the hydrogenation of the α-glucans in the solution, removing the remaining Raney nickel from the solution, subjecting the resulting solution to purification steps such as decoloration with an activated charcoal, desalting the solution with an ion-exchange resin, and concentrating the resulting purified solution.

In the case of preparing the branched α-glucan through enzymatic reactions, the WSDF content and the molecular weight distribution of the α-glucan can be controlled by a combination reaction using α-glucosyltransferase and other well-known amylase. For example, it can be advantageously practiced in such a manner of subjecting a liquefied amylaceous solution to the actions of α-glucosyltransferase and α-amylase or cyclomaltodextrin glucanotransferase (may be called "CGTase", hereinafter), which catalyze the hydrolysis of the internal α-1,4 linkage of starch to form a newly generated glucose residue at the non-reducing end, to narrow the range of the molecular weight distribution and the viscosity of the resulting mixture, as well as increasing the ratio of linkages other than α-1,4 linkage (i.e., α-1,6 or 1,3 linkage) that contributes to the improvement of anti-retrogradation and the reduction of digestibility (improvement of the property as a water-soluble dietary fiber) of the α-glucan. By using starch debranching enzymes, such as isoamylase, in combination, the range of molecular weight distribution and the viscosity of the glucan can be reduced.

When the branching ratio of the branched α-glucan is aimed to increase or to improve the property as a water-soluble dietary fiber and the antiretrogradation, CGTase is particularly preferable for such use as an enzyme used in combination with α-glucosyltransferase; the combination use of α-glucosyltrasnferase and CGTase to act on amylaceous materials particularly increases the branching ratio (number) of the branched α-glucan. The mechanism of increment of the branching ratio (number) of branched α-glucans obtainable by the above combination use can be speculated, for example, as follows:

(1) CGTase hydrolyzes α-1,4 glucan to lower the molecular weight of the glucan to generate a new non-reducing end through the hydrolytic action of the enzyme.
(2) α-Glucosyltransferase introduces a branched structure into the branched α-glucan by transferring a glucosyl residue to the non-reducing end of α-1,4 glucan in a fashion of α-1,6 or α-1,3 glucosyltrasferation via the transferring action of the enzyme.
(3) CGTase transfers an α-1,4 glucan chain to the C-4 hydroxyl group of a glucose residue in the branched structure, introduced in the above (2), to elongate the chain length of the branched structure via the intermolecular transferring action of the enzyme.
(4) α-Glucosyltransferase transfers an additional glucosyl group to the non-reducing end of the α-1,4 glucan formed as a result of chain-length elongation and introduces an additional branched structure via α-1,6 or α-1,3 glucosyltransferation.
(5) The above reactions (1) to (4) are proceeded with the two enzymes in an indistinctly combinational manner to form a branched α-glucan with a relatively rich in α-1,6 and/or α-1,3 linkage.

The content of the branched α-glucan, contained in the agent for use in treating or preventing lifestyle-related diseases of the present invention, should not specifically be restricted; however, the agent should preferably contain the branched α-glucan in an amount of 1 to 100% by mass, preferably, 5 to 100% by mass, and more preferably, 10 to 100% by mass. The agent for use in treating or preventing lifestyle-related diseases of the present invention is preferably ingested in an amount of or administered at a dose of, usually, in the range of 0.5 to 100 g/adult (60 kg body mass)/day in terms of the WSDF content in the branched α-glucan contained in the agent. If necessary, the agent for use in treating or preventing lifestyle-related diseases of the present invention can be used in an appropriate combination ratio of one or more ingredients selected from water, salt solution, saline, proteins, peptides, polyphenols, minerals, antimicrobial substances, enzymes, indigestible polysaccharides, sweeteners (e.g., sugar, maltose, glucose, isomerized sugars, trehalose, honey, maple sugar, sorbitol, maltitol, etc.), high intensity sweeteners (e.g., NEOTAME, ASPARTAME, stevia extract, enzyme-treated stevia, sucralose, acesulfame K, saccharin, Thaumatin, glycyrrhizin, etc.), colors, flavors, starch adhesives, stabilizers, excipients/diluents/adjuvants/vehicles, fillers, pH-controlling agents, etc., one or more of which can be used in an appropriate combination in a ratio of 0.1 to 40% by mass.

The agent for use in treating or preventing lifestyle-related diseases according to the present invention containing the branched α-glucan can be used intact according to the present invention or used after adding one or more additional ingredients selected from polyphenols, sweeteners, and high intensity sweeteners to make it into oral compositions .

Polyphenols are used in the oral composition for use according to the present invention to stabilize and/or enhance the effect of the branched α-glucan as an agent for lifestyle-related diseases; preferable examples of such include polyphenols such as glycosyl-rutin, glycosyl-hesperidin, glycosyl-naringin, rutin, and hesperidin. The polyphenols can be those in an isolated form or in a composition form containing in food materials such as tea drinks including black and green teas. Any one of these polyphenols can be incorporated into the oral composition, or two or more of them can be incorporated in combination. The total amount of such polyphenols used is usually in the range of 0.01 to 99% by mass, preferably, 0.1 to 70% by mass, more preferably, 1 to 50% by mass, and further more preferably, 1 to 30% by mass. Among these polyphenols, the combination of the branched α-glucan and one or more of glycosyl-rutin, glycosyl-hesperidin, and glycosyl-naringin is a more preferable combination because it stably, continuously exerts the desired effect of preventing or improving lifestyle-related diseases, resulting in attaining a distinct effect at least additive.

Further, sweeteners and high intensity sweeteners are used in the oral composition to facilitate its intake daily, easily, and continuously. Examples of such sweeteners include one or more ingredients selected from sugar, maltose, glucose, isomerized sugar, trehalose, honey, maple sugar, sorbitol, and maltitol. These ingredients are used in practicing the present invention usually in a ratio of 0.001 to 50% by mass, preferably, 0.01 to 70% by mass, more preferably, 0.1 to 50% by mass, and more further preferably, 1 to 30% by mass in terms of their total amount.

Examples of high intensity sweeteners used in the oral composition include one or more ingredients selected from NEOTAME, ASPARTAME, stevia extract, enzyme-treated stevia, sucralose, acesulfame K, saccharin, Thaumatin, and glycyrrhizin, which are usually used in a ratio of 0.001 to 50% by mass, preferably, 0.01 to 70% by mass, more preferably, 0.1 to 50% by mass in terms of their total amount. Particularly, among the above high intensity sweeteners, NEOTAME, ASPARTAME, stevia extract, enzyme-treated stevia, sucralose, and acesulfame-K are specifically recommended in terms of their ability of facilitating the intake of the oral composition because the combination use of one or more of the sweeteners and the branched α-glucan do not spoil the desired effect of the present invention and the branched α-glucan has an effect of effectively improving the unfavorable after tastes of the listed high intensity sweeteners.

In addition to the above-mentioned ingredients, the oral composition can be appropriately incorporated with one or more materials selected from water, salt solution, saline, proteins, peptides, polyphenols, minerals, antimicrobial substances, enzymes, indigestible polysaccharides, colors, flavors, starch adhesives, stabilizers, excipients/diluents/adjuvants/vehicles, fillers, pH-controlling agents, etc., in a total ratio of 0.01 to 50% by mass, and preferably, 0.1 to 40% by mass.

The oral composition can be made into compositions with appropriate forms such as a powder, granule, liquid, paste, cream, tablet, capsule, caplet, soft capsule, lozenge, rod, plate, block, pellet/pill, solid, gel, jelly, gummy, wafer, biscuit, candy, chewable, syrup, or stick. Such compositions can be incorporated into food products such as specified health foods, dietary supplements, or health foods, as well as pharmaceuticals and quasi-drugs. Concrete examples of such food products include beverages such as a carbonated beverage, milk beverage, jelly beverage, isotonic drink, vinegar drink, soymilk drink, iron-containing beverage, lactic acid bacteria beverage, green tea, black tea, cocoa, and coffee; foods such as a cooked rice, rice gruel, bread, noodle, soup, *miso* soup, and yoghurt; confectionaries such as a soft candy, hard candy, gummy candy, jelly, cookie, rice cracker, cubic rice cracker, millet-and-rice cake, starch paste, rice cake, bracken-starch dumpling, steamed bean-jam bun, sweet rice jelly, bean jam, sweet jelly of bean, soft adzuki-bean jelly, *kingyoku* (a confectionary prepared with agar and sugar), jelly, pectin jelly, sponge cake, biscuit, cracker, pie, pudding, butter cream, custard cream, cream puff, wafer, sponge cake, pancake, muffin, doughnut, chocolate, ganache, cereal bar, chewing gum, caramel, nougat, wheat/flour paste, peanut paste, fruit paste, jam, and marmalade; edible ices such as ice cream, sherbet, and gelato; and seasonings and cooked products such as a soy sauce, powdered soy sauce, *miso* (a bean paste), powdered *miso, moromi* (a raw unrefined Japanese rice wine), salted flesh, *furikake* (a dried food sprinkled over rice), mayonnaise, dressing, vinegar, *sanbaizu* (a mixture of vinegar, soy sauce, and sugar or sweet rice wine), *funmatsu-sushi-su* (a powdered sweetened vinegar), *chukano-moto* (a seasoning for Chinese dishes), *tentsuyu* (a Japanese dipping sauce), *mentsuyu* (a seasoning soy sauce), sauce, tomato sauce, ketchup, grilled meats sauce, *yakitori* sauce (a char-broiled chicken), powder for deep-fried food, tempura flour (a flour for deep-fried seafoods and vegetables), curry roux, stew mix, soup mix, instant bouillon, seasoning and combined seasoning, *mirin* (a sweet Japanese rice wine for seasoning), *shin-mirin* (a modified *mirin*), table sugar, and coffee sugar. The oral composition can be incorporated into medicaments for preventing or treating lifestyle-related diseases; those in the form of a liquid, syrup, enteric nutrient, tablet, capsule, troche, sublingual, granule, dispersant, powder, emulsion, or aerosolized agent. Further, the oral composition can be used in pet foods and feeds ingested by animals excluding humans.

The oral composition can be optionally administered to the stomach, digestive tract, or skin tissue by peroral administration methods such as intubational and percutaneous administrations. The oral composition effectively prevents or treats various lifestyle-related diseases including fatty liver independently of its oral or peroral administration route. Accordingly, unless specified otherwise, "oral compositions" in practicing the present invention include, by definition, compositions, which are administered by parenteral administrations such as intubational and percutaneous administrations, in addition to conventional oral compositions in the usual sense.

Also, the agent for lifestyle-related diseases and the oral composition containing the same for use according to the present invention can be appropriately incorporated into high fat diets to be appropriately used as high fat diets with the agent or the oral composition. Since the high fat diets contain the branched α-glucan, they have an effect of effectively preventing or treating lifestyle-related diseases, such as fatty liver caused by the intake of high fat diets, depending on the branched α-glucan contained therein, when digested. In addition, the high fat diets retain the flavor and taste of the diets as foods, *per se,* which are incorporated with the branched α-glucan and readily ingestible, because the α-glucan does not affect the taste, flavor, texture, etc., of foods.

The term "high fat diets" as referred to in the present specification means foods in general being relatively rich in fatty acids and means those which contain fatty acids in a total amount of 5 to 100% to a food by mass. The term "fatty acids" as referred to herein is defined as fatty acids responsible for lifestyle-related diseases such as fatty liver; myristic acid, myristoleic acid, pentadecanoic acid, palmitic acid, palmitoleic acid, heptadecanoic acid, heptadecenoic acid, stearic acid, oleic acid, linoleic acid, linolenic acid, arachidonic acid, eicosenoic acid, behenic acid, and docosahexaenoic acid. Among them, saturated fatty acids such as palmitic acid and stearic acid, monounsaturated fatty acids such as myristoleic acid and oleic acid, and the like are fatty acids that are likely to cause lifestyle-related diseases such as fatty liver. The content of the above fatty acids can be determined by conventional method. Concretely, they can be quantified by the method disclosed in Japanese Industrial Standards , JISK0070 and JISK3331, and more particularly, they can be quantified by conventional methods of extracting lipids from a sample in a mixture of chloroform and methanol, and quantifying the extracted lipids; or subjecting a sample to alkaline saponification by adding an internal standard to the sample, allowing the resulting fatty acids to effect methyl esterification in total with boron trifluoride-methanol solution or the like, separatory collecting the methyl esters, and analyzing the collected methyl esters on gas chromatography.

Concrete examples of high fatty diets suitably incorporated with the agent or the oral composition for use in treating or preventing lifestyle-related diseases of the present invention include beef, pork, horse meat, mutton, chicken, fish eggs (a cod roe, herring roe, yellowtail roe, and salmon roe), fish oils (a sardine oil, herring oil, tuna oil, bonito oil, saury oil, menhaden oil, etc.), cod-liver oil, other fish oils, fish cakes, bone oil, beef tallow, lard, horse fat, shea fat, fish oil extract, livestock/fish meat processed foods (a ham, sausage, wiener, bacon, fried chicken, meatball, etc.), poultry eggs (a chicken egg, quail egg, etc.), milk, dairy products (including fermented dairy products such as yoghurts), avocado oil, linseed oil, olive oil, cacao butter, mustard oil, tung oil, rice bran oil, safflower oil, soybean oil, corn oil, canola oil, palm oil, palm kernel oil, castor oil, nut oils (a grape seed oil, jojoba oil, cotton seed oil, palm oil, earthnut oil, kapok oil, sesame oil, poppy oil, sunflower oil, etc.), breads/cakes/confectionaries rich in oils and fats (a pudding, chocolate, cookie, cracker, doughnut, french-fried potato, etc.), hamburger patty, hamburger, butter, margarine, mayonnaise, dressing, ice cream, powdered milk, Chinese food, Chinese noodle, instant noodle (an instant Chinese noodle, etc.), soup, deep-fried seafoods and vegetables, fried food, deep-fried tofu, etc.

It is said that, when humans ingest high fat diets relatively rich in fatty acids in a relatively large amount and for a relatively long period of time or at a relatively high frequency, the onset of lifestyle-related diseases such as fatty liver might possibly increases; however, by using the high fat diet for use according to the present invention, users can unintentionally ingest both the branched α-glucan contained in the diets and other ingredients such as polyphenols simultaneously with the intake of the diets; an advantageous effect that lifestyle-related diseases such as fatty liver can be effectively prevented or treated while ingesting high fat diets.

The dose of the branched α-glucan to be incorporated into the high fat diet can be appropriately determined depending on the type and the content of fatty acids contained in the intended high fat diets; the branched α-glucan is usually set to a level of at least 0.5 g/day/adult (60 kg body mass), preferably, 1 to 50 g, and more preferably, 3 to 30 g, in terms of the WSDF content in the α-glucan. More particularly, the branched α-glucan as an effective ingredient can be incorporated into a high fat diet in an amount of, usually, at least two percent by mass, preferably, at least four percent by mass, and more preferably, at least ten percent by mass to the total intake amount of the high fat diet, i.e. , the intake amount of fatty acids, in terms of the WSDF content. Of course, the agent for lifestyle-related diseases or the oral composition, which contains the branched α-glucan, can be ingested simultaneously with, before, or after the intake of a high fat diet so as to give the above ratio to the total intake amount of the high fat diet, instead of directly incorporating the α-glucan into the diet.

The branched α-glucan can be incorporated into high fat diets in an appropriate production step before or after the completion of processings of the diets, and the means for such incorporation can be any one of, for example, blending, kneading, mixing, stirring, suspending, dispersing, spraying, dissolving, coating, etc., which are generally used in the art.

In addition to the facts that the branched α-glucan contained in the agent for lifestyle-related diseases, the oral composition, and the high fat diet is a food material with a relatively low GI value, the α-glucan promotes the generation of hydrogen gas in the intestines from intestinal bacteria and reduces the oxidation stress through the removal of active oxygen, it effectively prevents and treats various lifestyle-related diseases. When ingested, the branched α-glucan imparts a relatively high sense of fullness and satisfaction to users and it also retains the effect, resulting in preventing overeating and effectively preventing or treating various lifestyle-related diseases. The timing for the ingestion of any of the above agent, oral composition, and the diets is preferably just before or simultaneously with meals at a dose ranging from 0.5 to 100 g/adult (60 kg body mass) to attain a sense of fullness. Although the factor for obtaining such a sense is not clear, it can be speculated that the later described secretion promotion of active GLP-1 is one of the possible factors. The agent for lifestyle-related diseases, the oral composition, and the high fat diet for use according to the present invention are distinctly useful when used as agents for preventing or treating lifestyle-related diseases because they effectively prevent or treat, among lifestyle-related diseases, high-fat-dietic or alcoholic fatty liver induced by an excessive intake of, particularly, high fat diets or alcoholic beverages.

The term "alcohols or alcoholic beverages" as referred to in the present specification means alcoholic beverages in general; *sake* (a Japanese rice wine), distilled spirit, beer, low-malt beer, *huangjiu* (a Chinese wine, etc.), cider, wine, fortified wine (an aperitif wine, Madeira wine, port wine, sherry wine, Marsala wine, etc.), cocktail, spirits (a brandy, gin, rhum, vodka, whiskey, etc.), liqueur, tequila, etc.

Now explaining the intake amount of the agent for lifestyle-related diseases or the oral composition with reference to, for example, sake having an alcoholic concentration of about 15%, when used as an agent for preventing or improving alcoholic fatty liver, the intake amount of ethanol is about 25 g/day when *sake* is drunk in an amount of 1 *go* (i.e., 180 ml) in a day. Inthiscase, fatty liver resulting from alcohols can be effectively prevented or treated by the oral or peroral administration of the branched α-glucan in an amount of, usually, as mentioned above, at least two percent (i.e., at least about 0.5 g), more preferably, at least five percent (i.e., at least about 1.0 g), and more preferably, at least 10% (i.e., at least about 2.5 g) in terms of the WSDF content. Either of the agent for lifestyle-related diseases and the oral composition, both of which contain the branched α-glucan, can be ingested simultaneously with, before, or after alcohol intake; or the agent or the oral composition can be incorporated into foods (i.e., side dishes, snacks, etc.), which are ingested simultaneously, before, or after the intake of alcoholic beverages, so as to make the WSDF content to be within the above range.

The following explains examples of producing the branched α-glucan. Rightfully, the process for producing the branched a-glucan is not restricted by the following examples.

1. Preparation of enzyme

1-1. Preparation Example 1

The following explains an example of preparing an α-glucosyltransferase which is used for producing the branched α-glucan. *Bacillus circulans* PP710, FERMBP-10771, was cultivated using a fermentor about 24 hours according to the method described in International Patent Application No. WO2008/136331. After completion of the cultivation, culture broth was centrifuged and the resulting culture supernatant was collected. Then, the culture supernatant was salted out by adding ammonium sulfate to give finally 80% saturation and allowing it to stand at 4°C for 24 hours. The resultant precipitates were collected by centrifuging, dissolved in 20 mM acetate buffer (pH6.0), dialyzed against the same buffer and concentrated using a membrane to make into Concentrated Crude Enzyme Solution 1. The α-glucosyltransferase activity of Concentrated Crude Enzyme Solution 1 was assayed and determined to be 200 units/ml. About 25 units/ml of α-amylase activity was also detected in Concentrated Crude Enzyme Solution 1.

1-2. Preparation Example 2

The following explains an example of preparing an α-glucosyltransferase except for the above which is used for producing the branched α-glucan. *Arthrobacter globiformis* PP349, FERM BP-10770, was cultivated using a fermentor about 24 hours according to the method described in a International Patent Application No. WO2008/136331. After completion of the cultivation, culture broth was centrifuged and the resulting culture supernatant was collected. Then, the culture supernatant was salted out by adding ammonium sulfate to give finally 80% saturation and allowing it to stand at 4°C for 24 hours. The resultant precipitates were collected by centrifuging, dissolved in 20 mM acetate buffer (pH 6.0), dialyzed against the same buffer and concentrated using a membrane to make into Concentrated Crude Enzyme Solution 2. The α-glucosyltransferase activity of Concentrated Crude Enzyme Solution 2 was assayed and determined to be 50 units/ml.

2. Production of the branched α-glucan

2-1. Production Example 1

The following explains an example of producing the branched α-glucan using Concentrated Crude Enzyme Solution 1 obtained the above Preparation Example 1. A liquefied corn starch solution (DE 3.5) with a concentration of 30.0% by mass was admixed with calcium chloride to give a final concentration of 1 mM and then cooled to 50°C. The liquefied starch solution was admixed with Concentrated Crude Enzyme Solution 1 to give the amount of α-glucosyltransferase of 11.3 units/g-dry solid starch and followed by the enzymatic reaction at pH 6.0 and 50°C for 48 hours. After heating the reaction mixture at 80°C for 60 minutes, it was cooled and filtrated. According to the conventional methods, the resulting filtrate was decolored using activated charcoal, deionized using H- and OH- form ion-exchanger resins, concentrated, and spray-dried to obtain a powdery Branched α-Glucan 1. According to the aforementioned reaction mechanism of the α-glucosyltransferase, Branched α-Glucan 1 has a branched structure with a glucose polymerization degree of one or higher, which is bound via a linkage except for α-1,4 linkage to the non-reducing end glucose residue of a linear glucan with a glucose polymerization degree of 3 or higher, which has a structure of binding glucose via α-1,4 linkage.

The molecular weight distribution of Branched α-glucan 1 was analyzed by the conventional gel-filtration HPLC. The gel-filtration HPLC was carried out by using the following conditions:

Column: "TSK GEL α-3000", produced byTosoh Corporation, Tokyo, Japan, (two columns were connected in series);
Eluent: 10 mM sodium phosphate buffer (pH 7.0)
Column temperature: 40°C
Flow rate: 0.5 ml/min
Detector: "RID-10A", a refractive index detector produced by Shimadzu Corporation, Kyoto, Japan.

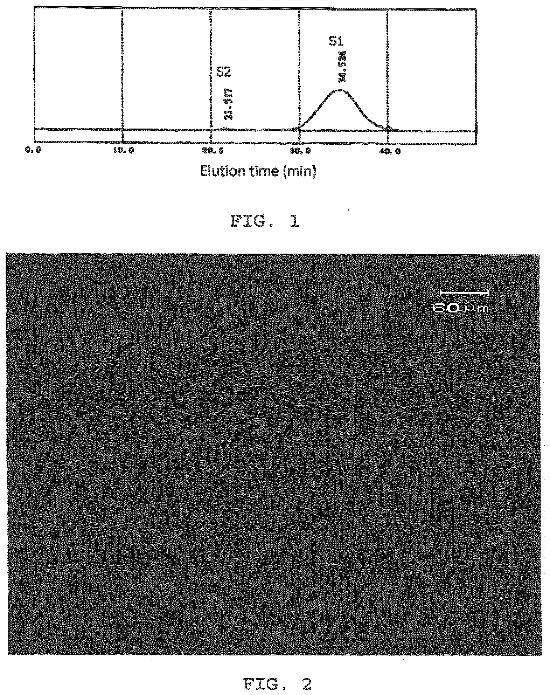

Figure 1 shows a gel-filtration HPLC chromatogram of Branched α-Glucan1. As is evident from the gel-filtrationchromatogram, Branched α-Glucan 1, obtained in Production example 1, contains a fraction of branched α-glucan (S1 fraction) and a fraction of water soluble polysaccharide (S2 fraction) with a high molecular weight. The mass ratio of S1 fraction and S2 fraction was 100:0.9. In addition, it was revealed that Branched α-Glucan 1 is a mixture of branched α-glucans with glucose polymerization degree of 6 to 210.

In order to measure the average molecular weights of the above S1 and S2 fractions, each fraction was subjected to a size exclusion chromatography and the average molecular weight of each fraction was measured by the following conditions:

Column: "ShodexSB-806M", produced by Showa Denko K. K., Tokyo, Japan;
Eluent: 10 mM sodium phosphate buffer (pH 7.0)
Flow rate: 1 ml/min
Detector: "DAWN HELEOS", a multi-angle light scattering detector produced by Wyatta Technology, USA; and "Optilab rEX", a refractive index detector produced by Wyatta Technology, USA.

The weight-average molecular weight (Mw), number-average molecular weight (Mn), and the value of Mw divided with Mn, (Mw/Mn), of S1 and S2 fractions were shown in Table 1, respectively.

Table 1

|  | S1 | S2 |
|---|---|---|
| Number-average molecular weight (Mn) | 4,345 | 22,660,000 |
| Weight-average molecular weight (Mw) | 5,042 | 23,000,000 |

(continued)

|  | S1 | S2 |
|---|---|---|
| Mw/Mn | 1.2 | 1.0 |

According to the method described in Section 8, Dietary fiber, (2) High-performance liquid chromatography (Enzyme-HPLC method), "Methods for analyzing nutritional components (Appendix 1-3 of Nutrition Labeling Standard) in Nutrition Labeling Standard (Notification No. 146 of Ministry of Health, Labor, and Welfare, May, 1996)" , the water soluble dietary fiber (WSDF) content of Branched α-Glucan 1 was determined by the following methods. "DIETARY FIBER, TOTAL ASSAY, CONTROL KIT", a kit for determining total amount of dietary fiber, commercialized by Sigma-Aldrich Japan, was used as a kit for enzymatic treatments.

To a test tube, 0.1g-dry solid of Branched α-Glucan 1 was sampled and then admixed with 5 ml of 0.08 M sodium phosphate buffer to adjust the pH to 6.0. Successively, 0.01 ml of a thermostable α-amylase (which is derived from *Bacillus licheniformis* and commercialized by Sigma-Aldrich Japan) solution, attached to the kit, was admixed with the above solution and then the tube containing the mixture was wrapped with aluminum foil, and followed by the enzyme reaction in a boiling water bath with stirring at 5 min-interval for 30min. After the reaction, the reaction mixture was cooled and adjusted the pH to 7.5 by adding 1 ml of 0.275 M sodium hydroxide solution. The resulting solution was admixed with 0.01 ml of a protease (which is derived from *Bacillus licheniformis* and commercialized by Sigma-Aldrich Japan) solution, attached to the kit, and then the tube containing the mixture was wrapped with aluminum foil, and followed by the enzyme reaction in a water bath with shaking at 60°C for 30 min, and then cooled. After adjusting the pH of the resulting solution after the protease treatment to 4.3 by adding about 1 ml of 0.325 M hydrochloric acid solution, the resulting solution was further admixed with 0. 01 ml of the amyloglucosidase (which is derived from *Aspergillus niger* and commercialized by Sigma-Aldrich Japan) solution, attached to the kit, and then the tube containing the mixture was wrapped with aluminum foil, and followed by the enzyme reaction in a water bath with shaking at 60°C for 30 min, and then cooled. Successively, about 7 ml of the resulting reaction mixture was subjected to an ion- exchange column, which is prepared by mixing "AMBERLITE™ IRA-67" (OH-form) and "AMBERLITE™ 200CT" (H-form), both commercialized by Organo Corporation, Tokyo, Japan, in a ratio of 1:1, eluted at SV 1.0 for desalting, and further eluted with about 3-folds volume of deionized water, and then filled up to the total volume of about 28 ml. The elute was concentrated using an evaporator, filtrated using a membrane filter with a pore size of 0.45 μm, and then filled up to 25 ml using a measuring flask to make into a sample solution for the analysis. The obtained test sample solution was subjected to a high performance liquid chromatography under the following conditions:

Column: "TSK gel™ G2500PWXL" (ID 7.8 mm x length 300 mm), produced by Tosoh Corporation, Tokyo, Japan; two columns were connected in series
Eluent: Deionized water
Saccharide concentration of test sample: 0.8% by mass
Column temperature: 80°C
Flow rate: 0.5 ml/min
Detector: Refractive index detector
Time for analysis: 50 min

In the chromatogram obtained by the above HPLC, undigested glucan which remained after the enzyme treatments was assumed to water soluble dietary fiber (WSDF) . The peak areas of the WSDF and D-glucose formed by the digestion were measured, respectively. Separately, the amount of D-glucose in the test sample was determined by conventional glucose oxidase-peroxidase method. Using the values of the above peak areas and the amount of D-glucose, the amount of the WSDF was calculated by the following Formula 1. Then, the WSDF content in the test sample was calculated by the following Formula 2. The WSDF content of Branched α-Glucan 1, calculated by the above Enzyme-HPLC method, was 80.0% by mass.

```
Formula 1

The amount of WSDF* (mg)

  = {(The peak area of WSDF)/(The peak area of glucose)} x (The amount

of glucose in the test sample solution)**(mg)
```

*: Water soluble dietary fiber

**: Concentration of glucose in the test sample solution (mg/ml) x 25 ml

```
Formula 2

The WSDF content (% by mass)

 = {(The amount of WSDF in the test sample) (mg)/(The amount of the test

sample)(mg)} x 100
```

As described above, Branched α-glucan, obtained in Production Example 1, was characterized as follows:

(a) being constructed by glucose molecules;
(b) having a branched structure with a glucose polymerization degree of one or higher, which is bound via a linkage except for α-1,4 linkage to the non-reducing end glucose residue of a linear glucan with a glucose polymerization degree of 3 or higher which has a structure of binding glucose via α-1,4 linkage;
(c) being a mixture of branched α-glucans with a glucose polymerization degree of 6 to 210, and its Mw/Mn is 1.2; and
(d) having a WSDF content of 80.0% by mass.

According to a conventional methylation analysis, linkages of D-glucose residues in Branched α-glucan 1 were analyzed. The partially methylated products obtained in the course of methylation analysis were subjected to the gas chromatography under the following conditions:

Column: "DB-15" (ID 0.25 mm x length 30 m, film thickness 1 μm), a capillary column produced by J&W Scientific, Tokyo, Japan;
Carrier gas: Helium
Column temperature: kept at 130°C for 2 min, heated to 250°C in a rate of 5°C/min, and then kept at 250°C for 20 min
Flow rate: 1 ml/min
Detector: FID

As a result, Branched α-glucan 1 showed the following characteristics by the methylation analysis:

(1) Ratio of 2,3,6-trimethylated product and 2,3,4-trimethylated product is 1:2.4;
(2) Total content of 2,3,6-trimethylated product and 2,3,4-trimethylated product is 70.5% in the partially methylated products;
(3) Content of 2,4,6-trimethylated product is 2.4% in the partially methylated products; and
(4) Content of 2,4-dimethylated product is 6.4% in the partially methylated products.

In order to characterize the structures of Branched α-Glucan 1, isomaltodextranase digestion was carried out. An aqueous solution of Branched α-Glucan 1 with a final concentration of 1% (w/v) was admixed with 100 units/g-solid substrate of isomaltodextranase, derived from *Arthrobacter globiformis* and prepared in Hayashibara Co., Ltd. , Okayama, Japan; and followed the enzyme reaction at pH 5.0 and 50°C for 16 hours. After stopping the reaction by keeping at 100°C for 10 min, the saccharide composition in the resulting reaction mixture was determined using high-performance liquid chromatography (HPLC) and gas chromatography (GC). HPLC was carried out under the following conditions:

Column: "MCI GEL CK04SS", produced by Mitsubishi Chemical Corporation, Tokyo, Japan; two columns were connected in series
Eluent: Water
Column temperature: 80°C
Flow rate: 0.4 ml/min
Detector: "RID-10A", a refractive index detector produced by Shimadzu Corporation, Kyoto, Japan.

GC was carried out after converting saccharides into trimethylsiliyl- derivatives (TMS-derivatives) and under the following conditions:

Column: "2% Silicon OV-17 Chromosorb W/AW-DMS", produced by GL Science, Tokyo, Japan;

Column temperature: raised 160°C to 320°C in a rate of 7.5°C/min
Carrier gas: Nitrogen
Detector: FID.

By the isomaltodextranase digestion, isomaltose in an amount of 35.0% by mass was formed from Branched α-Glucan 1. The result indicates that Branched α-Glucan 1 has an isomaltose structure in an amount of at least 35.0% by mass.

2-1. Production Example 2

Separately, the branched α-glucan was produced by the process different from Production Example 1. "PINEDEX #100", a partial starch hydrolyzate commercialized by Matsutani Chemical Industries Co., Ltd., Hyogo, Japan, was dissolved in water to give a concentration of 33% by mass and the pH of the solution was adjusted to 6.0. Concentrated Crude Enzyme Solution 2, obtained in Preparation Example 2, was admixed with the above solution to give an α-glucosyltransferase activity of 22 units/g-dry solid of substrate, and followed by the enzyme reaction at 40°C for 72 hours. After completion of the reaction, the reaction mixture was heated at 95°C for 10 minutes, cooled, and then filtrated. According to the conventional methods, the resulting filtrate was decolored using activated charcoal, deionized using H- and OH-form ion-exchanger resins, and concentrated to obtain a liquid Branched α-Glucan 2. According to the aforementioned reaction mechanism of the α-glucosyltransferase, Branched α-Glucan 2 has a branched structure with a glucose polymerization degree of one or higher, which is bound via a linkage except for α-1,4 linkage to the non-reducing end glucose residue of a linear glucan with a glucose polymerization degree of 3 or higher, which has a structure of binding glucose via α-1,4 linkage.

The molecular weight distribution of Branched α-Glucan 2 was analyzed by the similar method with Production Example 1. As a result, it was revealed that Branched α-Glucan 2 is a mixture of branched α-glucans with glucose polymerization degree of 10 to 430. The Mw, Mn and Mw/Mn of Branched α-Glucan 2 were 5,700 dalton, 2,375 dalton and 2.4, respectively.

The water soluble dietary fiber (WSDF) content of Branched α-Glucan 2, determined by the similar method with Production Example 1, was 81.0% by mass. The linkages of D-glucose residues in Branched α-Glucan 2 were analyzed by methylation analysis similarly with Production Example 1. As a result, Branched α-Glucan 2 showed the following characteristics:

(1) Ratio of 2,3,6-trimethylated product and 2,3,4-trimethylated product is 1:2.8;
(2) Total content of 2,3,6-trimethylated product and 2,3,4-trimethylated product is 68.0% in the partially methylated products;
(3) Content of 2,4,6-trimethylated product is 2.6% in the partially methylated products; and
(4) Content of 2,4-dimethylated product is 7.0% in the partially methylated products.

Further, by the isomaltodextranase digestion, isomaltose in an amount of 33.0% by mass was formed from Branched α-Glucan 2.

The following explains the present invention in more detail based on experiments.

Experiment 1

Inhibition of fatty liver caused by alcohol ingestion by the branched α-glucan

Effect of the powdery Branched a-Glucan 1, obtained in Production Example 1, on a fatty liver caused by alcohol ingestion was investigated by using fatty liver model rats induced by administrating alcohol (ethanol) . Twenty-four SD male rats (five weeks old), purchased from CLEA Japan, Inc., were divided into four groups (Test groups 1 to 4) consisting of six rats/group, and all rats were fed for one week by using "NFM", a normal feed commercialized by Oriental Yeast Co., Ltd. , Tokyo, Japan, and then fed for one week by using "F2LCW", a liquid feed with the composition in Table 2, commercialized by Oriental Yeast Co. , Ltd., Tokyo, Japan, as a preliminary feeding (feeding period: total 2 weeks) . Then, as shown in Table 2, rats in Test groups 1, 2, 3, and 4 were fed for one week with Liquid feeds A, B, C, and D, respectively. In order to habituate rats in Test groups 3 and 4 to ethanol, rats were fed by increasing the amount of ethanol in Liquid feed A in a stepwise fashion from 1% (w/v) to 5% (w/v) during one week. Successively, rats in each Test group were fed for 5 weeks using each liquid feed as a main feeding test. Rats were allowed to ingest the feed and liquid feed without any inhibition during the preliminary and the main feeding test period, and body mass and the amount of ingested feed of rat in respective Test group were measured over time. After 2 weeks or 4 weeks from the beginning of the main feeding test, rats in Test groups 1 to 4 were immediately fasted for 6 hours and the blood of each rat was collected for measuring the concentrations of TG, GOT, and GPT in serum by the conventional methods. Further, at 32 days-after from the beginning of the main feeding test, rats in Test groups 3 and 4 were fasted for 6 hours and the blood of each rat was collected for measuring the ethanol concentration in the blood. After the completion of the main feeding test for 5 weeks, all rats in Test groups were fasted for overnight and killed under ether anesthesia for sampling the blood and liver. Successively, the concentrations of TG, GOT, GPT, and free fatty acid (abbreviated as "NEFA", hereinafter) in serum, and the contents of TG, cholesterol, and NEFA in liver were measured. When livers of rats in each Test group were sampled, isolated liver was weighted and a part of liver was fixed with formalin by the conventional method. Then, hematoxylin-eosin (H-E) stained tissue specimen was prepared from the formalin-fixed liver and observed the presence of absence of fatty liver histologically. Since Branched α-Glucan 1, obtained in Production Example 1, contains 80.0% by mass of water soluble dietary fiber (WSDF), 6 grams of Branched α-Glucan contains about 5 grams of WSDF.

Table 2

| Ingredient (g/L) | Liquid feed A (Normal feed) | Liquid feed B (Normal feed + Branched α-glucan) | Liquid feed C (Normal feed + Alcohol) | Liquid feed D (Normal feed + Alcohol + Branched α-glucan) |
|---|---|---|---|---|
| Casein sodium | 41.4 | 41.4 | 41.4 | 41.4 |
| 1-Cysteine | 0.5 | 0.5 | 0.5 | 0.5 |
| d1-Methionine | 0.3 | 0.3 | 0.3 | 0.3 |
| Corn oil | 8.5 | 8.5 | 8.5 | 8.5 |
| Olive oil | 28.4 | 28.4 | 28.4 | 28.4 |
| Safflower oil | 2.7 | 2.7 | 2.7 | 2.7 |
| Vitamin mixture | 2.5 | 2.5 | 2.5 | 2.5 |
| Mineral mixture | 8.75 | 8.75 | 8.75 | 8.75 |
| Mixture of maltose and dextrin (1:1) | 115.2 | 115.2 | 25.6 | 25.6 |
| Cellulose | 10 | 10 | 10 | 10 |
| Choline bitartrate | 0.53 | 0.53 | 0.53 | 0.53 |
| xanthane gum | 3 | 3 | 3 | 3 |
| Ethanol (added when preparing the feed) | 0 | 0 | 50 | 50 |
| Branched α-Glucan* | 0 | 6 | 0 | 6 |
| Total | 221.78 | 227.78 | 182.18 | 188.18 |
| Calorie (kcal/L) | 1003 | 1027 | 995 | 1019 |
| Calorie (kcal/kg-feed) | 955 | 978 | 995 | 1019 |
| *: WSDF content in the branched α-glucan is 80.0% by mass | | | | |

The average body mass of rats at the beginning of the main feeding test (zero day) after the preliminary feeding and at 35 days-after from the beginning of the main feeding test are shown in Table 3.

Table 3

| Test group | Body mass (g)* | |
| --- | --- | --- |
| | At the beginning (Zero day) of the main feeding test (g) | 35 days-after from the beginning of the main feeding test (g) |
| Test group 1 | 324.0 ± 6.30 | 515.5 ± 15.8 |
| Test group 2 | 324.6 ± 12.3 | 543.3 ± 19.3** |
| Test group 3 | 299.3 ± 12.8** | 436.6 ± 32.5** |
| Test group 4 | 299.2 ± 13.7 | 443.6 ± 33.8 |
| *: Average ± standard deviation **: Significantly different against Test group 1 (p<0.05) | | |

As is evident from the results in Table 3, it was revealed that the body mass of rats in Test groups 2 and 4, fed by the feed admixed with Branched $\alpha$-Glucan 1, showed a almost the same or slightly increased level in comparison with those of rats in Test groups 1 and 3, fed by the feed not containing Branched $\alpha$-Glucan 1, based on the body mass changes of rats in Test group 1, fed by the normal feed; in Test group 2, fed by a feed admixed with Branched $\alpha$-Glucan 1; in Test group 3, fed by a feed admixed with alcohol; and in Test group 4, fed by a feed admixed with alcohol and Branched $\alpha$-Glucan 1. The results indicate that Branched $\alpha$-Glucan 1 is a safe material which does not inhibit the growth of living organism.

The average values of feed intake (g/day), calorie intake (kcal/day), total intake of alcohol (g), and the branched $\alpha$-glucan intake (g/day-kg mass) of rats during the main feeding test are in Table 4.

Table 4

| | Test group | | | |
| --- | --- | --- | --- | --- |
| | Test group 1 | Test group 2 | Test group 3 | Test group 4 |
| Feed intake (g/day)* | 1.10 ± 0.11 | 1.62 ± 0.13 | 1.40 ± 0.15** | 1.40 ± 0.15** |
| Calorie intake (kcal/day)* | 32.0 ± 3.6 | 58.1 ± 13.0 | 52.4 ± 8.3 | 52.4 ± 8.3 |
| Total intake of ethanol (g)* | 12.6 ± 1.6 | 22.7 ± 6.2 | 16.8 ± 3.5** | 16.8 ± 3.5** |
| Branched $\alpha$-glucan intake (g/day-kg mass)* | 10.6 ± 6.7 | 121 ± 25.7 | 86.5 ± 17.9** | 86.5 ± 17.9** |
| *: Average ± standard deviation **: Significantly different against Test group 1 (p<0.05) | | | | |

As shown in Table 4, the feed intakes of rats in Test groups 3 and 4, fed by the feed admixed with alcohol, were slightly low than those of rats in Test groups 1 and 2, fed by the normal feed. The reason of this was considered that alcohol was avoided by rats. In comparison with the average feed intake of rats in Test group 1, fed by the normal feed, and that of rats in Test group 2, fed by the normal feed admixed with Branched $\alpha$-Glucan 1; that of rats in Test group 3, fed by the feed admixed with alcohol, and that of rats in Test group 4, fed by the feed admixed with alcohol and Branched $\alpha$-Glucan 1; the average feed intakes of rats in Test groups 2 and 4, fed by the feed admixed with Branched $\alpha$-Glucan 1, were slightly larger than those of rats in Test groups 1 and 3, fed by the feed not containing Branched $\alpha$-Glucan 1. The results indicate that Branched $\alpha$-Glucan 1 is a safe material which does not cause eating disorder. Total intake of alcohol (ethanol) of rats in Test group 4, fed by the feed admixed with alcohol and Branched $\alpha$-Glucan 1, was slightly larger than that of rats in Test group 3, fed by the feed admixed with alcohol.

At the time-point elapsed for 2, 4 and 5 weeks from the beginning of the main feeding test, the concentrations of GOT and GPT in the serum of rats were measured and shown in Table 5.

Table 5

| | | Test group | | | |
|---|---|---|---|---|---|
| | | Test group 1 | Test group 2 | Test group 3 | Test group 4 |
| 2 weeks* | GOT (KU) | 72.8 ± 27.1 | 55.2 ± 6.4 | 69.4 ± 14.2 | 67.4 ± 16.9 |
| | GPT (KU) | 20.2 ± 5.4 | 17.9 ± 4.6 | 23.4 ± 4.8 | 23.0 ± 7.7 |
| 4 weeks* | GOT (KU) | 79.5 ± 16.8 | 90.3 ± 26.1 | 107.4 ± 24.6 | 107.8 ± 36.9 |
| | GPT (KU) | 19.5 ± 3.6 | 19.8 ± 4.5 | 38.0 ± 9.6 | 31.0 ± 10.3 |
| 5 weeks* | GOT (KU) | 80.9 ± 10.8 | 72.8 ± 9.74 | 366.9 ± 119** | 198.2 ± 69.6*** |
| | GPT (KU) | 12.2 ± 4.42 | 10.2 ± 1.77 | 99.0 ± 33.7** | 59.8 ± 33.7*** |
| *: Average ± standard deviation<br>**: Significantly different against Test group 1 (p<0.05)<br>***: Significantly different against Test group 3 (p<0.05) | | | | | |

As is evident from the results in Table 5, the concentrations of GOT and GPT in the serum of rats in Test groups 3 and 4, fed by the feed containing alcohol, were higher than those in Test groups 1 and 2, fed by the normal feed, at the points of 2 weeks-after, 4 weeks-after, and 5 weeks-after from the beginning of the main feeding test. Between Test groups 1 and 2, the concentrations of GOT and GPT in the serum were not so different and the effect of Branched $\alpha$-Glucan 1 was not observed. On the contrary, in Test groups 3 and 4, lowering effects of Branched $\alpha$-Glucan 1 on GOT and GPT were remarkably observed with the increasing feeding period. The results indicate that liver damages, caused and worsen by alcohol ingestion, was inhibited effectively by Branched $\alpha$-Glucan 1.

At the end-point of the main feeding test, the mass of liver and the contents of TG, cholesterol, and NEFA in liver were measured and shown in Table 6.

Table 6

| | Test group | | | |
|---|---|---|---|---|
| | Test group 1 | Test group 2 | Test group 3 | Test group 4 |
| Mass of liver (g)* | 2.78 ± 0.36 | 2.80 ± 0.16 | 3.46 ± 0.28* | 3.38 ± 0.19 |
| TG (mg/g-liver)* | 15.9 ± 7.86 | 13.1 ± 4.38 | 27.4 ± 12.2 | 13.6 ± 8.49*** |
| Cholesterol (mg/g-liver)* | 2.02 ± 0.40 | 1.80 ± 0.62 | 3.54 ± 1.74 | 2.27 ± 0.80 |
| NEFA ($\mu$Eq/liver)* | 2.09 ± 0.78 | 2.37 ± 0.95 | 2.54 ± 1.07 | 2.04 ± 0.72 |
| *: Average ± standard deviation<br>**: Significantly different against Test group 1 (p<0.05)<br>***: Significantly different against Test group 3 (p<0.05) | | | | |

As is evident from the results in Table 6, in the case of rats in Test group 3, fed by the feed containing alcohol, the average liver mass and contents of TG, cholesterol, and NEFA in liver, at the end point of the main feeding test, showed a tendency of increase in comparison with the case of rats in Test group 1, fed by the normal feed. The average liver mass and contents of TG, cholesterol, and NEFA in liver, used as index of fatty liver, of rats in Test group 3, fed by the feed containing alcohol, were increased to about 1.2 to 1.8-fold of those of rats in Test group 1, fed by the normal feed. On the contrary, in the case of rats in Test group 4, fed by the feed containing alcohol and Branched $\alpha$-Glucan 1, the contents of TG, cholesterol, and NEFA in liver were almost same levels with those of rats in Test group 1. The results indicate that Branched $\alpha$-Glucan 1 can be used effectively for inhibiting the increase of the contents of TG, cholesterol, and NEFA in liver. In the cases of rats in Test group 1, fed by the normal feed, and rats in Test group 2, fed by the normal feed admixed with Branched $\alpha$-Glucan 1, the substantial difference was not observed in the liver mass at the end point of the main feeding test. However, in the case of rats in Test group 2, the contents of TG and cholesterol in liver showed a tendency of decrease and the contents of NEFA showed a tendency of increase in comparison with the case of rats in Test group 1.

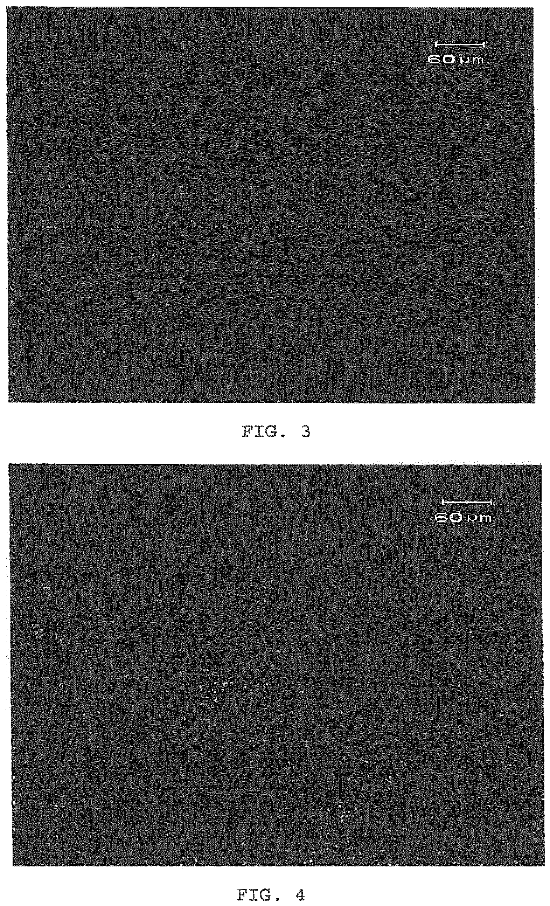

According to the histological appearance of liver of rats in each Test group, every liver of rats in Test group 3 showed ocher-like color specific to fatty liver. As is evident from halftone pictures of microscope photographs of H-E stained samples of rats in Test groups 1 to 3, respectively shown in Figs. 2 to 4, in the cases of livers of rats in Test group 3, moderate to severe (fine to large vacuolar) vacuolar degeneration (fat accumulation) were observed in entire area of lobular. Further, the degree of the fat accumulation in liver cells of rats in Test group 3 was obviously increased in comparison with the cases of Test groups 1 and 2, and fatty liver was obviously induced. However, in the case of rats in Test group 4, the liver showed normal color. As is evident from halftone pictures of microscope photographs of H-E stained samples of rats in Test groups 1, 2, and 4, respectively shown in Figs. 2, 3, and 5, in the case of rat in Test group 4, the degree of the fat accumulation in liver cells was almost same degree with those of rats in Test group 1 although fine vacuolar degeneration was observed in entire area of lobular, and the fatty liver was not induced.

From the above experimental results, it was revealed that the branched a-glucan has an activity of preventing or improving fatty liver caused by the ingestion of alcohol, effectively. Therefore, the agent for use in treating lifestyle-related diseases of the present invention and the oral composition containing the same can be used for preventing or inhibiting liver disorders such as alcoholic hepatitis, hepatic cirrhosis, etc., caused by the progression of alcoholic fatty liver, as well as alcoholic fatty liver, effectively.

Experiment 2

Inhibition of fatty liver caused by high-fat food ingestion by the branched $\alpha$-glucan

The effect of the branched $\alpha$-glucan1 on fatty liver caused by high-fat feed ingestion was investigated by using fatty liver model mice induced by administrating high-fat feed. Twenty-one C57BL/6J male mice (seven weeks old), purchased from CLEA Japan, Inc., were divided into three groups (Test groups 1 to 3) consisting of seven mice/group to give the almost equal average body mass. Mice in Test group 1 were fed for eight weeks using "CE-2", a normal feed commercialized by CLEA Japan, Inc. Mice in Test group 2 were fed for eight weeks using "High Fat diet 32 (HF32)", a high-fat feed commercialized by CLEA Japan, Inc. Distilled water was used for mice in Test groups 1 and 2 as a drinking water. Mice in Test group 3 were fed for eight weeks using the above high-fat feed and administrated a drinking water, prepared by admixing and dissolving Branched $\alpha$-Glucan 1, obtained in Production Example 1, to give a concentration of 2% by mass. The feed and drinking water were allowed to ingest without any inhibition in any case of Test groups 1 to 3. During the test period, body mass, the amount of ingested feed, and the amount of drank water of mouse in respective Test group were measured over time.

Contents of general constituents in 100 g of the above normal feed and the high-fat feed are as follows.

<Contents of general constituents in the normal feed, "CE-2">

| | |
|---|---|
| Moisture | 8.9 g |
| Crude protein | 25.4 g |
| Crude fat | 4.4 g |
| Crude ash | 4.1 g |
| Crude fiber | 6.9 g |
| Nitrogen-free extract | 50.3 g |
| Total | 100 g |

<Contents of general constituents in the high-fat feed, "HFD32">

| | |
|---|---|
| Moisture | 6.2 g |
| Crude protein | 25.5 g |
| Crude fat | 32.0 g |
| Crude ash | 4.0 g |
| Crude fiber | 2.9 g |
| Nitrogen-free extract | 29.4 g |
| Total | 100 g |

The composition of the above high-fat feed is as follows.

<Composition of the high-fat feed, "HFD32">

| | |
|---|---|
| Milk casein | 24.5% by mass |

(continued)

| | |
|---|---|
| Powdery egg whites | 5% by mass |
| L-Cystine | 0.43% by mass |
| Beef tallow (beef tallow content: 80%) | 15.88% by mass |
| Safflower oil (high-oleic acid type) | 20.00% by mass |
| Crystalline cellulose | 5.5% by mass |
| Maltodextrin | 8.25% by mass |
| Lactose | 6.928% by mass |
| Sucrose | 6.75% by mass |
| Vitamin mixture, "AIN93" | 1.4% by mass |
| Mineral mixture, "AIN93G" | 5.0% by mass |
| Choline bitartrate | 0.36% by mass |
| Tertiary-butyl hydroquinone | 0.002% by mass |
| Total | 100% by mass |

Contents of fatty acids in 100 g of the above high-fat feed are in Table 7.

Table 7

| | | Content (g) | %* |
|---|---|---|---|
| Total fatty acids | | 31.90 | 100.0 |
| Saturated fatty acid | | 7.10 | 22.3 |
| Monovalent unsaturated fatty acid | | 21.18 | 66.5 |
| Polyvalent unsaturated fatty acid | | 3.30 | 10.4 |
| Fatty acid | Myristic acid | 0.35 | 1.1 |
| | Myristoleic acid | 0.10 | 0.3 |
| | Pentdecanoic acid | 0.03 | 0.1 |
| | Palmitic acid | 4.03 | 12.6 |
| | Palmitoleic acid | 0.38 | 1.2 |
| | Heptadecanoic acid | 0.13 | 0.4 |
| | Heptadecenoic acid | 0.10 | 0.3 |
| | Stearic acid | 2.40 | 7.5 |
| | Oleic acid | 20.50 | 64.3 |
| | Linoleic acid | 3.26 | 10.2 |
| | Linolenic acid | 0.06 | 0.2 |
| | Arachidic acid | 0.10 | 0.3 |
| | Eicosenoic acid | 0.10 | 0.3 |
| | Behenic acid | 0.06 | 0.2 |
| | Unknown | 0.26 | 0.8 |
| *: Relative amount calculated by assuming the total fatty acids as 100%. | | | |

After the completion of the feeding test for 8 weeks, mice of Test groups 1 to 3 were fasted for overnight, and then killed under ether anesthesia to sample blood and liver. Successively, the contents of TG and NEFA in liver and the concentrations of GOT and GPT in serum were measured by the conventional methods. On the sampling of liver of each Test group, liver was removed, weighted, and checked the presence or absence of fat liver. The body mass of mice at the beginning and end of the feeding test were in Table 8.

Table 8

| Test group | Body mass (g)* | |
| --- | --- | --- |
| | At the beginning of feeding test | At the end of feeding test |
| Test group 1 | 22.64 ± 1.58 | 25.24 ± 2.31 |
| Test group 2 | 22.77 ± 0.55 | 39.80 ± 1.31 |
| Test group 3 | 22.76 ± 0.50 | 36.96 ± 1.74** |
| *: Average ± standard deviation  **: Significantly different against Test group 2 (p<0.05) | | |

As is evident from the results in Table 8, at the end of feeding test, the average body mass of mouse in Test group 2, fed with high-fat feed, was increased to about 1.6-fold compared with that in Test group 1, fed with normal feed. The average body mass of mouse in Test group 3, fed with high-fat feed admixed with Branched $\alpha$-Glucan 1, was significantly lowered to about 0.93-fold compared with that of Test group 2. The results indicate that the body mass increase, caused by ingesting high-fat foods, is significantly inhibited by Branched $\alpha$-Glucan 1.

The total feed intake, total calorie intake, and water intake during the feeding test were in Table 9.

Table 9

| | Test group | | |
| --- | --- | --- | --- |
| | Test group 1 | Test group 2 | Test group 3 |
| Total feed intake* (Feed intake /day) | 191.4 ± 11.5 g (3.4 g) | 154.6 ± 6.1 g (2.8 g) | 146.8 ± 2.9 g (2.6 g) |
| Total calorie intake* (Calorie intake /day) | 658.3 ± 39.4kcal (11.7 kcal) | 785.3 ± 30.8kcal (14.0 kcal) | 745.5 ± 14.7 kcal (13.2 kcal) |
| Water intake /day** | 4.9 g | 3.2 g | 3.1 g |
| *: Average ± standard deviation  **: Water, administrated to Test group 3, contains 2% by mass of Branched $\alpha$-Glucan 1. | | | |

As is evident from the results in Table 9, the total calorie intake of mice in Test group 2, fed by high-fat feed, and that of mice in Test group 3, fed by the feed containing high- fat feed and Branched $\alpha$-Glucan 1, showed a tendency of increase in comparison with mice in Test group 1, fed by normal feed, even though the total feed intake were low. Compared with mice in Test group 2, fed by high-fat feed, and those in Test group 3, fed by the feed containing high-fat feed and Branched $\alpha$-Glucan 1, those total feed intake and total calorie intake were almost the same levels. Regarding to water intake, mice in Test group 2 and Test group 3 showed lower values than that of mice in Test group 1, the levels of those were substantially the same in Test groups 2 and 3. The results indicate that Branched $\alpha$-Glucan 1 is a safe material which does not cause eating disorder.

Further, the mass of liver, the concentrations of GOT and GPT, and the contents of TG and NEFA, at the end-point of the feeding test, were measured and shown in Table 10.

Table 10

| | Test group | | |
| --- | --- | --- | --- |
| | Test group 1 | Test group 2 | Test group 3 |
| Mass of liver (g)* | 1.10 ± 0.11 | 1.62 ± 0.13 | 1.40 ± 0.15** |
| GOT (KU)* | 32.0 ± 3.6 | 58.1 ± 13.0 | 52.4 ± 8.3 |
| GPT (KU)* | 12.6 ± 1.6 | 22.7 ± 6.2 | 16.8 ± 3.5 |
| TG (mg/g-liver)* | 10.6 ± 6.7 | 121 ± 25.7 | 86.5 ± 17.9** |
| Cholesterol (mg/g-liver)* | 1.25 ± 0.43 | 3.24 ± 1.3 | 2.77 ± 1.7 |

(continued)

|  | Test group | | |
|---|---|---|---|
|  | Test group 1 | Test group 2 | Test group 3 |
| NEFA (μEq/liver)* | 3.1 ± 0.8 | 16.5 ± 8.8 | 12.4 ± 2.8 |
| *: Average ± standard deviation<br>**: Significantly different against Test group 2 (p<0.05) | | | |

As is evident from the results in Table 10, the average mass of liver of mice in Test group 3, fed by the feed containing high-fat feed and Branched α-Glucan 1, is about 86% of that of mice in Test group 2, fed by high-fat feed, and the increase of liver mass, induced by high-fat feed, was significantly inhibited. The contents of TG and NEFA in liver of mice in Test group 3 were about 72% and about 75% of those of mice in Test group 2, respectively, and the increase of the contents of TG and NEFA in liver, caused by high-fat feed, were significantly and remarkably inhibited. Similarly, the average concentrations of GOT and GPT in serum of mice in Test group 3 were about 90% and about 74% of those in Test group 2, and significantly and remarkably lowered. These results indicate that Branched α-Glucan 1 has activities of significantly and remarkably inhibiting the increase of the contents of TG and NEFA in mouse liver and the increase of the concentrations of GOT and GPT in serum, induced by the ingestion of high-fat feed.

From the results of above experiment, it was revealed that the branched α-glucan has an activity of preventing or improving fat liver caused by ingesting high-fat foods, effectively. Since the agent for lifestyle-related diseases and oral composition, which contains the branched α-glucan, can be used for preventing or lowering fat liver and body mass gain, caused by excess ingestion of high-fat foods, effectively, they can be used for preventing the metabolic syndrome and lifestyle-related diseases as well as fat liver.

Experiment 3

Inhibition of hyperlipidemia after eating caused by high-fat food ingestion by the branched α-glucan

The effect of Branched α-Glucan 1 obtained in Production Example 1 on a hyperlipidemia after eating, caused by high-fat feed ingestion, was investigated by using fatty liver model mouse induced by administrating high-fat feed. The same test system with Experiment 2 was used except for feeding period and measuring items. Twenty one C57BL/6J male mice (seven weeks old), purchased from CLEA Japan, Inc. , were divided into three groups (Test groups 1 to 3) consisting of seven mice/group to give the almost equal average body mass. Mice in Test group 1 were fed for 3 weeks using "CE-2", a normal feed commercialized by CLEA Japan, Inc. Mice in Test group 2 were fed for 3 weeks using "High Fat diet 32 (HFD32)", a high-fat feed commercialized by CLEA Japan, Inc. Distilled water was used for mice in Test groups 1 and 2 as a drinking water. Mice in Test group 3 were fed for 3 weeks using the above high-fat feed and administrated a drinking water, prepared by admixing and dissolving Branched α-Glucan 1 to give a concentration of 2% by mass. The feed and drinking water were allowed to ingest without any inhibition in any case of Test groups 1 to 3.

After the completion of the feeding test for 3 weeks, mice of Test groups 1 to 3 were fasted for overnight, then the blood was drawn from tail vein of each mouse (before the administration) . Successively, 0.2 ml of olive oil was administrated into the stomach of each mouse. Then, the blood was drawn from tail vein of each mouse over time and TG content in serum was measured, respectively. The TG contents in serum are in Table 11.

Table 11

|  | Test group | | |
|---|---|---|---|
|  | Test group 1 | Test group 2 | Test group 3 |

(continued)

| | | Test group | | |
|---|---|---|---|---|
| | | Test group 1 | Test group 2 | Test group 3 |
| Serum TG* (mg/dL) | Before the administration | 92.01 ± 16.53 | 115.71 ± 27.84 | 89.97 ± 21.42 |
| | 1 hour after the administration | 157.06 ± 39.35 | 303.99 ± 90.30** | 201.02 ± 65.31*** |
| | 2 hours after the administration | 228.73 ± 65.96 | 485.21 ± 83.36** | 266.86 ± 104.59**** |
| | 4 hours after the administration | 151.83 ± 40.11 | 141.73 ± 26.14 | 114.33 ± 41.02 |
| | 6 hours after the administration | 106.51 ± 26.26 | 101.39 ± 24.85 | 133.95 ± 43.12 |

*: Average ± standard deviation
**: Significantly different against Test group 1 ($p < 0.01$)
***: Significantly different against Test group 2 ($p < 0.05$)
****: Significantly different against Test group 2 ($p < 0.01$)

As is evident from the results in Table 11, the average TG content of mouse in Test group 3, fed with high-fat feed admixed with Branched α-Glucan 1, was significantly lower than that in Test group 2, fed with high-fat feed. From the results, it was revealed that hyperlipidemia after eating, induced by high-fat feed, was significantly inhibited by the ingestion of Branched α-Glucan 1. The average content of serum TG of rats in Test group 3, which administrated high-fat feed containing Branched α-Glucan 1, was lowered with the same level to that of rat in Test group 1, which administrated normal feed, during 6 hours after the administration of olive oil. The results indicate that Branched α-Glucan 1 has an activity of remarkably inhibiting the hyperlipidemia after eating, which induced by continuous ingestion of high-fat feed.

Experiment 4

Digestibility and absorbability of the branched α-glucan

The digestibility and absorbability of the branched α-glucan in rats and humans were investigated using Branched α-Glucan 1 produced in Production Example 1.

Experiment 4-1

Digestibility and absorbability of the branched α-glucan in rats

The digestibility and absorbability of the branched α-glucan were investigated by using rats. Five male Wister rats (seven weeks old) were preliminary fasted for overnight, and then an aqueous solution containing Branched α-Glucan 1, obtained in Production Example 1, or "PINEDEX #1", a partial starch hydrolyzate commercialized by Matsutani Chemical Industries Co, Ltd., Hyogo, Japan, was orally administrated to the rats using a gastric sonde. The dose was set to 3.3 g-solid/kg-rat body mass. At 1 hour-after, 3 hours-after, and 6 hours-after from the administration, all contents of stomach, small intestine, and intestinal cecum were withdrawn from each rat and then suspended in distilled water and extracted sample by stirring, respectively. The concentration of the sample was determined by phenol-sulfate method. The results are in Table 12 (The amount of administrated sample was assumed to 100%, and the remaining amount of the sample was expressed by % by mass).

Table 12

| | Partial starch hydrolyzate | | | Branched α-glucan | | |
|---|---|---|---|---|---|---|
| | 1 hour | 3 hours | 6 hours | 1 hour | 3 hours | 6 hours |
| Content of stomach (%) | 35.3 | 0.3 | 0 | 16.0 | 0 | 0 |
| Content of small intestine (%) | 19.9 | 1.7 | 0.6 | 44.0 | 27.8 | 5.1 |
| Content of intestinal cecum (%) | 0.6 | 0.5 | 0.6 | 0.6 | 3.5 | 13.8 |

As is evident from Table 12, it was revealed that almost all partial starch hydrolyzate was disappeared from gastrointestinal tract (stomach, small intestine and intestinal cecum) at the point of 3 hours-after from the administration.

It was suggested that almost all partial starch hydrolyzate was digested and absorbed during 3 hours after the administration. On the contrary, it was revealed that 27.8% and 3.5% of Branched $\alpha$-Glucan 1 was remained in small intestine and intestinal cecum at the point of 3 hours-after from the administration. It means that at least 31.3% of Branched $\alpha$-Glucan 1 was remained in gastrointestinal tract. It was revealed that at least about 30% of Branched $\alpha$-Glucan 1 is not digested and absorbed in gastrointestinal tract.

Experiment 4-2

Digestibility and absorbability of the branched $\alpha$-glucan in humans

Successively, the digestibility and absorbability of the branched $\alpha$-glucan in humans were investigated by using Branched $\alpha$-Glucan 1 prepared in Production Example 1. Glycemic index value (GI value) has been used as an index for evaluate the digestibility and absorbability of saccharides in humans. GI value is defined as a relative value that the degree of the increase of blood sugar concentration when 50 g of a carbohydrate is ingested, assuming the degree of the increase of blood sugar concentration when 50 g of D-glucose is ingested as 100, and is calculated according to the following Formula 3.

Formula 3

$$GI = \{(AUC^* \text{ when the test sample is administrated})/(AUC^* \text{ when D-glucose is administrated})\} \times 100$$

*: Area under the blood sugar concentration-time curve

It is known that the ingestion of foods with low GI values lowers a risk of various diseases (lifestyle-related diseases) in comparison with the case of ingesting foods with high GI values (see non-patent literatures 3 and 4) . GI value of the branched $\alpha$-glucan was measured as follows.

Effects of glucose and Branched $\alpha$-Glucan 1 on the blood sugar (glucose) level were investigated using "TOLERAN G Solution", a commercially available glucose, produced by Ajinomoto Co., Inc., Tokyo, Japan, and powdery Branched $\alpha$-Glucan 1 obtained by the method of Production Example 1. Four male and seven female, total eleven healthy volunteers were selected as subject, and the ingestion of foods except for water was prohibited from 5 hours before of the beginning of the test. First, the subjects were allowed to ingest a test solution, prepared by dissolving 50 g of glucose in 150 ml of water. Then, the blood was collected from the subjects at the times of just before the ingestion and 15, 30, 45, 60, 90, and 120 min-after the ingestion, respectively. Successively, after one week or more intervals, the same subjects were allowed to ingest another test solution, prepared by dissolving 50 g of Branched $\alpha$-Glucan in 150 ml of water, and the blood of subjects was collected in the same manner with the case of glucose. The blood sugar level of each subject was measured; and the difference of the blood sugar level over time and the area under the blood sugar level-time curve (AUC) during a period just before the ingestion to 120 min-after after the ingestion, calculated based on the difference, are in Table 13.

Table 13

| Time (minute) | Elevated blood-sugar levels (mg/dL)* | |
| --- | --- | --- |
| | D-Glucose (Control) | Branched $\alpha$-glucan |
| Just before the ingestion | 0 | 0 |
| 15 | 23.7 $\pm$ 12.4 | 11.8 $\pm$ 12.4 |
| 30 | 49.9 $\pm$ 17.4 | 28.9 $\pm$ 15.4 |
| 45 | 64.8 $\pm$ 17.8 | 34.7 $\pm$ 17.7 |
| 60 | 59.0 $\pm$ 20.9 | 31.5 $\pm$ 16.6 |
| 90 | 39.1 $\pm$ 20.7 | 25.9 $\pm$ 22.6 |
| 120 | 21.5 $\pm$ 24.3 | 10.9 $\pm$ 12.2 |
| Area under the blood sugar concentration-time curve | 82.3 $\pm$ 25.1 | 46.6 $\pm$ 19.4 |

(continued)

| Time (minute) | Elevated blood-sugar levels (mg/dL)* | |
|---|---|---|
| | D-Glucose (Control) | Branched α-glucan |
| GI value | 100 | 57 |
| *: Average ± standard deviation | | |

As shown in Table 13, the GI value of Branched α-Glucan 1 was 57. From the result, it was revealed that about 60% of Eranched α-Glucan 1 was digested and absorbed in gastrointestinal tract and about 40% of that was not. The result indicates the GI value of Branched α-Glucan is low and also suggests that various risks of diseases can be lowered and lifestyle-related diseases can be prevented or improved by ingesting the branched α-glucan.

Experiment 5

Formation of hydrogen gas by the fermentation of the branched α-glucan in large intestine

It has been known that hydrogen gas was formed when a water soluble dietary fiber reached to large intestine and was degraded and fermented by intestinal bacteria. It was reported that the formed hydrogen gas has an antioxidative potency (active oxygen-eliminating potency) and exhibits the prevention and improvement of diseases caused by oxidation stress, for example, lifestyle-related diseases, cancer, various inflammatory diseases, etc. (See Non-Patent Literature 1) It has been also known that water-soluble dietary fiber, reached to large intestine, increases beneficial bacteria, improves intestinal environment and improves bowel movement. Accordingly, in order to investigate water soluble dietary fiber in the branched α-glucan, reached to large intestine, was degraded and fermented or not, the presence or absence of hydrogen gas in the breath of human who ingested Branched α-Glucan 1, obtained in Production Example 1, was determined by the following method.

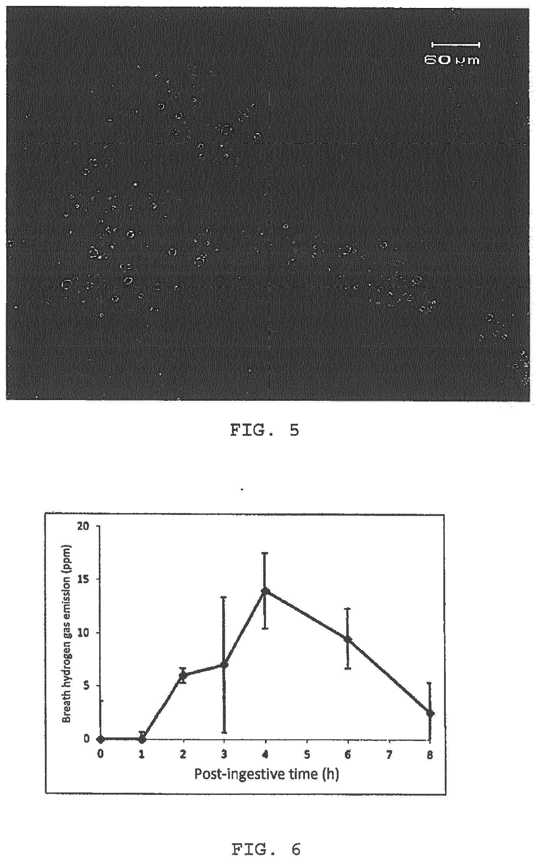

A test solution prepared by dissolving 50 g of powdery Branched α-Glucan 1, obtained in Production Example 1, in 150 ml of water was allowed to ingest to two healthy male volunteers. The breath of volunteers was collected at before ingestion, after 1, 2, 3, 4, 6, and 8 hours, respectively, and the amount of hydrogen gas in the breath was measured using "BGA-1000D", a hydrogen-methane in breath analyzer, produced by BREATHLAB Co., Ltd., Nara, Japan, with time. The results are in Fig. 6. As shown in Fig. 6, when the volunteer ingested Branched α-Glucan, hydrogen gas was formed at 2-hours after the ingestion in the breath, reached a peak at 4 -hours after, and then gradually decreased. In the case of not ingesting Branched α-Glucan 1, hydrogen gas was not detected in the breath of volunteers. Accordingly, it was revealed that a part of Branched α-Glucan 1, not digested in gastrointestinal tract, reached to large intestine and was degraded and fermented by intestinal bacteria.

The above results indicate that water-soluble dietary fiber in the branched α-glucan reached to large intestine and hydrogen gas was formed by the fermentation of the branched α-glucan by intestinal bacteria. It was revealed that the intestinal environment was improved by ingesting the branched α-glucan, and the branched α-glucan exhibited an effect of improving bowel movements. By ingesting the branched α-glucan, hydrogen gas is formed by intestinal bacteria and the formed hydrogen gas prevents and improves diseases caused by oxidation stress, for example, lifestyle-related diseases, cancer, various inflammatory diseases, etc.

Experiment 6

Effect of the branched α-glucan to improve bowel movement on humans

The effect of the ingestion of the branched α-glucan to improve bowel movement on humans was investigated by allowing humans to ingest Branched α-Glucan produced in Production Example 1. Twenty adult female, who lives in healthy regular manner and has 2 to 4 bowel movements/week, were selected as subjects.

Ten grams of powdery Branched α-Glucan was dissolved into 100 ml of water to make into a test solution, and 100 ml of water not containing Branched α-Glucan 1 was also prepared as a control solution. Above 20 subjects were divided into 2 groups containing 10 subjects/group. The subjects in the test group and those in the control group were allowed to ingest 100 ml of the test solution and 100 ml of the control solution, respectively, after each meal for 4 weeks. At the points of the beginning of ingestion and 4 weeks-after from the beginning of the ingestion, the subjects were allowed to answer a questionnaire on the following items:

(a) Number of defecated day (days/week)

(b) Number of bowel movement (times/week)
(c) Amount of feces (pieces/week, converting the amount of feces into number of pieces of L-size hen egg).

Further, intestinal bacteria in feces, collected at the beginning of ingestion and 4 weeks-after from the beginning of the ingestion, were analyzed by a conventional quantitative real-time PCR.

As the results, number of defecated day, number of bowel movement, and the amount of feces were significantly increased in the test group as follows:

(a) Number of defecated day increased from 3.3 days/week (before ingestion) to 4.4 days/week (4 weeks-after from the beginning of the ingestion);
(b) Number of bowel movement increased from 3.6 times/week (before ingestion) to 5.2 times/week (4 weeks-after from the beginning of the ingestion);
(c) Amount of feces increased from 8.2 pieces/week (before ingestion) to 12.7 pieces/week (4 weeks-after from the beginning of the ingestion) .

On the contrary, in the case of the control group, almost no difference was observed in number of defecated day, number of bowel movement, and the amount of feces between the before of the ingestion and 4 weeks-after from the beginning of the ingestion. The above experimental results indicate that the bowel movement of humans is improved by ingesting the branched α-glucan.

In the analysis of intestinal bacteria, in the case of the test group, the content of bifidobacteria in intestine was increased from 32% (before the ingestion) to 42% (4 weeks-after from the beginning of the ingestion) . On the contrary, in the case of the control group, the content of bifidobacteria in intestine was not changed during the test period. The above experimental result indicates that the ingestion of the branched α-glucan increases the content of bifidobacteria in intestine and regulates the function of intestine.

From the above results, it was revealed that the ingestion of the branched α-glucan improves bowel movement, increases the content of bifidobacteria in intestine, improves intestinal environment, and prevents and improves various diseases caused by constipation such as lifestyle-related diseases, colorectal cancer, abdominal pains, etc. Throughout the all tests, any side effect and physical deconditioning by the ingestion of the branched α-glucan were not observed to the subjects. The results indicate that the branched α-glucan can be ingested continuously and safely.

Experiment 7

Effect of the branched α-glucan on the keeping of a sense of fullness

The effect of the ingestion of the branched α-glucan on "Sense of fullness" to humans was investigatedusing Branched α-Glucan 1 produced in Production Example 1. In addition, in order to confirm one of mechanisms of providing "Sense of fullness", effect of the ingestion of the branched α-glucan on the secretion of glucagon-like peptide 1 (hereinafter, abbreviated as "GLP-1") in rat was investigated.

Experiment 7-1-1

Effect of the branched α-glucan on the keeping of a sense of fullness in humans (1)

The effect of the ingestion of the branched α-glucan on "Sense of fullness" to humans was investigated using Branched α-Glucan 1 produced in Production Example 1. Twenty-eight adults, who lives in healthy regular manner and has a habitude of eating meal at 3 times/day (morning, noon, and evening), were selected as subjects. The all subjects were allowed to complete the meal until 21 o'clock of the day before the test, get adequate sleep, and get into shape.

Fifty grams of powdery Branched α-Glucan 1, produced in Production Example 1, was dissolved into 200 ml of water to make into a test solution (hereinafter, abbreviated as "Branched Glucan Solution"), and50 gof "PINEDEX #1", powdery maltodextrin commercialized by Matsutani Chemical Industries Inc., Hyogo, Japan, was dissolved into 200 ml of water as a control solution (hereinafter, abbreviated as "Maltodextrin Solution"). Twice tests were given to each subject with an interval of 5 days or more. At one test, the subjects were allowed to ingest 200 ml of Branched Glucan Solution, and at another test, the subjects were allowed to ingest 200 ml of Maltodextrin Solution. The tests were carried out by double-blind crossover method, and under the condition where the subject does not know to ingest either solution. The subjects were allowed to drink 50 ml of water at just after the ingestion, and at every point of 30 min-interval during 2 to 3.5 hours after from the ingestion, but not allowed to take other foods and beverages. The appetite score of each subject was evaluated using VAS (Visual Analogue Scale) method as follows. The VAS method has been reported, for example, in "HIMAN-KENKYU (Studies on obesity), Japan Society for the Study of Obesity, Vol. 18, No. 1, p. 39-51 (2012).

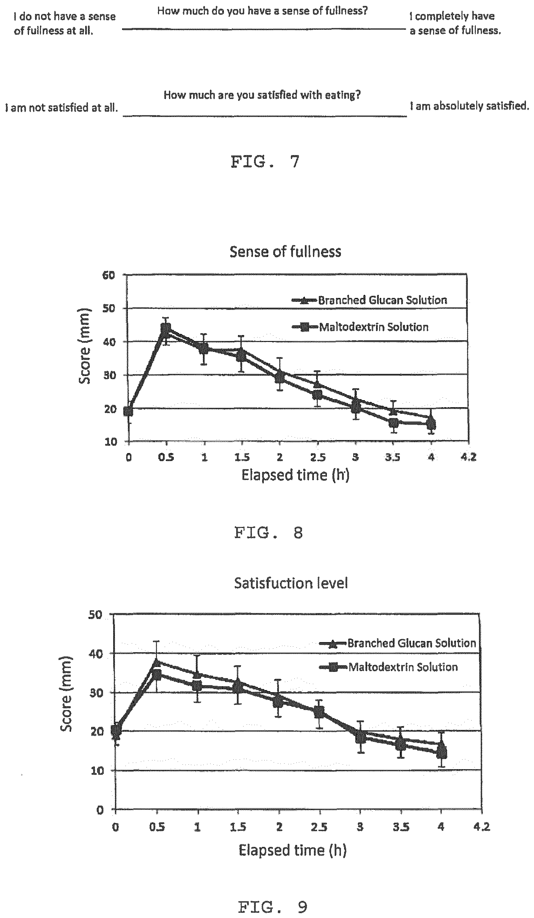

The degrees of "Sense of fullness" and "Satisfaction level" of the subjects were evaluated by using a questionnaire, a segment of a line with a length of 100 mm, according to VAS method, shown in Fig.7. Each subject was allowed to check a mark on the segment of line in Fig. 7 according to his own degrees of "Sense of fullness" and "Satisfaction level" at every 30 min-intervals during a period of just after and 4 hours-after the ingestion of Branched Glucan Solution and Maltodextrin Solution. The length (mm) of the mark from the left terminus of the segment of line (0 mm) was measured, and the degrees of "Sense of fullness" and "Satisfaction level" at each point of time were evaluated by the length as a score.

The average scores on "Sense of fullness" of the subjects were shown in Fig. 8. As is evident from Fig. 8, in the case of ingesting Branched Glucan Solution containing the branched α-glucan, the subjects showed larger sense of fullness during a period of after 1.5 to 4 hours from the ingestion, in comparison with the case of ingesting Maltodextrin Solution containing maltodextrin. In addition, the average scores on "Satisfaction level" of the subjects were also shown in Fig. 9. As is evident from Fig. 9, in the case of ingesting Branched Glucan Solution containing the branched α-glucan, the subjects showed larger degree of satisfaction during a period from just after the ingestion to 4 hours from the ingestion, in comparison with the case of ingesting Maltodextrin Solution containing maltodextrin.

Experiment 7-1-2

Effect of the branched α-glucan to maintain a sense of fullness in humans (2)

The effect of the ingestion of the branched α-glucan on "Sense of fullness" to humans was investigated by allowing subjects to ingest Branched α-Glucan 1, produced in Production Example 1, and bread at the same time. Twenty-eight adults, who lives in healthy regular manner and has a habitude of eating meal at 3 times/day (at morning, noon, and evening), were selected as subjects. The all subjects were allowed to complete the meal until 21 o'clock of the day before the test, get adequate sleep, and get into shape.

Branched Glucan Solution and Maltodextrin Solution, used in Experiment 7-1-1, were also used in this experiment. Twice tests were given to each subject with an interval of 5 days or more. At one test, the subjects were allowed to ingest 200 ml of Branched Glucan Solution and two butter rolls (product of Yamazaki Baking Co., Ltd., 99 kcal/piece, containing 3.0 g-protein, 2.5 g-fat, and 16.1 g-carbohydrate), and at another test, the subjects were allowed to ingest 200 ml of Maltodextrin Solution and the two butter rolls. In both tests, the subjects were allowed to complete the ingestion of Branched Glucan Solution or Maltodextrin Solution and two butter rolls in 5 min or later but not later than 10min from the beginning of the ingestion. The tests were carried out by double-blind crossover method, and under the condition where the subject does not know to ingest either solution. The subjects were allowed to drink 50 ml of water at just after the ingestion, and at every point of 30 min-interval during 2 to 3.5 hours after from the ingestion, but not allowed to take other foods and beverages. "Sense of fullness" and "Satisfaction level" of the subjects were evaluated by VAS method similarly in Experiment 7-1-1 at the points of every 30 min from just after the ingestion to 4 hours-after from the ingestion.

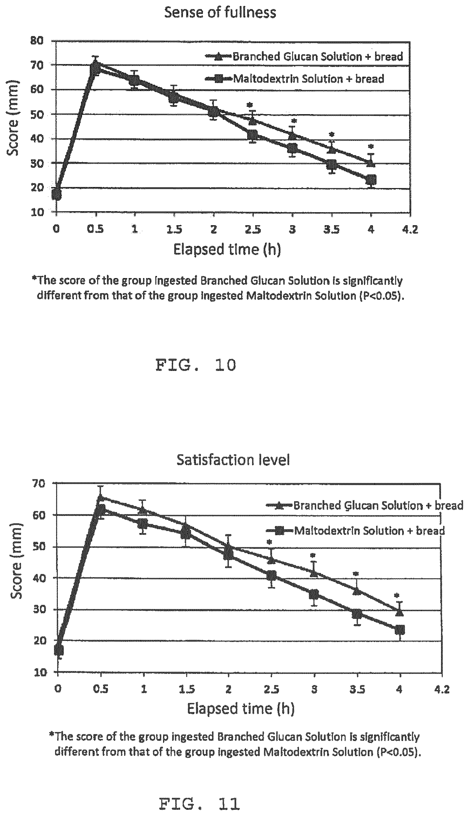

The average scores on "Sense of fullness" of the subjects were shown in Fig. 10. As is evident from Fig. 10, in the case of ingesting Branched Glucan Solution containing the branched α-glucan and bread at the same time, the subjects showed a significantly larger "Sense of fullness" during a period of after 2.5 to 4 hours from the ingestion, in comparison with the case of ingesting Maltodextrin Solution containing maltodextrin and bread at the same time. It was also revealed that, in the case of ingesting Branched Glucan Solution containing the branched α-glucan and bread at the same time, larger "Sense of fullness" tends to continue at least 4 hours from just after ingestion in comparison with the case of ingesting Maltodextrin Solution containing maltodextrin and bread at the same time. In addition, the average scores on "Satisfaction level" of the subjects were also shown in Fig. 11. As is evident from Fig. 11, in the case of ingesting Branched Glucan Solution containing the branched α-glucan and bread at the same time, the subjects showed significantly larger "Satisfaction level" during a period of after 2.5 to 4 hours from the ingestion, in comparison with the case of ingesting Maltodextrin Solution containing maltodextrin and bread at the same time. It was also revealed that, in the case of ingesting Branched Glucan Solution containing the branched α-glucan and bread at the same time, larger "Satisfaction level" tends to continue at least 4 hours from just after ingestion in comparison with the case of ingesting Maltodextrin Solution containing maltodextrin and bread at the same time.

The above results in Experiments 7-1-1 and 7-1-2 indicate that the ingestion of the branched α-glucan give larger "Sense of fullness" and "Satisfaction level" to humans in comparison with the ingestion of maltodextrin, and the effects continued for a long times. Further, the above results indicate that the ingestion of the branched a-glucan and a food such as bread or a food containing fat at the same time give larger "Sense of fullness" and "Satisfaction level" to humans in comparison with the ingestion of the branched α-glucan only, and the effects continued for a long times. Accordingly, by ingesting the branched α-glucan only or in combination with other foods and beverages, humans can lower the amount and frequency of meal and snack, prevent overeating, and lower the total calorie intake. It was also revealed that, by ingesting the branched a-glucan only or in combination with other foods and beverages, lifestyle-related diseases caused by overeating, particularly, "fatty liver" caused by the increase of the ingestion of high-fat foods or alcohol, can be effectively prevented or improved. Throughout the every test, no side effect and change of physical shape by the ingestion of the branched α-glucan were observed in subjects. The results indicate that the branched α-glucan can be ingested continuously and safely.

Experiment 7-2

Effect of the branched α-glucan on the inhibition of activated GLP-1 secretion

GLP-1, which is well-known for a hormone related to the inhibition of food intake, is divided into active GLP-1 and inactive GLP-1. It is said that especially increasing the active GLP-1 promotes increasing and maintaining "Sense of fullness" and induces the inhibition of food intake (refer to, for example, "NIPPON RINSHO (Japanese Journal of Clinical Medicine) " Vol. 69, No. 5, Kabushiki Kaisha Nippon Rinsho Sha, pp. 826-829 and pp. 946-950 (2011)). Thus, it was examined, as described below, that how the branched α-glucan affects the secretion of active GLP-1 when rats ingest it.

Sixteen Wister male rats (nine weeks old) fasted for overnight were divided into eight-by-eight two groups, and then rats in one group and in the other group were orally administrated Branched α-Glucan 1, obtained in Production Example 1, and "PINEDEX #1", a partial starch hydrolyzate commercialized by Matsutani Chemical Industries Co, Ltd. , Hyogo, Japan, by a gastric tube in an amount of 2 g/kg-body mass of each rat, respectively. The portal blood was collected from each rat under anesthesia using Somnopentyl at the times of just before ingestion and 30 min-after the ingestion. The blood plasma was obtained by transferring the blood into a plasma separating tube which was treated using EDTA-2Na and contained 500 KIU/mL of Aprotinin (#599-01283, commercialized by Wako Pure Chemical Industries, Ltd.), icing and centrifuging the resulting plasma separating tube. The resulting blood plasma was cryopreserved at -80°C immediately after adding DPP-4 inhibitor (#DPP4-010, commercialized by Millipore Corporation) in the concentration of 20 μL/mL. The amount of active GLP-1 in the blood plasma was measured using Lbis GLP-1 (active) ELISA KIT (#AKMGP-011, commercialized by Shibayagi Co., Ltd.).

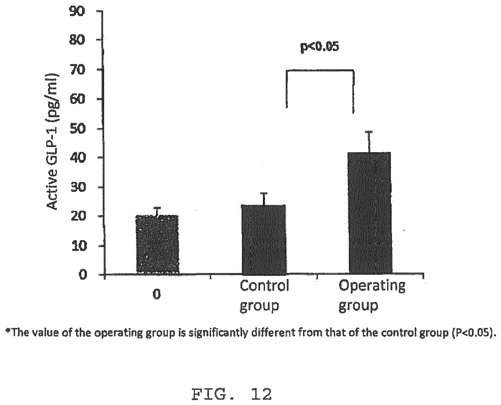

The average values of measurements at the times of just before ingestion and 30 min-after the ingestion are in Fig. 12. In Fig. 12, "0" shows the result of the measurement at the time of just before ingestion which is the total average value of the operating group and the control group. As shown in Fig. 12, the measured value of active GLP-1 in the control group ingested maltodextrin remained virtually unchanged, and the secretion of active GLP-1 was not observed. By contrast, it was indicated that the measured value of active GLP-1 in the operating group ingested the branched α-glucan was approximately twice higher than that at the time of just before ingestion, and significantly increased compared with that of the control group ingested maltodextrin.

As explained above, the increase of the secretion of active GLP-1 by ingesting the branched α-glucan can be considered one of the mechanisms of increasing "Sense of fullness" and "Satisfaction level" and inducing the inhibition of food intake.

Experiment 8

Effect of the branched α-glucan on ulcerative colitis

Effects of the ingestion of the branched α-glucan on ulcerative colitis in rats were investigated using Branched α-Glucan 1 produced in Production Example 1. In this experiment, feeding periods 1 and 2 were provided. The rats were fed by a feed composition (hereinafter referred to as "normal feed") shown in Table 14 in the feeding period 1, and then fed by a feed prepared in such a manner of allowing Sodium Dextran Sulfate (average molecular weight of 5,000, hereinafter referred to as "DSS feed") to contain in an amount of 5% by mass by replacing some usual cornstarch with DSS produced by Wako Pure Chemical Industries Ltd., Osaka, Japan, in the feeding period 2 to induce ulcerative colitis (see "J. Gastroenterology. Hepatic." Vol. 16, pp. 160-168 (2001)) . The following explain this Experiment in detail.

Table 14

| Ingredient | Amount (parts by mass) | |
| --- | --- | --- |
| | Normal feed | DSS feed |
| Casein | 146 | 146 |
| Cellulose powder | 30 | 30 |
| Corn oil | 50 | 50 |
| Mineral mixture (Product name: AIN-93G, commercialized by Oriental Yeast Co., Ltd., Tokyo, Japan) | 35 | 35 |

(continued)

| Ingredient | Amount (parts by mass) | |
|---|---|---|
| | Normal feed | DSS feed |
| Vitamin mixture (Product name: AIN-93, commercialized by Oriental Yeast Co., Ltd. Tokyo, Japan) | 10 | 10 |
| Corn starch | 729 | 679 |
| DSS | 0 | 50 |
| Total | 1000 | 1000 |

SD male rats (four weeks old), purchased from Charles River Laboratories Japan Inc., Kanagawa, Japan, were used as a test animal. At first, the rats were preliminary reared for one week while feeding "CE-2", a normal feed produced by CLEA Japan Inc., Tokyo, Japan, as that used in Experiment 2, by feeder. After being preliminary reared, rats were divided into 3 groups containing 6 rats/group, of Control group, Test group 1 and Test group 2 so that the average body mass of each group was equalized. These rats were allowed to ingest feed and beverages, as shown below, while first, it provided Feeding period 1 for 7 days and provided Feeding period 2 for 8 days as the induction period of ulcerative colitis from the end of the next day of Feeding period 1. In Feeding period 1, rats in the control group were fed by normal feed of a feed composition as shown Table 14 and fed 20 ml/day of distilled water as a beverage. On the other hand, rats in Test group 1 were fed 20 ml/day of the thing, to dissolve 1.0% by mass of Branched $\alpha$-Glucan 1 in distilled water, as a beverage, while rats were fed normal feed shown in Table 14. Rats in Test group 2 were fed 20 ml/day of the thing, to dissolve 2.0% by mass of Branched $\alpha$-Glucan 1 in distilled water, as a beverage, while rats were fed normal feed shown in Table 14. Subsequently, in "Feeding period 2", the rats was raised instead of the feed shown in Table 14 to rats in all the groups, while providing the DSS feed composition shown in Table 14, wherein, as it continues to be taken the beverage in Feeding period 1 in each group of rats.

For each group, Feeding period 2 and the next day for a total of 9 days, the symptoms of ulcerative colitis induced by DSS feed, was evaluated by symptoms of diarrhea and anemia, which were known as characteristic symptoms in ulcerative colitis.

Diarrheal symptoms were evaluated by a symptom score (Disease Activity Index: hereinafter, abbreviated to as "DAI") based on the megascopic observation of properties of feces and blood feces (see Tsubouchi et al., "Digestion" Vol. 74, pp. 91-100 (2006)). The fecal properties were evaluated by the following 4 levels:

Normal feces (score: 0);
Slightly soft feces (score: 1);
Soft feces (score: 2); and
Watery feces (score: 3).
Blood feces were evaluated by the following 4 levels based on their color:
Normal color (score: 0);
Brown (score: 1);
Browny red (score: 2); and
Bloody red (score: 3).

The total score of the fecal property and blood feces was defined to the score of diarrheal symptoms (DAI). The significant difference of DAI among the groups was statistically judged by Mann-Whitney U Test, and the value with $p < 0.05$ was judged to be significantly different. The change of score of each group and the number of survival rats of the next day of the completion of Feeding period 2 are in Table 15.

Table 15

| | Number of days elapsed after the beginning of the ingestion of DSS feed | | | | | | | | | Number of survival rats at the next day of the end of Feeding period 2 |
|---|---|---|---|---|---|---|---|---|---|---|
| | 0 | 1 | 2 | 3 | 4 | 5 | 6 | 7 | 8 | |
| Control group | 0.0 | 0.7 | 1.7 | 2.5 | 3.5 | 4.5 | 5.2 | 5.5 | 6.0 | 5 |
| Test group 1 | 0.0 | 0.7 | 1.5 | 2.7 | 2.8 | 3.0* | 3.5* | 4.2 | 4.3* | 6 |

(continued)

| | Number of days elapsed after the beginning of the ingestion of DSS feed | | | | | | | | | Number of survival rats at the next day of the end of Feeding period 2 |
| --- | --- | --- | --- | --- | --- | --- | --- | --- | --- | --- |
| | 0 | 1 | 2 | 3 | 4 | 5 | 6 | 7 | 8 | |
| Test group 2 | 0.0 | 0.3 | 1.5 | 2.5 | 2.8 | 3.2* | 3.3* | 3.7* | 4.0* | 6 |
| *: Significantly different against Control group ($p<0.05$) | | | | | | | | | | |

As is evident from Table 15, after the ingestion of DSS, all rats showed the increase of DAI, revealing the induction of ulcerative colitis. The DAI of the rats in Control group, not ingested the branched α-glucan, was 6.0 at after 8 days from the ingestion of DSS, and the rats showed watery feces including blood feces and one dead rat was observed. On the contrary, the DAI of rats in Test groups 1 and 2 showed significantly lower values than that of Control group at after 5 days from the ingestion of DSS, and dead rat was not observed. The results indicate that the branched α-glucan has an activity of improving the diarrheal symptoms (properties of feces, blood feces) specific to ulcerative colitis.

Successively, each survival rat in Control group and Test group was killed and dissected under anesthesia, and then the blood was collected from abdominal large vein and the hematocrit value (%) as an index of anemia was measured by conventional microhematocrit method. The hematicrit value of Control group was 33.8%, but that of Test group 2, ingesting 2.0% by mass of the branched α-glucan, was 44.5% and the lowering of the hematocrit value was significantly inhibited in Test group 2 in comparison with Control group. Since it was reported that the normal hematocrit value of rat is, usually, 43 to 49%, it was revealed that the hematcrit value of Test group 2 was in a normal level. Accordingly, it was confirmed that the branched α-glucan has an activity of improving the lowering of hematocrit value as an index of anemia specific to ulcerative colitis. The above results indicate that the branched α-glucan has an activity of inhibiting the advance of ulcerative colitis and can be used for preventing and treating ulcerative colitis in humans.

The following examples illustrate products useful in the practice of the invention.

Example 1

<Agent for lifestyle-related diseases>

Five-gram aliquots of powdery Branched α-Glucan 1, obtained by the method in Production Example 1, were filled into polyethylene containers and sealed to make into an agent for lifestyle-related diseases.

By ingesting the product, it exhibits bowel movement-improving effect, lasts sense of fullness, and is able to prevent or improve lifestyle-related diseases, fatty liver, and ulcerative colitis, etc. Although the intake of the product varies depending on the total intake of a high-fat food or an alcohol, the product can be taken directly, by dissolving into a beverage such as water, tea, and coffee, or by admixing into a high-fat food or the alcohol in the range of, usually, about 0.5 to about 100 g/day/adult (60kg body mass) in such a manner of allowing the branched α-glucan to be orally taken in an amount of 2% by mass or higher against the total intake of the high-fat food or the alcohol in terms of water soluble dietary fiber of the powder. It is natural that the product can be taken before or after taking the high-fat food or the alcohol. The product is stable for one or more years without absorbing moisture and discoloring under a room temperature.

Example 2

<Agent for lifestyle-related diseases>

Five-gram aliquots of a fraction, which had been prepared by removing water soluble polysaccharide polymer from powdery Branched α-Glucan 1 obtained by the method in Production Example 1 by conventional manner, were filled into polyethylene containers and sealed to make into an agent for lifestyle-related diseases.

By ingesting the product, it exhibits bowel movement-improving effect, lasts sense of fullness, and is able to prevent or improve lifestyle-related disease, fatty liver, and ulcerative colitis, etc. Although the intake of the product varies depending on the total intake of a high-fat food or an alcohol, the product can be taken directly, by dissolving into a beverage such as water, tea, and coffee, or by admixing into high-fat food or the alcohol in the range of, usually, about 0.5 to about 100 g/day/adult (60 kg body mass) in such a manner of allowing the branched α-glucan to be orally taken in an amount of 2% by mass or higher against the total intake of the high-fat food or the alcohol in terms of water soluble dietary fiber of the powder. It is natural that the product can be taken before or after taking the high-fat food or the alcohol.

The product is stable for one or more years without absorbing moisture and discoloring under a room temperature.

Example 3

<Agent for lifestyle-related diseases>

Branched α-Glucan 2 solution obtained by the method in Production Example 2 was filtrated by conventional manner and filled aseptically into a 100 ml bottle to obtain a liquid agent for lifestyle-related diseases.
By ingesting the product, it exhibits bowel movement-improving effect, lasts sense of fullness, and is able to prevent or improve lifestyle-related diseases, fatty liver, and ulcerative colitis, etc. Although the intake of the product varies depending on the total intake of a high-fat food or an alcohol, the product can be taken directly, to mix the beverages such as water, tea, and coffee, or to add the high-fat food or the alcohol in the range of, usually, about 0.5 to about 100 g/day/adult (60 kg body mass) in such a manner of allowing the branched α-glucan to be orally taken in an amount of 2% by mass or higher against the total intake of the high-fat food or the alcohol in terms of water-soluble dietary fiber of the powder. It is natural that the product can be taken before or after taking the high-fat food or the alcohol. This product is stable for one or more years without absorbing moisture and discoloring under a room temperature.

Example 4

<Agent for lifestyle-related diseases>

At first, a concentrated crude enzyme solution of an α-glucosyltransferase, obtained by the method in Preparation Example 1, was purified by the method in Experiment 6 of "International Patent Publication No. WO 2008/136331" until it was a single band on a polyacrylamide gel electrophoreses; a purified α-glucosyltransf erase was prepared. A 30% tapioca starch suspension was adjusted to pH 6.5, admixed with 0.2 % by mass/g-starch of an α-amylase which was produced by Novo Industries A/S, Copenhagen, Denmark, and then incubated at 95°C for 15 minutes. After autoclaving at 120°C for 10 minutes, the reaction mixture was cooled to 40°C. The resulting mixture was admixed with 10 units/g-dry solid of the purified α-glucosyltransferase obtained in above, and followed by the enzymatic reaction at pH 6.0 and 40°C for 24 hours. After heating the reaction mixture at 95°C for 10 minutes, it was cooled and filtrated. According to conventional manner, the resulting filtrate was decolored using an activated charcoal, deionized using H- and OH-form ion-exchange resins, concentrated, and spray-dried to obtain a branched α-glucan powder. Methylation analysis of the branched α-glucan gave a 2,3,4-trimethylated product and 2,3,6-trimethylated product ratio of 1:0.6, and a total content of 2,3,4-trimethylated product and 2,3,6-trimethylated product of 80.0% in the resulting partially methylated products thereof. Further, the contents of 2,3,4-trimethylated product and 2,4-dimethylated product were respectively 1.1% and 0.8% in the resulting partially methylated products thereof. The branched α-glucan was a mixture of 6 to 210 of glucose polymerization degree. The branched α-glucan had a weight-average molecular weight of 97,450 daltons and a number average molecular weight of 5,530 daltons, and the value of dividing the weight-average molecular weight with the number average molecular weight (Mw/Mn) was 17.6. In addition, a water-soluble dietary fiber content of the branched α-glucan, measured by the Enzyme-HPLC method, was 40.1% by mass.
By ingesting the product, it exhibits bowel movement-improving effect, lasts sense of fullness, and is able to prevent or improve lifestyle-related diseases, fatty liver, and ulcerative colitis, etc. Although the intake of the product varies depending on the total intake of a high-fat food or an alcohol, the product can be taken directly, by admixing into a beverages such as water, tea, and coffee, or by admixing into a high-fat food or alcohol in the range of, usually, about 0.5 to about 100 g/day/adult (60 kg body mass) in such a manner of allowing the branched α-glucan to be orally taken in an amount of 2% by mass or higher against the total intake of the high-fat food or the alcohol in terms of water soluble dietary fiber of the powder. It is natural that the product can be taken before or after taking the high-fat food or the alcohol.

Example 5

<Agent for lifestyle-related diseases>

A tapioca starch suspension with a concentration of 30% by mass was adjusted topH6.5, admixed with 0.2%/g-starch of an α -amylase produced by Novo Industries A/S, Copenhagen, Denmark, and then incubated at 95°C for 15 minutes. After autoclaving at 120°C for 10 minutes, the reaction mixture was cooled to 40°C. The resulting mixture was admixed with both 10 units/g-dry solid of the purified α-glucosyltransferase obtained in Example 4 and 0.1 units/g-dry solid of a CGTase derived from *Bacillus stearothermophilus* produced by Hayashibara Co., Ltd., Okayama, Japan, then followed by the enzymatic reaction at pH 6.0 and 40°C for 24 hours. After heating the reaction mixture at 95°C for 10 minutes, it was cooled and filtrated. According to conventional manner, the resulting filtrate was decolored using an activated charcoal, deionized using H- and OH-form ion-exchange resins, concentrated, and spray-dried to obtain a branched $\alpha$-glucan powder. Methylation analysis of the branched $\alpha$-glucan gave a 2,3,4-trimethylated product and 2,3,6-trimethylated product ratio of 1:1.6, and a total content of 2,3,4-trimethylated product and 2,3,6-trimethylated product of 72.0% in the resulting partially methylated products thereof. Further, the contents of 2,4,6-trimethylated product and 2,4-dimethylated product were respectively 1.4% and 1.7% in the resulting partially methylated products thereof. The branched $\alpha$-glucan was a mixture of glucans with a glucose polymerization degree of 6 to 62. The branched $\alpha$-glucan had a weight-average molecular weight of 32,830 daltons and the number average molecular weight of 4,730 daltons, and the value of dividing the weight-average molecular weight with the number average molecular weight (Mw/Mn) was 6.9. In addition, a water soluble dietary fiber content of the branched $\alpha$-glucan, measured by the Enzyme-HPLC method, was 57. 5 % by mass.

By ingesting the product, it exhibits bowel movement-improving effect, lasts sense of fullness, and is able to prevent or improve lifestyle-related diseases, fatty liver, and ulcerative colitis, etc. Although the intake of the product varies depending on the total intake of a high-fat food or an alcohol, the product can be taken directly, by admixing into a beverage such as water, tea, and coffee, or by admixing into a high-fat food or alcohol in the range of, usually, about 0.5 to about 100 g/day/adult (60 kg body mass) in such a manner of allowing the branched $\alpha$-glucan to be orally taken in an amount of 2% by mass or higher against the total intake of the high-fat food or the alcohol in terms of water soluble dietary fiber of the powder. It is natural that the product can be taken before or after taking the high-fat food or the alcohol.

Example 6

<Agent for lifestyle-related diseases>

Except for using a commercially available $\alpha$-glucosidase, instead of an $\alpha$-glucosyltransferase derived from *Aspergillus niger,* a branched $\alpha$-glucan powder was prepared according to the method in Example 5. Methylation analysis of the branched $\alpha$-glucan gave a 2,3,4-trimethylated product and 2,3,6-trimethylated product ratio of 1:0.9, and a total content of 2,3,4-trimethylated product and 2,3,6-trimethylated product of 60.0% in the resulting partially methylated products thereof. Further, the contents of 2,4,6-trimethylated product and 2,4-dimethylated product were respectively 0.7% and 0.8% in the resulting partially methylated products thereof. The branched $\alpha$-glucan was a mixture of glucans with a glucose polymerization degree of 6 to 62. The branched $\alpha$-glucan had a weight-average molecular weight of 860 daltons and a number average molecular weight of 470 daltons, and the value of dividing the weight-average molecular weight with the number average molecular weight (Mw/Mn) was 1.8. In addition, a water-soluble dietary fiber content of the branched $\alpha$-glucan, measured by the Enzyme-HPLC method, was 21.5% by mass.

By ingesting the product, it exhibits bowel movement -improving effect, lasts sense of fullness, and is able to prevent or improve lifestyle-related diseases, fatty liver, and ulcerative colitis, etc. Although the intake of the product varies depending on the total intake of a high-fat food or an alcohol, the product can be taken directly, by admixing into a beverage such as water, tea, and coffee, or by admixing into a high-fat food or alcohol in the range of, usually, about 0.5 to about 100 g/day/adult (60 kg body mass) in such a manner of allowing the branched $\alpha$-glucan to be orally taken in an amount of 2% by mass or higher against the total intake of the high-fat food or the alcohol in terms of water soluble dietary fiber of the powder. It is natural that the product can be taken before or after taking the high-fat food or the alcohol.

Example 7

<Oral composition>

Both nine grams of powdery Branched $\alpha$-Glucan 1 obtained by the method in Production Example 1 and one gram of "HAYASHIBARA HESPERIDIN S", a product name of a glucosyl-hesperidin, produced by Hayashibara Co., Ltd., Okayama, Japan, were uniformly mixed, 5 g aliquots of the resulting mixture were filled into polyethylene containers and sealed to make into a powdery oral composition of the present invention.

By ingesting the product, it exhibits bowel movement-improving effect, lasts sense of fullness, and is able to prevent or improve lifestyle-related diseases, fatty liver, and ulcerative colitis, etc. In addition to the liver protective effect of the glucosyl- hesperidin; this product exhibits an effect of the branched $\alpha$-glucan, it is useful as the composition for preventing or improving fatty liver caused by a high-fat food or an alcohol. Although the intake of the product varies depending on the total intake of the high-fat food or the alcohol, the product can be taken directly, by dissolving into a beverage such as water, tea, and coffee, or by admixing into a high-fat food or alcohol in the range of, usually, about 0.5 to about 100 g/day/adult (60 kg body mass) in such a manner of allowing the branched $\alpha$-glucan to be orally taken in an amount of 2% by mass or higher against the total intake of the high-fat food or the alcohol in terms of water soluble dietary fiber of the powder.

Example 8

<Oral composition>

Ten grams of Branched α-Glucan 2 solution obtained by the method in Production Example 2, 2 g of "HAYASHIBARA HESPERIDIN S", a product name of a glucosyl-hesperidin, produced by Hayashibara Co., Ltd., Okayama, Japan, and 0.05 g of aspartame were added in 100 ml of a refined water, mixed, and dissolved. Fifty ml aliquots of the resulting solution were micro-filtrated and filled into aseptic containers to make into a liquid oral composition.

By ingesting the product, it exhibits bowel movement-improving effect, lasts sense of fullness, and is able to prevent or improve lifestyle-related diseases, fatty liver, and ulcerative colitis, etc. In addition to the river protective effect of the glucosyl-hesperidin; the product exhibits an effect of the branched α-glucan, it is useful as the composition for preventing or improving fatty liver caused by a high-fat food or an alcohol. Although the intake of the product varies depending on the total intake of the high-fat food or the alcohol, this product can be taken in the range of, usually, about 10 to 500 ml/day/adult (60 kg body mass) in such a manner of allowing the branched α-glucan to be orally taken in an amount of 2% by mass or higher against the total intake of the high-fat food or the alcohol in terms of water soluble dietary fiber of the powder. The product is easy to be taken because the moderate sweetness is imparted by aspartame, and is very easy to drink because to improve the bad aftertaste (bitterness and astringency) of aspartame. The product can be taken directly, by admixing into a beverage such as water, tea, and coffee, or by admixing into a high-fat food or alcohol.

Example 9

<Oral composition>

Ten grams of Branched α-Glucan 2 solution obtained by the method in Production Example 2, one gram of a glucosyl-rutin, two grams of "HAYASHIBARA HESPERIDIN S", a product name of a glucosyl-hesperidin, produced by Hayashibara Co. , Ltd., Okayama, Japan, one gram of a glucosyl-naringin produced by Hayashibara Co., Ltd., Okayama, Japan, and 0.002 gof aspartame were added in 100 ml of a refinedwater, mixed, and dissolved. Fifty ml aliquots of the resulting solution were micro-filtrated and filled into aseptic containers to make into a liquid oral composition.

By ingesting the product, it exhibits bowel movement-improving effect, lasts sense of fullness, and is able to prevent or improve lifestyle-related diseases, fatty liver, and ulcerative colitis, etc. In addition to the river protective effect of the glucosyl- rutin, the glucosyl-hesperidin and the glucosyl-naringin; this product exhibits an effect of the branched α-glucan, it is useful as the composition for preventing or improving fatty liver caused by a high-fat food or an alcohol. It is useful as the composition for preventing or improving fatty liver caused by ingesting high-fat food or alcohol. Although the intake of the product varies depending on the total intake of the high-fat food or the alcohol, the product can be taken in the range of, usually, about 25 to about 500 ml/day/adult (60 kg body mass) in such a manner of allowing the branched α-glucan to be orally taken in an amount of 2% by mass or higher against the total intake of the high-fat food or the alcohol. The product is easy to be taken because the moderate sweetness is imparted by aspartame, and is very easy to drink because to improve the bad aftertaste (bitterness and astringency) of aspartame. The product can be taken directly, by admixing into a beverage such as water, tea, and coffee, or by admixing into a high-fat food or alcohol.

Example 10

<Oral composition>

Both 10 grams of powdery Branched α-Glucan 1 obtained by the method in Production Example 1 and 0.01 g of neotame were uniformly mixed, 5 g aliquots of the resulting mixture were filled into polyethylene containers and sealed to make into an oral composition.

By ingesting the product, it exhibits bowel movement-improving effect, lasts sense of fullness, and is able to prevent or improve lifestyle-related diseases, fatty liver, and ulcerative colitis, etc. Although the intake of the product varies depending on the total intake of the high-fat food or the alcohol, the product can be taken in the range of, usually, about 5 to about 100 g/day/adult (60 kg body mass) in such a manner of allowing the branched α-glucan to be orally taken in an amount of 2% by mass or higher against the total intake of the high-fat food or the alcohol. This product is easy to be taken because the moderate sweetness is imparted by neotame, and is very easy to drink because to improve the bad aftertaste (bitterness and astringency) of neotame . The product can be taken directly, by admixing into a beverage such as water, tea, and coffee, or by admixing into a high-fat food or alcohol.

Example 11

<Oral composition>

Twenty-five grams of Branched α-Glucan 1 powder obtained by the method in Preparation Example 1, 15 g of sorbitol, 10 g of crystalline cellulose, and 35 g of "SUNMALT MIDORI", a product name of a maltose, produced by Hayashibara Co., Ltd., Okayama, Japan, were uniformly mixed, tableted by a tabletting machine by conventional manner to obtain a tablet-type oral composition; about 6 mm thickness, about 2,600 mg hardness.
By ingesting the product, it exhibits bowel movement-improving effect, lasts sense of fullness, and is able to prevent or improve lifestyle-related diseases, fatty liver, and ulcerative colitis, etc. Although the intake of this product varies depending on the total intake of the high-fat food or the alcohol, this product can be taken in the range of, usually, about 5 to about 100 g/day/adult (60 kg body mass) in such a manner of allowing the branched α-glucan to be orally taken in an amount of 2% by mass or higher against the total intake of the high-fat food or the alcohol. The product is easy to be taken because the moderate sweetness is imparted by the maltose, has sufficient physical strength and shows a good disintegrating property in water.

Example 12

<Oral composition>

One hundred parts by mass of powdery Branched α-Glucan 1 obtained by the method in Production Example 1, one part by mass of licorice and 5 parts by mass of "HAYASHIBARA HESPERIDIN S", a product name of a glucosyl-hesperidin, produced by Hayashibara Co., Ltd. , Okayama, Japan, were uniformly mixed, granulated through granulator by conventional manner. The resulting granules were filled into hard capsules to obtain a capsule-type oral composition of the present invention; one gram of Branched α-Glucan 1 per one capsule.
By ingesting the product, it exhibits bowel movement- improving effect, lasts sense of fullness, and is able to prevent or improve lifestyle-related diseases, fatty liver, and ulcerative colitis, etc. Although the intake of the product varies depending on the total intake of the high-fat food or the alcohol, the product can be taken directly, by dissolving into a beverage such as water, tea, and coffee, or byb admixing into a high-fat food or alcohol in the range of, usually, about 5 to about 100 g/day/adult (60 kg body mass) in such a manner of allowing the branched α-glucan to be orally taken in an amount of 2% by mass or higher against the total intake of the high-fat food or the alcohol. The product is excellent in handling and storage stability and in addition to the river protective effect or the neutral fat-reducing action of the glucosyl-hesperidin and licorice; the product can effectively prevent or improve lifestyle-related disease such as fatty liver etc.

Example 13

<Oral composition>

Ten parts by mass of powdery Branched α-Glucan 1 obtained by the method in Production Example 1, 0.1 part by mass of turmeric and one part by mass of a glucosyl-rutin were uniformly mixed, granulated by conventional manner. The resulting granules were obtained a granule-type oral composition.
By ingesting the product, it exhibits bowel movement-improving effect, lasts sense of fullness, and able to prevent or improve lifestyle-related diseases, fatty liver, and ulcerative colitis, etc. Although the intake of the product varies depending on the total intake of the high-fat food or the alcohol, the product can be taken in the range of, usually, about 5 to about 100 g/day/adult (60 kg body mass) in such a manner of allowing the branched α-glucan to be orally taken in an amount of 2% by mass or higher against the total intake of the high-fat food or the alcohol. The product is excellent in handling and storage stability and in addition to the river protective effect or the neutral fat-reducing action of the glucosyl-hesperidin and turmeric. The product can effectively prevent or improve on lifestyle-related disease of fatty liver, etc. The product can be taken directly, by dissolving into a beverage such as water, tea, and coffee, or by admixing into a high-fat food or alcohol.

Example 14

<Oral composition>

One hundred parts by mass of powdery Branched α-Glucan 1 obtained by the method in Production Example 1, 3 parts by mass of "HAYASHIBARA HESPERIDIN S", a product name of a glucosyl-hesperidin, produced by Hayashibara Co., Ltd., Okayama, Japan, 3 parts by mass of and 1 part by mass of a glucosyl-rutin and 0.1 part by mass of gluconolactone were uniformly mixed, granulated through granulator by conventional manner. The resulting granules were filled into hard capsules to obtain a capsule-type oral composition; three grams of Branched α-Glucan 1 per one capsule.

By ingesting the product, it exhibits bowel movement-improving effect, lasts sense of fullness, and is able to prevent or improve lifestyle-related diseases, fatty liver, and ulcerative colitis, etc. Although the intake of the product varies depending on the total intake of the high-fat food or the alcohol, the product can be taken in the range of, usually, about 5 to about 100 g/day/adult (60 kg body mass) in such a manner of allowing the branched α-glucan to be orally taken in an amount of 2% by mass or higher against the total intake of the high-fat food or the alcohol. The product is excellent in handling and storage stability and in addition to the river protective effect or the neutral fat-reducing action of the glucosyl-hesperidin, the glucosyl-rutin and the gluconolactone; the product can effectively prevent or improve lifestyle-related disease such as fatty liver etc.

Example 15

<Oral composition (carbonated beverages)>

Using powdery Branched α-Glucan 1 obtained by the method in Production Example 1, carbonated beverages, a type of oral composition, were prepared. Fifty grams of corn syrup, one gram of citric acid, 0.3 g of sodium citrate and 0.02 g of flavor were mix with 500 ml of carbonated water. While stirring, the resulting mixture solution was added to 40 g of sucrose and added in a ratio of 5, 10 or 20% by mass of powdery Branched α-Glucan 1 to prepare beverages calledA, B, and C, respectively. Except for not admixing Branched α-Glucan 1, the carbonated beverage prepared according to the same method as described above was used as a control. Compared to the control without the branched α-glucan, carbonated beverages A to C were evaluated to have improved palatable and flavor senses on a sensory test with 10 of men and women ranging from their 20's to 50's. Therefore, by adding in a ratio of 5 to 20% by mass of the branched α-glucan, in addition to the effect of improved palatable and flavor senses, the palatability of the carbonated beverages was revealed.

By ingesting the product, it exhibits bowel movement-improving effect, lasts sense of fullness, and is able to prevent or improve lifestyle-related diseases, fatty liver, and ulcerative colitis, etc. Because the branched α-glucan has an effect of improving a palatable sense and a flavor sense of carbonated beverages, the product is quite easy to drink and is able to prevent or improve lifestyle-related disease such as fatty liver etc., effectively.

Example 16

<Oral composition (tea beverage)>

Using powdery Branched α-Glucan 1 obtained by the method in Production Example 1, tea beverages, an oral composition, were prepared. One liter of boiling water was added into 40 g of tea leaves, and the resulting tea leaves were filtered to obtain one L of tea extracts. The tea was added in a ratio of 3, 5 or 8% by mass of powdery Branched α-Glucan 1 to prepare tea beverages called A, B, and C, respectively. Except for not admixing Branched α-Glucan 1, a tea beverage was prepared as a control according to the same method as described above. Compared to the control without the branched α-glucan, tea beverages A to C were evaluated to have improved bitterness and astringency on a sensory test with 10 of men and women ranging from their 20's to 50's. By adding the branched a-glucan, in addition to the effect of reducing bitterness and astringency, the effect of masking the bitterness and astringency specific topolyphenol (s) in the tea beverage was revealed. Furthermore, being subjected to a similar test using green tea beverages instead of the tea beverages , the green tea beverages containing Branched α-Glucan 1 were also evaluated to have reduced bitterness and astringency. The branched α-glucan is obtained evaluation as being reduced bitterness and astringency similarly for green tea. It was revealed that the branched a-glucan exhibits the effect of masking the bitterness and astringency, specific to polyphenol (s) contained in the tea beverages.

By ingesting the product, it exhibits bowel movement-improving effect, lasts sense of fullness, and is able to prevent or improve lifestyle-related diseases, fatty liver, and ulcerative colitis, etc. Because the branched α-glucan has an effect of inhibiting bitterness and astringency of tea beverages, the product is quiet easy to drink and is able to prevent or improve on lifestyle-related disease such as fatty liver etc., effectively.

Example 17

<Oral composition (Lactic acid bacteria beverages)

Lactic acid bacteria beverages, a type of oral composition, were produced using powdery Branched $\alpha$-Glucan 1 obtained by the method in Production Example 1. Commercially available lactic acid bacteria beverages were added in a ratio of 5, 10 or 20% by mass of the powdery branched $\alpha$-glucan to make into lactic acid bacteria beverages called A, B, and C, respectively. Compared to a control lactic acid bacteria beverage without Branched $\alpha$-Glucan 1, the lactic acid bacteria beverages A and B were evaluated to have improved palatable sense and easy drinkability on a sensory test with 10 of men and women ranging from their 20's to 50's. Although the palatable sense of the lactic acid bacteria beverage C was more improved than the control, the lactic acid bacteria beverage C was evaluated as equivalent in terms of the easy drinkability with the lactic acid bacteria beverage without the branched $\alpha$-glucan. Therefore, by adding the branched $\alpha$-glucan to give a content of 5 to 20%, it was found to improve the palatable sense of the lactic acid bacteria beverages. And by adding the branched $\alpha$-glucan to give a content of 5 to 10%, it was found to improve the easy drinkability of the lactic acid bacteria beverages.

By ingesting the product, it exhibits bowel movement-improving effect, lasts sense of fullness, and is able to prevent or improve lifestyle-related diseases, fatty liver, and ulcerative colitis, etc. The branched $\alpha$-glucan improves to the palatable sense of the lactic acid bacteria beverages and makes it easy to drink; this product is very easy to drink and is the lactic acid bacteria beverages to prevent or improve lifestyle-related disease such as fatty liver effectively. The branched $\alpha$-glucan increases the palatable sense of the lactic acid bacterium beverage and makes it easy to drink; this product is really easy to drink and the intake of this product is able to prevent or improve lifestyle-related disease such as fatty liver effectively.

Example 18

<Oral composition (Soy milk beverages)>

Soy milk beverages, a type of oral composition, were produced using powdery Branched $\alpha$-Glucan 1 obtained by the method in Production Example 1. After 450 g of unadjusted soymilk and 50 g of water were mixed, the resulting mixtures were added in a ratio of 2 or 8% by mass of the powdery branched $\alpha$-glucan to make into soy milk beverages called A and B, respectively. And a control soy milk beverage was obtained by the same method described above without adding Branched $\alpha$-Glucan 1. Compared to the control soy milk beverage without Branched $\alpha$-Glucan 1, the soy milk beverages A and B were evaluated to have suppressed harsh taste or odor characteristic on a sensory test with 10 of men and women ranging from their 20's to 50's. By containing the branched $\alpha$-glucan, it was found to be obtained high palatability of the soy beverage . Therefore, it was revealed that the branched $\alpha$-Glucan had an effect of masking harsh taste or odor characteristic for the soy milk beverages .

By ingesting the product, it exhibits bowel movement-improving effect, lasts sense of fullness, and is able to prevent or improve lifestyle-related diseases, fatty liver, and ulcerative colitis, etc. Branched $\alpha$-Glucan is suppressing harsh taste or odor characteristic of soy milk beverage; this product is readily drinkable and the intake of the product is able to prevent or improve lifestyle-related disease such as fatty liver effectively.

Example 19

<Oral composition (Iron component beverages)>

Iron component beverages, a type of oral composition, were produced using powdery Branched $\alpha$-Glucan 1 obtained by the method in Production Example 1. Then, 0.03 g of ferric ammonium citrate, 9.3 g of fructose glucose solution, 0.05 g of citric acid and 0.016 g of sodium citrate were mixed with water. The resulting solution was admixed with powdery Branched $\alpha$-Glucan 1 to give a content of 3%, 5% or 8% to make into iron component beverages called A, B and C, respectively. And a control iron component beverage was obtained by the same method described above without admixing Branched $\alpha$-Glucan 1. Compared to the control iron component beverage without Branched $\alpha$-Glucan 1, the iron component beverages A to C were evaluated to have suppressed metallic taste characteristic of iron component beverage and to have high palatability on a sensory test with 10 of men and women ranging from their 20's to 50's. Therefore, it was revealed that the branched $\alpha$-glucan had an effect of masking metallic taste characteristic to iron-containing beverages.

By ingesting the product, it exhibits bowel movement-improving effect, lasts sense of fullness, and is able to prevent or improve lifestyle-related diseases, fatty liver, and ulcerative colitis, etc. The branched $\alpha$-glucan suppresses metallic taste characteristic of iron component beverage; the product is readily drinkable and the intake of the product is able to prevent or improve lifestyle-related disease such as fatty liver effectively.

Example 20

<Oral composition (Vinegar beverages)>

Vinegar beverages, a type of oral composition, were produced using powdery Branched α-Glucan 1 obtained by the method in Production Example 1. Twenty-seven grams of fructose glucose solution, 0.017 g of sucralose and 50 g of vinegar were mixed with 500 ml of water. The resulting solution was admixed with Branched α-Glucan 1 to give a content of 2% or 8% to make into vinegar beverages called A and B, respectively. And a control vinegar beverage was obtained by the same method described above without adding Branched α-Glucan 1. Compared to the control vinegar beverage without Branched α-Glucan 1, the vinegar beverages A and B were evaluated to have suppressed sour taste and sour smell characteristic and to be easy to drink on a sensory test with 10 of men and women ranging from their 20's to 50's. Therefore, it was revealed that the branched α-glucan had an effect of masking sour taste characteristic for vinegar beverages.

By ingesting the product, it exhibits bowel movement-improving effect, lasts sense of fullness, and is able to prevent or improve lifestyle-related diseases, fatty liver, and ulcerative colitis, etc. The branched α-glucan suppresses sour taste characteristic of vinegar beverage; the product is readily drinkable and the intake of the product is able to prevent or improve lifestyle-related disease such as fatty liver effectively.

Example 21

<Oral composition (Yoghurt)>

Yoghurt, a type of oral composition, was produced using powdery Branched α-Glucan 1 obtained by the method in Production Example 1. First, 100 g of commercially available yoghurt was added in an amount of 1.5 g, 5 g, 10 g and 20 g of powdery Branched α-Glucan 1 to prepare yoghurt called A, B, C and D, respectively. When these yogurts A to D were stored at 4°C for two days, the syneresis was not observed and these shapes had been also maintained. It was revealed that the branched α-glucan had a syneresis-inhibiting effect on yogurt. In addition, measuring hardness of yogurt by using a rheometer ("CR-500DX" manufactured by Sun Scientific Co., Ltd.) revealed that as increased of additional amount of Branched α-Glucan 1, gel strength and shape retention of yogurt increased. Furthermore, commercially available yogurt without Branched α-Glucan 1 was control yogurt. Compared to the control yogurt, the yogurt A to D were evaluated to have suppressed sour taste and sour smell fermentation odors characteristic of yoghurt on a sensory test with 10 of men and women ranging from their 20's to 50's. Therefore, it was revealed that the branched α-glucan had effects of suppressing sour taste and fermentation odors characteristic of yoghurt. The above results were the same when Branched α-Glucan 1 was added in the process of manufacturing yogurt (before or during fermentation of yogurt).

By ingesting the product, it exhibits bowel movement-improving effect, lasts sense of fullness, and is able to prevent or improve lifestyle-related diseases, fatty liver, and ulcerative colitis, etc. Branched α-Glucan is suppressing sour taste and fermentation odors characteristic of yoghurt; the product is readily drinkable and the intake of this product is able to prevent or improve lifestyle-related diseases such as fatty liver effectively.

Example 22

<Oral composition (Fruits sauce)>

Fruits sauce, a type of oral composition, was produced using powdery Branched α-Glucan 1 obtained by the method in Production Example 1. Then, 250g of pineapple prepared by processed in a blender and 125g of sucrose were mixed with water. Fruits sauce called A and B was prepared by adding powdery Branched α-Glucan 1 to the resulting mixture solution in an amount of 5 and 10% by mass, respectively, and heating. In addition, control fruits sauce was obtained by the same method described above except adding the Branched α-Glucan 1. Compared to the control fruits sauce without the Branched α-Glucan 1, the fruits sauces A and B were evaluated to have kept original taste of pineapple, to have given the palatable sense of the fruits sauce and to have been thick sauce on a sensory test with 10 of men and women ranging from their 20's to 50's. Therefore, it was revealed that the branched α-glucan had an effect of giving thickness for fruits sauce.

By ingesting the product, it exhibits bowel movement-improving effect, lasts sense of fullness, and is able to prevent or improve lifestyle-related diseases, fatty liver, and ulcerative colitis, etc. This product is able to prevent or improve lifestyle-related disease such as fatty liver effectively. The product can be ingested directly, ingested by dissolving it in beverages such as water, or adding it to confections such as breads and cakes, or cooked foods.

Example 23

<Oral composition (Tomato sauce)>

Tomato sauce, a type of oral composition, was produced using powdery Branched α-Glucan 1 obtained by the method in Production Example 1. Tomato sauce made from tomato puree, onion, sucrose, salt, granule consomme and a modified starch was admixed with Branched α-Glucan 1 to give a content of 5, 10 and 15% by mass. The resulting mixture was heated at 100°C in the oven to prepare tomato sauces called A, B and C, respectively. In addition, control tomato sauce was obtained by the same method described above without adding Branched α-Glucan 1. Compared to the control tomato sauce without Branched α-Glucan 1, the tomato sauces A to C were a source where the color of tomato sauce was vivid, tasty, and the moisture shift is few. When using this product to pizza andpasta, penetration of moisture into the pizza and pasta was suppressed, because the source could easily remain on the surface, there are often made effective food color and luster with this product. When using this product to noodles such as the pasta, etc., it was revealed that loosening noodles and mouthfeel improved. Therefore, it was revealed that the branched α-glucan had effects of suppressing moisture migration of tomato sauce, loosening noodles, and improving mouthfeel. In order to check whether there is the same effect on noodles other than pasta about the above effect of loosening noodles, using commercially available noodles, it was observed loosening of noodles. First, after the noodles were boiled by conventional manner, 150g of the boiled noodles were immersed for one minute in 500g of the water, which was incorporated with an amount of 5, 10 or 20% by mass of Branched α-Glucan 1, drained, stored at 4°C for 7 hours. Lift gently the noodles after storage, holds about 100 seconds; loosening noodles was observed. As the result, the noodles immersed in the water, which was incorporated with an amount of 5 to 20% by mass of the Branched α-Glucan 1, were revealed that had an effect of loosening noodles . This shows that the branched α-glucan can be used as a release agent for noodles.

By ingesting the product, it exhibits bowel movement-improving effect, lasts sense of fullness, and is able to prevent or improve lifestyle-related diseases, fatty liver, and ulcerative colitis, etc. This product is able to prevent or improve lifestyle-related diseases such as fatty liver effectively. This product can be ingested directly or by admixing into cooked foods for example, noodles such as the pasta or pizzas.

Example 24

<Oral composition (Ice cream)>

Ice cream (Gelato), a type of oral composition, was produced using powdery Branched α-Glucan 1 obtained by the method in Production Example 1. Thirty- five grams of orange juice, 25 g of granulated sucrose, 35 g of water and 5 g of powdery Branched α-Glucan 1 were mixed, heated at 90°C and cooled to room temperature. The ice cream was obtained by mixing the resulting mixture in an ice cream maker for 20 minutes. In addition, control ice cream was obtained by the same method described above without admixing Branched α-Glucan 1. Compared to the control ice cream, the ice cream of the present disclosure was evaluated to have a smooth texture of ice cream on a sensory test with 10 of men and women ranging from their 20's to 50's. Therefore, it was revealed that the branched α-glucan had an effect of giving a smooth texture for chilled sweets such as ice cream.

By ingesting the product, it exhibits bowel movement-improving effect, lasts sense of fullness, and is able to prevent or improve lifestyle-related diseases, fatty liver, and ulcerative colitis, etc. In addition, since the branched α-glucan improves texture, and does not give a bad influence on flavors such as taste and fragrance of the ice cream, the product is easy to ingest as a food.

Example 25

<Oral composition (Soft candy)>

Soft candy, a type of oral composition, was produced using powdery Branched α-Glucan 1 obtained by the method in Production Example 1. Two hundred grams of granulated sucrose, 40 g of water, 200g of starch syrup and 20 or 40 g of Branched α-Glucan 1 powder were added, heated in a pot, and boiled down to 126°C. And then 45.5 g of hydrogenated oil and 2.1 g of an emulsifier were added, and emulsified. Twelve grams of pre-dissolved 33% gelatin solution was added in the resulting mixture . After the temperature of the resulting dough was under 90°C or less, the resulting dough was mixed with 25 g of fondant. Soft candy was prepared by maturing the dough obtained in the above under 40°C or less and press-molding . The soft candies containing Branched α-Glucan 1 in an amount of 20 g and 40 g were used as the soft candies A and B, respectively. In addition, control soft candy was obtained by the same method described above except adding Branched α-Glucan 1. Compared to the control soft candy, the soft candies A and B containing Branched α-Glucan 1 was evaluated to reduce adhesion to the teeth, to make them easy to eat, and to make them difficult to dissolve on a sensory test with 10 of men and women ranging from their 20's to 50's. Therefore, it was revealed that the branched α-glucan had effects of reducing adhesion of the soft candy to the teeth and being difficult to dissolve (heat-resistant shape retention).

By ingesting the product, it exhibits bowel movement-improving effect, lasts sense of fullness, and is able to prevent or improve lifestyle-related diseases, fatty liver, and ulcerative colitis, etc. In addition, since the branched α-glucan reduces adhesion to the teeth, and does not give a bad influence on flavors such as taste and fragrance of the soft candy, the product is easy to ingest as a food.

Example 26

<Oral composition (Hard candy)>

Hard candy, a type of oral composition, was produced using powdery Branched α-Glucan 1 obtained by the method in Production Example 1. One hundred-eighty grams of granulated sucrose, 40 g of water, 160 g of starch syrup were mixed. The resulting mixture containing Branched α-Glucan 1 powder in an amount of 5, 10 or 20% by mass were boiled down to 150°C in a pot, cooled and performed molding to obtain the hard candies . The hard candies containing Branched α-Glucan 1 in an amount of 5, 10 or 20% by mass were used as the hard candies A, B and C, respectively. Control hard candy was prepared by the same method described above without adding Branched α-Glucan 1. Compared to the control hard candy, it was evaluated that the hard candies A, B and C containing Branched α-Glucan 1 had a fluffy-melty mouth feeling, was easy to eat, prolonged the retaining time in mouth and had a effect of giving a sense of heat to the person who licked this article on a sensory test with 10 of men and women ranging from their 20's to 50's. In addition, the hard candies containing Branched α-Glucan 1 was evaluated to give a sense of heat to the person who licked this article. Therefore, it was evaluated that the branched α-glucan had effects of improving texture of hard candy, prolonging the retaining time in mouth, and giving a sense of heat to the person who licked this article. Although a mechanism of giving a sense of heat to the person who ingested this article is not clear, it is considered as partly because heat of fusion is higher than the normal candy.

By ingesting the product, it exhibits bowel movement-improving effect, lasts sense of fullness, and is able to prevent or improve lifestyle-related diseases, fatty liver, and ulcerative colitis, etc. In addition, since the branched α-glucan improves texture of hard candies, and gives a sense of heat to the person who licked this article, the product is easy to ingest, and can be suitably used as throat candies, etc.

Example 27

<Oral composition (Soup)>

Soup, a type of oral composition, was produced using powdery Branched α-Glucan 1 obtained by the method in Production Example 1. First, a source of commercially available consomme soup was dissolved in a moderate amount of hot water. The soups containing Branched α-Glucan 1 in an amount of 5, 10 or 20% by mass were used as the soup A, B, and C, respectively. Control soup was prepared by the same method described above without adding Branched α-Glucan 1. Compared to the control soup without the Branched α-Glucan 1, it was evaluated that the soup A, B, and C improved a palatable sense and a rich taste, had a smooth texture, and was easy to drink. Therefore, it was revealed that the branched α-glucan had effects of giving a palatable sense and a rich taste to the soups, improving texture, and was easy to drink. In the cases of potage soup and cold soup, the same results were obtained.

By ingesting the product, it exhibits bowel movement-improving effect, lasts sense of fullness, and is able to prevent or improve lifestyle-related diseases, fatty liver, and ulcerative colitis, etc. In addition, since the branched α-glucan does not give bad effects of flavor such as taste and fragrance of the soup, and improves texture of it, the product is easy to ingest as a food.

Example 28

<Oral composition (Cooked rice)>

Cooked rice, a type of oral composition, was produced using powdery Branched α-Glucan 1 powder obtained by the method in Production Example 1. After washing 500 g of rice, cooked rice A, B, C, D, E, F were prepared by adding 750 ml of water and Branched α-Glucan 1 in an amount of 0.5, 1, 3, 5, 10 and 20% by mass of rice, respectively, and cooking in an electric rice cooker.

Compared the loosening of cooked rice A to F by mixing these products in a rice paddle, it was evaluated that the cooked rice containing Branched α-Glucan 1 in amount of 3 to 20% by mass improved loosening of it. Especially, it was evaluated that the cooked rice containing Branched α-Glucan 1 in amount of 10 to 20% by mass improved remarkably loosening of it. Furthermore, it was evaluated that the cooked rice containing Branched α-Glucan 1 in amount of 3 to 20% by mass improved grain feeling (texture). From the above, therefore, it was revealed that the branched α-glucan had effects of improving loosening and texture of the cooked rice.

By ingesting the product, it exhibits bowel movement-improving effect, lasts sense of fullness, and can prevent or improve lifestyle-related diseases, fatty liver, and ulcerative colitis, etc. In addition, since the branched α-glucan does not give bad effects of flavor such as taste and fragrance of the cooked rice, and improves texture of it, the product is easy to ingest as a food.

Example 29

<Oral composition (Bread)>

Bread was prepared by adding 1000 g of flour, 20 g of salt, 60 g of sucrose, 20 g of skimmed milk powder, 25 g of Baker's yeast, 700 ml of water and 20 g, 40 g or 80 g of powdery Branched α-Glucan 1 using conventional methods. The breads containing Branched α-Glucan 1 in an amount of 20 g, 40 g and 80 g were used as breads A, B and C, respectively. In addition, control bread was prepared by the same method described above without adding Branched α-Glucan 1. Compared to the control bread without Branched α-Glucan 1, volume of the breads A, B and C were expanded on putting them in an oven. Therefore, it was revealed that the branched α-glucan had an effect of expanding volume of the bread by promoting swelling of bread dough containing the branched α-glucan in baking. In rolls and biscuits, the same results were obtained.

By ingesting the product, it exhibits bowel movement-improving effect, lasts sense of fullness, and is able to prevent or improve lifestyle-related diseases, fatty liver, and ulcerative colitis, etc. In addition, since the branched α-glucan does not give bad effects of flavor such as taste and fragrance of the bread, and improves texture of it, the product is easy to ingest as a food.

Example 30

<High-fat food product (Hamburger)>

Hamburger, a type of high-fat food product, was produced using powdery Branched α-Glucan 1 obtained by the method in Production Example 1. The hamburger was obtained by mixing 100 g of mixed ground pork and beef containing fatty acid in an amount of 10 to 15% by mass, 2 g of salt and pepper, 12 g of milk, 25 g of whole egg, 12 g of raw bread crumb and 2 g of Branched α-Glucan 1 containing approximately 1.5 g of water soluble dietary fiber, kneading the resulting mixture until tenacity is provided, and mixing the mixture with the fried onion, producing prepared food dough. A hamburger was obtained by heating the prepared food dough using conventional methods.

The products contain the branched α-glucan in an amount of 2% by mass or higher in terms of water-soluble dietary fiber against fatty acid contained in the hamburger. Therefore, the products are superior high-fat foods which are less likely to evoke lifestyle-related diseases when ingested. Furthermore, since the products containing the branched α-glucan have bowel-movement-improving effect by ingested and gives relatively high level of sense of fullness even in relatively smaller amount of meals, which is conducive to suppression of food intake; in terms of these, it is said that the products are superior high-fat foods which are less likely to evoke lifestyle-related diseases such as fatty liver. In addition, since the branched α-glucan does not give a bad influence on flavors such as taste, fragrance and texture, the products are easy-to-eat high-fat foods which maintain natural flavors of hamburger.

Example 31

<High-fat food product (Butter cream)>

Butter creams, a type of high-fat food product, were produced using powdery Branched α-Glucan 1 obtained by the method in Production Example 1. The syrups were obtained by mixing 10 g of Branched α-Glucan 1 containing approximately 8 g of water soluble dietary fiber with 100 g of sugar, dissolving the resulting mixture with 100 g of hot water, and boiling down the mixture. Then, the meringue were obtained by mixing the syrups with 100 g of beat up whole egg containing fatty acid in an amount of approximately 8% by mass, and beating up the mixture using conventional methods. The butter creams high-fat food products were produced by mixing the meringue with 200 g of Salt-free margarine containing fatty acid in an amount of approximately 80% by mass, and flavor. A control butter cream was obtained by the same method described about except mixing Branched α-Glucan 1. Butter cream contained Branched α-Glucan 1 compared with the control butter cream, the products were provided evaluation that increasing the softness and the melt-in-the-mouth, having few air bubbles. Therefore, it was revealed that the branched α-Glucan had quality of taste improving for Butter creams.

The products contain the branched a-Glucan in an amount of 2% by mass or higher in terms of water-soluble dietary fiber against fatty acid contained in the butter creams. Therefore, the products are superior high-fat foods which are less likely to evoke lifestyle-related diseases when ingested. Furthermore, since the products containing the branched α-glucan have bowel-movement-improving effect by ingested and gives relatively high level of sense of fullness even in relatively smaller amount of meals, which is conducive to suppression of food intake; in terms of these, it is said that the products are superior high-fat foods which are less likely to evoke lifestyle-related diseases such as fatty liver. In addition, since the branched α-glucan has quality of taste improving effect on butter cream and does not have a bad influence on flavors such as taste, fragrance and texture, the products are easy-to-eat high-fat foods which maintain natural flavors of butter cream.

Example 32

<High-fat food product (Puddings)>

Puddings, a type of high-fat food product, were produced using powdery Branched α-Glucan 1 obtained by the method in Production Example 1. The puddings were obtained by mixing 4 g, 8g, 20 g or 40 g of Branched α-Glucan 1 with 40 g of sugar, dissolving the resulting mixture with 250 g of bovine milk at 80°C, mixing the resulting mixture with 100 g of whole egg containing approximately 8% fatty acid, sieving the resulting mixture to make liquid mixture of the ingredients, and heating the liquid mixture of the ingredients packed into plastics containers using conventional methods. The puddings containing Branched α-Glucan 1 in an amount of 4 g, 8 g, 20 g and 40 g were used as Pudding A to D of high-fat food products, respectively. A control pudding was obtained by the same method described above without admixing Branched α-Glucan 1.

In Pudding A to D, the more Branched α-Glucan 1 was contained, the softer they were and the smoother texture they had. They also maintained their shapes as well as the control pudding. Therefore, it was revealed that the branched α-glucan had texture-improving effect of giving softness and smoothness on egg products such as puddings.

The products contain the branched α-glucan in an amount of 2% by mass or higher in terms of water-soluble dietary fiber against fatty acid contained in the puddings. Therefore, the products are superior high-fat foods which are less likely to evoke lifestyle-related diseases such as fatty liver when ingested. Furthermore, since the products containing the branched α-glucan have an effect of improving bowel movement by ingested and provide a sense of fullness at relatively high level even in relatively smaller amount of meals, which is conducive to inhibition of food intake; in terms of these, it is said that the products are superior high-fat foods which are less likely to evoke lifestyle-related diseases such as fatty liver. In addition, since the branched α-glucan has texture-improving effect on puddings and does not have a bad influence on flavors such as taste, fragrance and texture, the products are easy-to-eat high-fat foods which maintain natural flavors of puddings.

Example 33

<High-fat food product (Chocolates)>

Chocolates, a type of high-fat food product, were produced using powdery Branched α-Glucan 1 obtained by the method in Production Example 1. First, black chocolate and fresh cream containing fatty acid in an amount of approximately 40% by mass was mixed at 60°C. Chocolate A, B, C and D were prepared by adding Branched α-Glucan 1 to the resulting mixture in an amount of 5%, 10%, 20% and 40% by mass, respectively, and shaping after cooling. Each chocolate could be manufactured without adherence . A control chocolate was obtained by the same method described above without admixing Branched α-Glucan 1.

When the thermostability of Chocolate A through D was tested by storing them in a incubator at 40°C for 30 minutes, Chocolate B and C containing 10% and 20% Branched α-Glucan 1, respectively, had higher thermostability and were harder to melt than the control chocolate. Therefore, it was revealed that the branched α-glucan had an effect of improving thermostability on chocolates, and preventing them from melting.

The products contain the branched α-glucan in an amount of 2% by mass or higher in terms of water-soluble dietary fiber against fatty acid contained in the chocolates. Therefore, the products are superior high-fat foods which are less likely to evoke lifestyle-related diseases such as fatty liver when ingested. Furthermore, since the products containing the branched α-glucan have a effect of improving bowel movement by ingested and provide a sense of fullness at relatively high level even in relatively smaller amount of meals, which is conducive to suppression of food intake; in terms of these, it is said that the products are superior high-fat foods which are less likely to evoke lifestyle-related diseases such as fatty liver. In addition, since the branched α-glucan has a effect of improving thermostability on chocolates, the products are hard to melt, and since the branched α-glucan does not have a bad influence on flavors such as taste, fragrance and texture, the products are easy-to-eat high-fat foods.

Example 34

<High-fat food product (Cookies)>

Cookies, a type of high-fat food product, were produced using powdery Branched α-Glucan 1 obtained by the method in Production Example 1. The cookie dough was obtained by mixing 8 g or 13 g of Branched α-Glucan 1 with 25 g of butter containing fatty acid in an amount of approximately 80% by mass, 17 g of sugar, 38 g of soft wheat flour and 12 g of whole egg, and beating the resulting mixture with a blender, and using conventional methods. After cooling the resulting dough, the cookies were obtained by cutting the dough to the proper size, arranging the cut dough extended to 10 mm thick by applying pressure on a baking sheet, baking the cut dough at 170°C for 15 minutes using electrical oven, and cooling the resulting cookies. The cookies containing Branched α-Glucan 1 in an amount of 8 g and 13 g were used as Cookie A and B, respectively. A control cookie was produced using the same dough without admixing Branched α-Glucan 1.

In manufacturing, adherence of the dough of Cookie A and B was suppressed compared to that of the control cookie which did not contain Branched α-Glucan 1. It was also revealed that, by adding the branched α-glucan, cookies became relatively harder and hard to crack, and textures of cookies became crispy. Therefore, the branched α-glucan has an effect of suppressing adherence of cookie dough, making cookies hard to crack, and improving their textures. In soft cookies and biscuits, the almost same results were obtained.

The products contain the branched α-glucan in an amount of 2% by mass or higher in terms of water-soluble dietary fiber against fatty acid contained in the cookies. Therefore, the products are superior high-fat foods which are less likely to evoke lifestyle-related diseases such as fatty liver when ingested. Furthermore, since the products containing the branched α-glucan have a effect of improving bowel movement by ingested and provide a sense of fullness at relatively high level even in relatively smaller amount of meals, which is conducive to suppression of food intake; in terms of these, it is said that the products are superior high-fat food product which are less likely to evoke lifestyle-related diseases such as fatty liver. In addition, since the branched α-glucan has a effect of increasing hardness on cookies, and since the branched α-glucan does not have a bad influence on flavors such as taste, fragrance and texture, the products are easy-to-eat high-fat foods.

Example 35

<High-fat food product (Deep-fried foods)>

Deep-fried foods, a type of high-fat food product, were produced using powdery Branched α-Glucan 1 obtained by the method in Production Example 1. Branched α-Glucan 1 was added to with a commercially available flour mix for deep fried food in an amount of 3, 5 or 10% by mass, and mixed. Deep-fried foods were obtained by dredging chicken thighs containing approximately 5% fatty acid by mass with 10 g of the resulting flour mixes per 100 g of the chicken thighs, frying the resulting chicken thighs in hot oil containing fatty acid in an amount of 100% by mass at 170°C using conventional methods. The Deep-fried foods obtained using the flour mixes containing 3%, 5% and 10% Branched α-Glucan 1 were used as Deep-Fried Food A, B and C, respectively. A control deep-fried food was obtained using the commercially available flour mix for deep fried food without adding Branched α-Glucan 1.

Deep-Fried Food A, B and C have crispier texture than the control deep-fried food. Therefore, it was revealed that the branched α-glucan had texture-improving effect of improving a feeling of crispy on fried foods such as deep-fried foods . Besides, deep-fried foods obtained using flour mixes for deep frying mixed with Branched α-Glucan 2 obtained by the method shown in Preparation Example 2 have crispy texture as well.

The products contain the branched α-glucan in an amount of 2% by mass or higher in terms of water-soluble dietary fiber against fatty acid contained in the deep-fried foods. Therefore, the products are superior high-fat foods which are less likely to evoke lifestyle-related diseases such as fatty liver when ingested. Furthermore, since the products containing the branched α-glucan have a effect of improving bowel movement by ingested and provide a sense of fullness at relatively high level even in relatively smaller amount of meals, which is conducive to suppression of food intake; in terms of these, it is said that the products are superior high-fat food product which are less likely to evoke lifestyle-related diseases such as fatty liver . In addition, since the branched α-glucan improve textures of deep-fried foods for feeling crispy as well as does not have a bad influence on taste, fragrance, colors, etc., the products are easy-to-eat high-fat foods which maintain natural flavor and *Umami* taste of deep-fried foods, and are improved their textures.

Example 36

<High-fat food product (Fritters)>

Fritters, a type of high-fat food product, were produced using powdery Branched α-Glucan 1 obtained by the method in Production Example 1. Fritters were obtained by adding Branched α-Glucan 1 in an amount of 5, 10 or 15% by mass to a commercially available flour mix for fritter, dredging ingredients with the resulting flour mixes, frying the resulting ingredients in hot oil containing fatty acid in an amount of 100% by mass at 170°C using conventional methods. The fritters obtained using the flour mixes containing 5, 10 and 15% Branched α-Glucan 1 were used as Fritter A, B and C, respectively. A control fritter was obtained using the commercially available flour mix for fritter without Branched α-Glucan 1.

In manufacturing Fritter A, B and C, since they have crispier texture than the control fritter, it was revealed that the branched α-glucan had texture-improving effect of improving a feeling of crispy on fritter.

Since the products contain the branched α-glucan, the products are superior high-fat foods which are less likely to evoke lifestyle-related diseases such as fatty liver when ingested. Furthermore, since the products containing the branched α-glucan have a effect of improving bowel movement by ingested and provide a sense of fullness at relatively high level even in relatively smaller amount of meals, which is conducive to suppression of food intake; in terms of these, it is said that the products are superior high-fat food product which are less likely to evoke lifestyle-related diseases such as fatty liver. In addition, since the branched α-glucan improve textures of fritters for feeling crispy as well as does not have a bad influence on taste, fragrance, colors, etc., the products are easy-to-eat high-fat food products which maintain natural flavor and *Umami* taste of deep-fried foods, and are improved their textures.

Example 37

<High-fat food product (Cakes)>

Cakes, a type of high-fat food product, were produced using powdery Branched α-Glucan 1 obtained by the method in Production Example 1. The dough of sponge cakes were obtained by mixing 8 g or 16 g of Branched α-Glucan 1 with 70 g of butter containing fatty acid in an amount of approximately 80% by mass, 250 g of sugar, 175 g of soft wheat flour and 350 g of whole egg, and beating using conventional methods. After cooling the resulting dough, the sponge cakes were obtained by cutting the dough to the proper size, baking the cut dough at 180°C for 30 minutes in oven, and cooling the resulting sponge cakes. The sponge cakes containing Branched α-Glucan 1 in an amount of 8 g and 16 g were used as Sponge Cake A and B, respectively. A control sponge cake was produced using the same dough without admixing Branched α-Glucan 1.

In manufacturing, adherence of the dough of Sponge Cake A and B was suppressed compared to that of the control sponge cake. It was also revealed that, by adding the branched α-glucan, the sponge cakes became easy to melt in the mouth, became light texture, and improving moist feelings. In muffins and pound cakes, the almost same results were obtained. Therefore, the branched α-glucan has an effect of suppressing adherence of dough of cakes and muffins, improving manufacturing efficiencies, and improving the textures of the resulting cakes, etc.

The products contain the branched α-glucan in an amount of 2% by mass or higher in terms of water-soluble dietary fiber against fatty acid contained in the puddings. Therefore, the products are superior high-fat foods which are less likely to evoke lifestyle-related diseases such as fatty liver when ingested. Furthermore, since the products containing the branched α-glucan have a effect of improving bowel movement by ingested and provide a sense of fullness at relatively high level even in relatively smaller amount of meals, which is conducive to suppression of food intake; in terms of these, it is said that the products are superior high-fat foods which are less likely to evoke lifestyle-related diseases such as fatty liver. In addition, since the branched α-glucan has texture-improving effect on cakes and does not have a bad influence on flavors such as taste, fragrance and texture, the products are easy-to-eat high-fat foods.

## INDUSTRIAL APPLICABILITY

An agent for lifestyle-related diseases, an oral composition, and a high-fat food product can be used for preventing or treating lifestyle-related diseases such as fatty liver. An agent for lifestyle-related diseases, an oral composition, and a high-fat food product can also be used as an agent for improving bowel movement, an agent for lasting a sense of fullness, and an agent for preventing inflammatory bowel disease, and the industrial applicability of this disclosure is significant at the present time when dietary habits alter to Western-style and population is increasingly aging.

**Claims**

1. An agent for use in treating or preventing lifestyle-related diseases selected from the group consisting of fatty liver and ulcerative colitis, which agent comprises a branched α-glucan having the following characteristics (A) to (D):

   (A) being constructed by glucose molecules;
   (B) having a branched structure with a glucose polymerization degree of one or higher, which is bound *via* a linkage except for α-1,4 linkage to a glucose residue at the non-reducing end of a linear glucan with a glucose polymerization degree of 3 or higher which has a structure of binding glucose *via* α-1,4 linkage;
   (C) having a glucose polymerization degree of 6 to 430 and having a value of weight-average molecular weight (Mw) divided with number average molecular weight (Mn), Mw/Mn, being not more than 20; and
   (D) having a content of water-soluble dietary fiber, obtained by applying a high performance liquid chromatography method (Enzyme-HPLC method), of 20% by mass or higher.

2. The agent for use according to claim 1, wherein said branched α-glucan gives the following characteristics upon methylation analysis:

   (1) Ratio of 2,3,6-trimethyl-1,4,5-triacetyl-glucitol to 2,3,4-trimethyl-1,5,6-triacetyl-glucitol is in the range of 1:0.6 to 1:4;
   (2) Total content of 2,3,6-trimethyl-1,4,5-triacetyl-glucitol and 2,3,4-trimethyl-1,5,6-triacetyl-glucitol is 60% or higher in the partially methylated glucitol acetates;
   (3) Content of 2,4,6-trimethyl-1,3,5-triacetyl-glucitol is 0.5% or higher but not higher than 10% in the partially methylated glucitol acetates; and
   (4) Content of 2,4-dimethyl-1,3,5,6-tetraacetyl-glucitol is 0.5% or higher in the partially methylated glucitol acetates.

3. The agent for use according to claim 1 or claim 2, wherein the agent is comprised in an oral composition further comprising one or more members selected from the group consisting of a polyphenol, a sweetener, and a high intensity sweetener.

4. The agent for use according to claim 3, wherein said polyphenol is one or more members selected from the group consisting of glycosyl-rutin, glycosyl-hesperidin, and glycosyl-naringin.

5. The agent for use according to claim 3, wherein said high intensity sweetener is one or more members selected from the group consisting of neotame, aspartame, stevia extract, enzyme-treated stevia, suclarose, asesulfame-K, saccharin, thaumatin, and glycyrrhizin.

6. The agent for use according to any one of claims 1 to 5, wherein the agent is comprised in a high-fat food product.

7. Non-therapeutic use of an agent to maintain or increase a sense of fullness, wherein the agent comprises a branched α-glucan having the following characteristics (A) to (D):

   (A) being constructed by glucose molecules;
   (B) having a branched structure with a glucose polymerization degree of one or higher, which is bound *via* a linkage except for α-1,4 linkage to a glucose residue at the non-reducing end of a linear glucan with a glucose polymerization degree of 3 or higher which has a structure of binding glucose *via* α-1,4 linkage;
   (C) having a glucose polymerization degree of 6 to 430 and having a value of weight-average molecular weight (Mw) divided with number average molecular weight (Mn), Mw/Mn, being not more than 20; and
   (D) having a content of water-soluble dietary fiber, obtained by applying a high performance liquid chromatography method (Enzyme-HPLC method), of 20% by mass or higher.

8. The non-therapeutic use according to claim 7, wherein said branched α-glucan gives the following characteristics upon methylation analysis:

   (1) Ratio of 2,3,6-trimethyl-1,4,5-triacetyl-glucitol to 2,3,4-trimethyl-1,5,6-triacetyl-glucitol is in the range of 1:0.6 to 1:4;
   (2) Total content of 2,3,6-trimethyl-1,4,5-triacetyl-glucitol and 2,3,4-trimethyl-1,5,6-triacetyl-glucitol is 60% or higher in the partially methylated glucitol acetates;

(3) Content of 2,4,6-trimethyl-1,3,5-triacetyl-glucitol is 0.5% or higher but not higher than 10% in the partially methylated glucitol acetates; and

(4) Content of 2,4-dimethyl-1,3,5,6-tetraacetyl-glucitol is 0.5% or higher in the partially methylated glucitol acetates.

**Patentansprüche**

1. Mittel zur Verwendung bei der Behandlung oder Verhütung von lebensstilbezogenen Krankheiten, ausgewählt aus der Gruppe bestehend aus Fettleber und Colitis ulcerosa, wobei das Mittel ein verzweigtes $\alpha$-Glucan mit den folgenden Eigenschaften (A) bis (D) umfasst:

(A) es ist aus Glucosemolekülen aufgebaut;

(B) es hat eine verzweigte Struktur mit einem Glucosepolymerisationsgrad von eins oder höher, die über eine Verknüpfung, mit Ausnahme einer a-1,4-Verknüpfung, an einen Glucoserest am nicht reduzierenden Ende eines linearen Glucans mit einem Glucosepolymerisationsgrad von 3 oder höher gebunden ist, das eine Struktur zur Bindung von Glucose über eine $\alpha$-1,4-Verknüpfung hat;

(C) es hat einen Glucosepolymerisationsgrad von 6 bis 430 und einen gewichtsgemittelten Molekülmassen-(Mw)-Wert, dividiert durch die zahlengemittelte Molekülmasse (Mn), Mw/Mn, von nicht mehr als 20; und

(D) es hat einen Gehalt an wasserlöslichen Ballaststoffen, erhalten durch Anwenden eines Hochleistungsflüssigkeitschromatographieverfahrens (Enzym-HPLC-Verfahren), von 20 Masse-% oder mehr.

2. Mittel zur Verwendung nach Anspruch 1, wobei das genannte verzweigte $\alpha$-Glucan nach einer Methylierungsanalyse die folgenden Eigenschaften hat:

(1) das Verhältnis zwischen 2,3,6-Trimethyl-1,4,5-triacetyl-glucitol und 2,3,4-Trimethyl-1,5,6-triacetyl-glucitol liegt im Bereich von 1:0,6 bis 1:4;

(2) der Gesamtgehalt an 2,3,6-Trimethyl-1,4,5-triacetyl-glucitol und 2,3,4-Trimethyl-1,5,6-triacetyl-glucitol beträgt 60 % oder mehr in den partiell methylierten Glucitolacetaten;

(3) der Gehalt an 2,4,6-Trimethyl-1,3,5-triacetyl-glucitol beträgt 0,5 % oder mehr, aber nicht mehr als 10 % in den partiell methylierten Glucitolacetaten; und

(4) der Gehalt an 2,4-Dimethyl-1,3,5,6-tetraacetyl-glucitol beträgt 0,5 % oder mehr in den partiell methylierten Glucitolacetaten.

3. Mittel zur Verwendung nach Anspruch 1 oder Anspruch 2, wobei das Mittel in einer oralen Zusammensetzung enthalten ist, die ferner ein oder mehrere Mitglieder der Gruppe bestehend aus einem Polyphenol, einem Süßstoff und einem hochintensiven Süßstoff umfasst.

4. Mittel zur Verwendung nach Anspruch 3, wobei das genannte Polyphenol ein oder mehrere Mitglieder der Gruppe bestehend aus Glycosylrutin, Glycosylhesperidin und Glycosylnaringin ist.

5. Mittel zur Verwendung nach Anspruch 3, wobei der genannte hochintensive Süßstoff ein oder mehrere Mitglieder der Gruppe bestehend aus Neotam, Aspartam, Stevia-Extrakt, enzymbehandeltem Stevia, Sucralose, Acesulfam K, Saccharin, Thaumatin und Glycyrrhizin ist.

6. Mittel zur Verwendung nach einem der Ansprüche 1 bis 5, wobei das Mittel in einem fettreichen Lebensmittelprodukt enthalten ist.

7. Nicht therapeutische Verwendung eines Mittels zum Aufrechterhalten oder Erhöhen eines Völlegefühls, wobei das Mittel ein verzweigtes $\alpha$-Glucan mit den folgenden Eigenschaften (A) bis (D) umfasst:

(A) es ist aus Glucosemolekülen aufgebaut;

(B) es hat eine verzweigte Struktur mit einem Glucosepolymerisationsgrad von eins oder höher, die über eine Verknüpfung, mit Ausnahme einer $\alpha$-1,4-Verknüpfung, an einen Glucoserest am nicht reduzierenden Ende eines linearen Glucans mit einem Glucosepolymerisationsgrad von 3 oder höher gebunden ist, das eine Struktur zur Bindung von Glucose über eine $\alpha$-1,4-Verknüpfung hat;

(C) es hat einen Glucosepolymerisationsgrad von 6 bis 430 und einen gewichtsgemittelten Molekülmassen-(Mw)-Wert, dividiert durch die zahlengemittelte Molekülmasse (Mn), Mw/Mn, von nicht mehr als 20; und

(D) es hat einen Gehalt an wasserlöslichen Ballaststoffen, erhalten durch Anwenden eines Hochleistungsflüssigkeitschromatographieverfahrens (Enzym-HPLC-Verfahren), von 20 Masse-% oder mehr.

8.  Nicht therapeutische Verwendung nach Anspruch 7, wobei das genannte verzweigte $\alpha$-Glucan nach einer Methylierungsanalyse die folgenden Eigenschaften hat:

    (1) dass Verhältnis zwischen 2,3,6-Trimethyl-1,4,5-triacetyl-glucitol und 2,3,4-Trimethyl-1,5,6-triacetyl-glucitol liegt im Bereich von 1:0,6 bis 1:4;
    (2) der Gesamtgehalt an 2,3,6-Trimethyl-1,4,5-triacetyl-glucitol und 2,3,4-Trimethyl-1,5,6-triacetyl-glucitol beträgt 60 % oder mehr in den partiell methylierten Glucitolacetaten;
    (3) der Gehalt an 2,4,6-Trimethyl-1,3,5-triacetyl-glucitol beträgt 0,5 % oder mehr, aber nicht mehr als 10 % in den partiell methylierten Glucitolacetaten; und
    (4) der Gehalt an 2,4-Dimethyl-1,3,5,6-tetraacetyl-glucitol beträgt 0,5 % oder mehr in den partiell methylierten Glucitolacetaten.

## Revendications

1.  Agent pour une utilisation dans le traitement ou la prévention de maladies liées au mode de vie sélectionnées dans le groupe consistant en la stéatose hépatique et la rectocolite hémorragique, ledit agent comprenant $\alpha$-glucane ramifié qui présente les caractéristiques (A) à (D) suivantes :

    (A) constitué de molécules de glucose ;
    (B) comportant une structure ramifiée ayant un degré de polymérisation du glucose de un ou plus qui est liée par une liaison autre qu'une liaison $\alpha$-1,4 à un résidu glucose à l'extrémité non réductrice d'un glucane linéaire ayant un degré de polymérisation du glucose de 3 ou plus qui contient une structure de liaison au glucose par une liaison $\alpha$-1,4 ;
    (C) ayant un degré de polymérisation du glucose qui va de 6 à 430 et une valeur de masse moléculaire moyenne en poids (Mw) divisée par la masse moléculaire moyenne en nombre (Mn), Mw/Mn, qui ne dépasse pas 20 ; et
    (D) ayant une teneur en fibres alimentaires hydrosolubles, déterminée en appliquant une méthode de chromatographie liquide à haute performance (méthode de CLHP par voie enzymatique), égale ou supérieure à 20 % en masse.

2.  Agent pour une utilisation selon la revendication 1, où ledit $\alpha$-glucane ramifié présente les caractéristiques suivantes à l'analyse de la méthylation :

    (1) Le rapport 2,3,6-triméthyl-1,4,5-triacétyl-glucitol/2,3,4-triméthyl-1,5,6-triacétyl-glucitol est dans la plage de 1:0,6 à 1:4 ;
    (2) La teneur totale en 2,3,6-triméthyl-1,4,5-triacétyl-glucitol et 2,3,4-triméthyl-1,5,6-triacétyl-glucitol est égale ou supérieure à 60 % dans les acétates de glucitol partiellement méthylés ;
    (3) La teneur en 2,4,6-triméthyl-1,3,5-triacétyl-glucitol est égale ou supérieure à 0,5 % mais ne dépasse pas 10 % dans les acétates de glucitol partiellement méthylés ; et
    (4) La teneur en 2,4-diméthyl-1,3,5,6-tétraacétyl-glucitol est égale ou supérieure à 0,5 % dans les acétates de glucitol partiellement méthylés.

3.  Agent pour une utilisation selon la revendication 1 ou la revendication 2, où l'agent est compris dans une composition orale comprenant en outre un ou plusieurs éléments sélectionnés dans le groupe consistant en un polyphénol, un édulcorant et un édulcorant de haute intensité.

4.  Agent pour une utilisation selon la revendication 3, où ledit polyphénol est un ou plusieurs éléments sélectionnés dans le groupe consistant en la glycosyl-rutine, la glycosyl-hespéridine et la glycosyl-naringine.

5.  Agent pour une utilisation selon la revendication 3, où ledit édulcorant de haute intensité est un ou plusieurs éléments sélectionnés dans le groupe consistant en le néotame, l'aspartame, l'extrait de stévia, le stévia traité enzymatiquement, le suclarose, l'asésulfame-K, la saccharine, la thaumatine et la glycyrrhizine.

6.  Agent pour une utilisation selon l'une quelconque des revendications 1 à 5, où l'agent est compris dans un produit alimentaire riche en graisses.

**7.** Utilisation non thérapeutique d'un agent pour maintenir ou augmenter une sensation de réplétion, où l'agent comprend un $\alpha$-glucane ramifié qui présente les caractéristiques (A) à (D) suivantes :

(A) constitué de molécules de glucose ;

(B) comportant une structure ramifiée ayant un degré de polymérisation du glucose de un ou plus qui est liée par une liaison autre qu'une liaison $\alpha$-1,4 à un résidu glucose à l'extrémité non réductrice d'un glucane linéaire ayant un degré de polymérisation du glucose de 3 ou plus qui contient une structure de liaison au glucose par une liaison $\alpha$-1,4 ;

(C) ayant un degré de polymérisation du glucose qui va de 6 à 430 et une valeur de masse moléculaire moyenne en poids (Mw) divisée par la masse moléculaire moyenne en nombre (Mn), Mw/Mn, qui ne dépasse pas 20 ; et

(D) ayant une teneur en fibres alimentaires hydrosolubles, déterminée en appliquant une méthode de chromatographie liquide à haute performance (méthode de CLHP par voie enzymatique), égale ou supérieure à 20 % en masse.

**8.** Utilisation non thérapeutique selon la revendication 7, où ledit $\alpha$-glucane ramifié présente les caractéristiques suivantes à l'analyse de la méthylation :

(1) Le rapport 2,3,6-triméthyl-1,4,5-triacétyl-glucitol/2,3,4-triméthyl-1,5,6-triacétyl-glucitol est dans la plage de 1:0,6 à 1:4 ;

(2) La teneur totale en 2,3,6-triméthyl-1,4,5-triacétyl-glucitol et 2,3,4-triméthyl-1,5,6-triacétyl-glucitol est égale ou supérieure à 60 % dans les acétates de glucitol partiellement méthylés ;

(3) La teneur en 2,4,6-triméthyl-1,3,5-triacétyl-glucitol est égale ou supérieure à 0,5 % mais ne dépasse pas 10 % dans les acétates de glucitol partiellement méthylés ; et

(4) La teneur en 2,4-diméthyl-1,3,5,6-tétraacétyl-glucitol est égale ou supérieure à 0,5 % dans les acétates de glucitol partiellement méthylés.

FIG. 1

FIG. 2

FIG. 3

FIG. 4

FIG. 5

FIG. 6

I do not have a sense
of fullness at all.

How much do you have a sense of fullness?

I completely have
a sense of fullness.

I am not satisfied at all.

How much are you satisfied with eating?

I am absolutely satisfied.

FIG. 7

**Sense of fullness**

FIG. 8

**Satisfuction level**

FIG. 9

## Sense of fullness

*The score of the group ingested Branched Glucan Solution is significantly different from that of the group ingested Maltodextrin Solution (P<0.05).

FIG. 10

## Satisfaction level

*The score of the group ingested Branched Glucan Solution is significantly different from that of the group ingested Maltodextrin Solution (P<0.05).

FIG. 11

*The value of the operating group is significantly different from that of the control group (P<0.05).

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4300828 A
- WO 2008136331 A
- WO 2010100583 A

**Non-patent literature cited in the description**

- Food Processing and Ingredients. UBM Media Co., Ltd, 2013, vol. 48, 15-17
- Shokumotsu-Seni, Kiso-to-Rinsho (Dietary Fiber - Foundation and Clinics). Asakura Publishing Co., Ltd, 1997, 191-203
- Food science. Kabushiki-Gaisha Shokuhin-to-Kagaku-Sha, August 2003, 83-87
- Nihon-Chori-Kagakukai-Shi. The Japan Society of Cookery Science, 2009, vol. 42, 386-393
- HIMAN-KENKYU (Studies on obesity). Japan Society for the Study of Obesity, 2012, vol. 18, 39-51
- NIPPON RINSHO (Japanese Journal of Clinical Medicine). Kabushiki Kaisha Nippon Rinsho Sha, 2011, vol. 69, 826-829, 946-950
- *J. Gastroenterology. Hepatic,* 2001, vol. 16, 160-168
- **TSUBOUCHI et al.** *Digestion,* 2006, vol. 74, 91-100